# EUROPEAN PATENT APPLICATION

(11) **EP 4 848 000 A2**
(43) Date of publication of application: **02.12.2026**
(21) Application number: 26212760.8
(22) Date of filing: 17.06.2024
(51) Int. Cl.: G03F 7/031

(54) **DYNAMIC COVALENT CHEMISTRY OF SPIROPYRANS FOR SYNTHESIS OF PHOTOSWITCHABLE PHOTOINITIATORS AND USES THEREOF**

(30) Priority: 19.06.2023 DE 102023115968
(62) Document number(s) of the earlier application(s) in accordance with Art. 76 EPC: 24733610.0 / 4 727 947
(71) Applicant: xolo GmbH, 12489 Berlin (DE)
(72) Inventor: Garmshausen, Yves, 12621 Berlin (DE)
(74) Representative: Erbacher, Martin

(57) **Abstract**

The present invention relates to spiropyrans and process for the manufacture of them. In particular, a method is provided to exchange the indole and salicylaldehyde part of a spiropyran in a reversible reaction. The method is used for the synthesis of dual color photoinitiators with specific substituents and substitution patterns, which result in improved dual color volumetric printing (xolography). Furthermore, a process for locally polymerizing a starting material by dual color photopolymerization is disclosed.

## Description

### Field of invention

The present invention relates to dynamic covalent chemistry of spiropyrans and application thereof. In particular, a process is provided to synthesize spiropyrans via an exchange reaction. Thereby, substitution patterns at spiropyrans may be firstly synthetically accessible. The provided spiropyrans contain specific substituents and substitution patterns and are used as dual color photoinitiators, which result in improved dual color volumetric printing (xolography).

### Background of the art

### Spiropyran, and merocyanine used as photoswitches

A definition of photoswitches, structures which are photochromic, and terms used in the field can be found in [Bouas-Laurent, Dürr, Pure and Applied Chemistry, 2001, 73, 639-665]. In general, a photoswitch is a photochromic compound which can undergo a reversible structural change in at least one direction via a photochemical path. A typical example is the spiropyran/merocyanine system, as illustrated below:

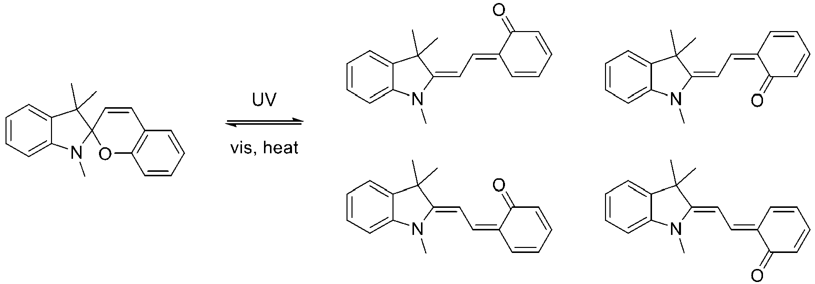

The colorless spiropyran is the thermodynamically favored form and undergoes a ring opening reaction upon irradiation with UV light to the metastable colored merocyanine form (positive photochromism). The merocyanine form can be a mixture of different isomers, where the bonds between the five-membered and six-membered ring are either E or Z configurated. Within this invention the term merocyanine refers to one or more of the different E/Z isomers. The reverse reaction from merocyanine to spiropyran can be conducted either by light or thermally (T-type photochromism).

Spiropyrans are generally synthesized by the condensation of a salicylaldehyde and a 2-methyleneindoline, which may be obtained by deprotonation of an indolenium salt. The spiropyran may be functionalized afterwards, especially if it contains suitable functional groups:

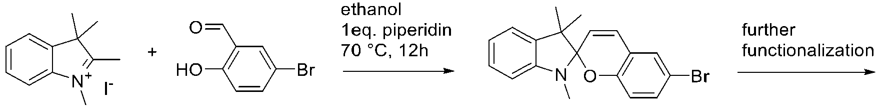

Due to the free hydroxy group and the free aldehyde group, the direct functionalization of salicylaldehydes is limited. There exists no direct protecting group for a salicylaldehyde. Salicylaldehydes are sensitive to nucleophiles, oxidative and reductive conditions. Typical reactions like the halide-metal exchange of a bromine with n-butyl lithium are not possible, since the hydroxy group is deprotonated, instead of undergoing the desired halide-metal exchange. Furthermore, the hydroxy group has a strong directing effect and limits the direct functionalization to the position *para* to the hydroxy group. Also, the aldehyde as a strong electron withdrawing group deactivates the aromatic system, which allows only very reactive reagents for the direct functionalization, such as acetic anhydride with aluminum chloride in a Friedl-Crafts-acylation. Other typical reactions, like the reduction of a nitro group are not possible in presence of the formyl group of the salicylaldehyde as the formyl group is reduced under similar conditions. Thus, functionalization of the salicylaldehyde is limited.

### Dynamic Covalent Chemistry

An overview on dynamic covalent chemistry with terms and definitions can be found in [Yinghua Jin, Chao Yu, Ryan J. Denman, Wei Zhang, Chemical Society Reviews, 2013, 42, 6634-6654, Peter T. Corbett, Julien Leclaire, Laurent Vial, Kevin R. West, Jean-Luc Wietor, Jeremy K. M. Sanders, Sijbren Otto Chemical Reviews, 2006, 106, 3652-3711 and Rowan et al. Angewandte Chemie International Edition, 2002, 41, 898-952.]. Dynamic covalent chemistry describes reversible chemical reactions which are under equilibrium control. The final product distribution in the thermodynamic minimum depends on the relative stabilities of the final products. Applications of dynamic covalent chemistry can be found in many self-organizing systems, such as host-guest interactions, covalent organic frameworks, dynamic combinatorial libraries, drug discovery, polymeric materials. To give an example, two imines can react in an exchange reaction. Therefore, imines have been widely applied in dynamic covalent chemistry:

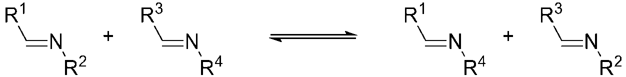

Currently, dynamic covalent chemistry is limited to the few reactions which are suited for the exchange. These are mainly: Aldol reaction, Diels-Alder reaction, Metathesis of C-C double and triple bonds, and the exchange of boronic and carboxylic esters, imines, aminals, disulfides, and boronic acid anhydrides. Importantly, dynamic covalent chemistry has not focused on spiropyrans yet.

### Photoinitiators

An overview on photoinitiators with terms and definitions can be found in [Green, Industrial Photoinitiators A Technical Guide, CRC Press, 2010; Fouassier and Lalevée, Photoinitiators Structures, Reactivity and Applications in Polymerization, Wiley VCH, 2021]. Curing of a material refers to the polymerization of monomers. Depending on the mechanism of the polymerization several functional groups have been found to be suitable monomers, which include but are not limited to acrylates, methacrylates, thiol + ene, epoxides, oxiranes, oxetanes, or vinylethers. Photoinitiators start the polymerization upon irradiation with light. The polymerization can be conducted in a radical fashion, cationic, anionic, or metal catalyzed. Photoinitiators are either type 1, where a single compound can start the polymerization or type 2, where a co-initiator is required. Type 1 photoinitiators can undergo homolytic (radical) or heterolytic bond cleavage (cationic/anionic) to form the reactive species. **In** the following reactions further species can be produced which can cause a different nature of the polymerization, e.g. an amine radical is initially produced by homolytic bond cleavage, which than abstracts a hydrogen atom from the environment to form an amine and start a base mediated polymerization. Type 2 photoinitiators can start the polymerization by two different mechanisms, where the first is hydrogen abstraction from a co-initiator, which produces two radicals and can cause further reactive species from follow up reactions. The second mechanism is a photoredox reaction, where an electron is transferred between co-initiator and photoinitiator. The photoredox reaction can be proceeded by further proton transfer reactions to produce radicals or other follow up reactions which result in further reactive species such as acids, bases or radicals. Several structural motives are known to initiate a polymerization and examples of the most important ones are shown below.

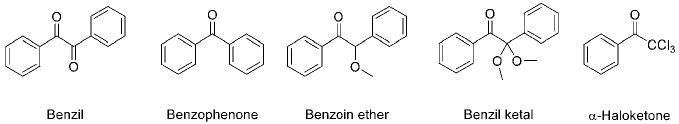

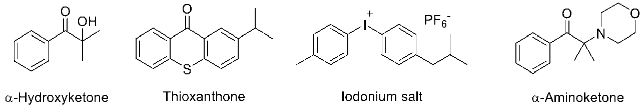

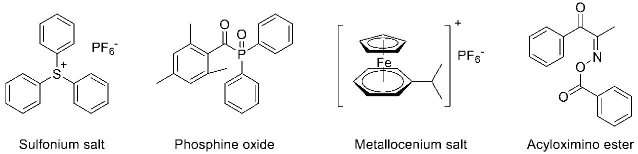

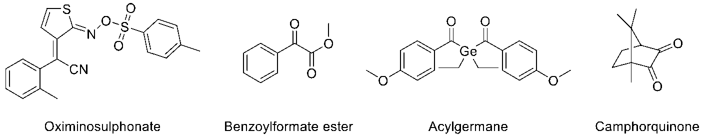

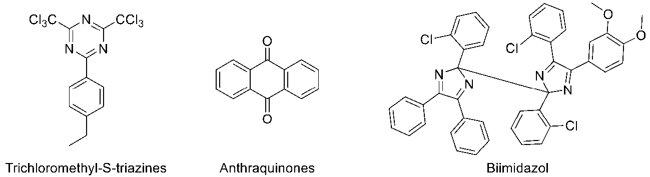

### Additive manufacturing/ 3D printing

Additive manufacturing or 3D printing can be performed with various techniques and materials and allow to produce customized parts. Compared to injection molding, additive manufacturing enables the formation of more complex structures, saving material and giving the opportunity to construct specialized parts for machines with superior performance. However, most of the additive manufacturing techniques rely on a layer by layer build sequence. Therefore, the object is sliced into many layers and one layer is printed after the other. Consequently, certain limitations are inherent, which is the necessity for support structures to prevent overhanging parts from falling apart. Usually, several mechanical steps are involved between printing of the layers, which lead to long production times. The starting material for a print must be in a form to allow for the mechanical operations without destruction of the object, which restricts starting materials to certain powder sizes or viscosities.

To overcome the limitations of classic additive manufacturing, volumetric printing has been proposed as a solution. In such an approach the resin is in a container and by the action of light curing does not happen on the walls of the container but inside the volume. This removes the necessity of support structures, allows for resins of low and high viscosity, allows the formation of soft objects, and does not suffer from the inhibition by oxygen or water at the surface.

To overcome the issues of two photon polymerization and tomographic reconstruction, a dual color photoinitiating system has been developed (xolography). This requires a photoinitiator which upon irradiation with light of a first wavelength λ1 is transformed from its thermodynamically stable ground state form A into a metastable ground state species B. When species B absorbs light of the second wavelength λ2, it can initiate a polymerization reaction via form C. Form C is an excited state of B, which produces radicals, cations or anions by further reaction with or without a co-initiator. In the sense of this invention the terms first wavelength, second wavelength, and third wavelength can refer to a range of wavelengths. For usage in a volumetric printing approach a back reaction from B to A must be possible to avoid hardening of unwanted areas. The back reaction from B to A can be triggered thermally or by irradiation with a third wavelength λ3.

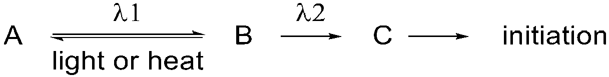

The dual color volumetric printing technology described above is known as xolography. Dual color photoinitiating systems suitable for this approach can be based on spiropyrans, which bear a carbonyl moiety [Garmshausen et al., WO2020245456A1]. The dual color photoinitiators described therein are synthesized by the reaction of a 2-methyleneindoline and a salicylaldehyde which maybe post-functionalized in subsequent steps.

JP H0375127A by Kenji and Ichiro claimed three photoswitches for volumetric printing. The molecules described therein are known for their photochromic properties, but do not show selective curing, where beams of both wavelengths would intersect as has been shown by Neckers [US005230986A, column 6, line 27-35]. Even if they were able to initiate with dual color irradiation, they show a slow thermal reverse reaction from B to A, which makes them unsuitable in volumetric printing applications. The spiropyran described therein is synthesized from a 2-methyleneindoline and a salicylaldehyde.

US005230986A by Neckers disclosed iodinated benzospiropyrans with suitable co-initiators as two photon radical photoinitiators with the following structure, where at least one of the two substituents X₁ and X₃ is an iodine:

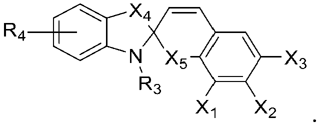

The disclosed molecules are of limited efficiency for photopolymerization, which makes high concentrations necessary [Lee, Neckers, Chem. Mater. 1991, 3, 852-858 and Lee, Neckers, Chem. Mater. 1991, 3, 858-864]. Consequently, the penetration depth of the light is limited and the volume suitable for printing cannot exceed 2 mm as has been shown by the same authors [Lee, Neckers, Chem. Mater. 1991, 3, 858-864, Figures 12 and 13]. The iodo substituent acts as a triplet sensitizer on the spiropyran form, so that the unwanted side reaction from irradiation using the first wavelength alone is dominant, where the spiropyran causes the photo redox reaction and hence formation of radicals, without isomerizing to the merocyanine. Iodinated benzospiropyrans are further limited by a slow thermal back reaction in more viscous media which makes them unsuitable for volumetric printing. The disclosed molecules are all synthesized via condensation of a 2-methyleneindoline and a salicylaldehyde.

Two other spiropyran-based two component systems have been reported for recording holograms [Jeudy, Robillard, Opt. Commun. 1975, 13, 25-28; Ichimura, Sakuragi, J. Polym. Sci. Polym. Lett, 1988, 26 185-189]. However, both are not suited for volumetric printing, since on the one hand the thermal back reaction in both cases is too slow for a commercially successful application and on the other hand both cause substantial polymerization with the first wavelength alone [Lee, Neckers, Chem. Mater. 1991, 3, 858-864]. In both publications the molecules are synthesized from the condensation of a 2-methyleneindoline and a salicylaldehyde.

The reaction of a spiropyran with 3,5-dinitrosalicylaldehyde is proposed to exchange the salicylaldehyde moiety without analytical proof [Bertelson, Techniques of Chemistry, Volume III, Photochromism, Chapter 3]. The reaction is further proposed to serve as a protecting strategy for salicylaldehydes. When the reaction is carried out as described by Bertelson, precipitation occurs accordingly. While this precipitate is claimed to be the exchanged spiropyran, analysis reveals, that 3,5-dinitrosalicylaldehyde acts as an acid in the mixture, protonating the original spiropyran and leading to precipitation of the protonated spiropyran without any exchange. Furthermore, the acid is found to catalyze an Aldol condensation, in cases where the spiropyran carries an acyl functionality capable of undergoing this reaction, such as acetyl. Other acid sensitive functionalities show decomposition when heated with 3,5-dinitrosalicylaldehyde.

Spiropyran has been proposed as a protecting group for salicylaldehydes, which can be cleaved using strong oxidants, such as potassium permanganate, sodium periodate, and ozone [Young Jin Cho et al. Tetrahedron Letters, 2000, 41, 3915-3917]. The oxidant causes the formation of salicylic acid derivatives and other side products, resulting in limited applicability. Furthermore, the protecting group cannot be recovered using this approach.

### Summary of the invention

### Problem to be solved

The problems to be solved and shortcomings are stated in the background of the art. Especially, spiropyrans which may be of interest for the application as photoinitiators, may not be accessible by the conventional synthetical methods stated above. Furthermore, some methods of the prior art may provide spiropyrans, in only low yield, low conversion, with high impurities and, thus, with only low efficiency. As a consequence, spiropyrans that can be used as photoinitiators, especially dual color photoinitiators, are only limited accessible, so that the provision of new photoinitiators, especially dual color photoinitiators which are crucial for the development of volumetric printing, is hampered and needs to be overcome. Another problem to be solved is to overcome the limitations of functionalizing a salicylaldehyde or 2-methyleneindoline efficiently, so that new substitution patterns of salicylaldehyde may become accessible for the synthesis of photoinitiators.

### Solution to the problem

The problem of the prior art is solved by the provision, the preparation, and the use of dual color photoinitiators according to the present disclosure, especially by the process for the manufacture of a spiropyran according to independent claim 1 and by the spiropyran according to independent claim 16, the process for the manufacture of a spiropyran according to dependent claims 6, or 12, the manufacture of a precursor of formula (2) according to claim 14, and a process for locally polymerizing a starting material according to claims 30 to 32.

Especially, it may be the object of the present invention to provide a synthetic method for the formation of a spiropyran structural motif. The provided technology shall be applicable to the fields of spiropyrans and/or dynamic covalent chemistry in general and to dual color photoinitiators in particular. More particular, it may be the object of the present invention to overcome limitations of the currently available dual color photoinitiators by providing spiropyrans. More particular a method for the synthesis of photoinitiators, which are spiropyrans, may be provided, which are not or only with difficulty accessible in the previously described synthetic pathways. The thereby provided photoinitiators cause curing of photopolymerizable formulations upon irradiation with two different wavelengths and may be used for volumetric printing (xolography).

The first aspect of the invention is a process for the manufacture of a spiropyran represented by the following formula (1): 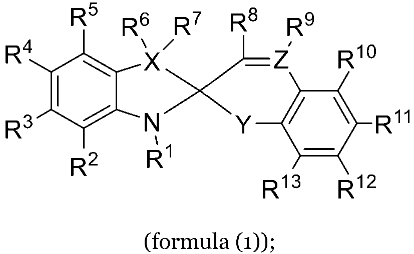 the process comprising the steps of
- providing a precursor, wherein the precursor is a spiropyran represented by the following formula (2): 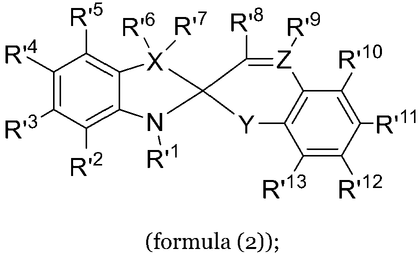
- providing a reactant, wherein the reactant is an indolenium salt represented by the following formula (3): 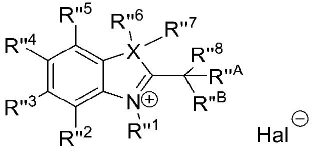 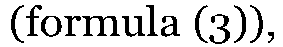
   or the corresponding 2-methyleneindoline compound; or
   a salicylaldehyde represented by the following formula (4): 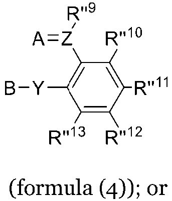
   a spiropyran represented by the following formula 5: 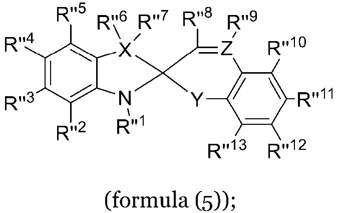
- optionally pre-activating the precursor
- providing a reaction mixture comprising the precursor or optionally a preactivated precursor, and the reactant to obtain said spiropyran of formula (1);

wherein the spiropyran, the precursor, and the reactant are different from each other;
wherein if the reactant is an indolenium salt of formula (3), the obtained spiropyran of formula (1) is represented by the following formula (1A): 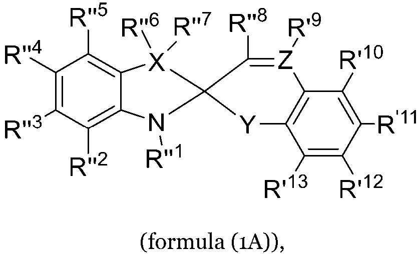
wherein R"¹ to R"⁸ of formula (1A) are independently the same as R"¹ to R"⁸ of formula (3) and R'⁹ to R'¹³ of formula (1A) are independently the same as R'⁹ to R'¹³ of formula (2);
wherein if the reactant is a salicylaldehyde of formula (4), the obtained spiropyran of formula (1) is represented by the following formula (1B): 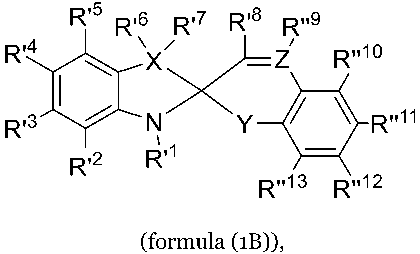
wherein R'¹ to R'⁸ of formula (1B) are independently the same as R'¹ to R'⁸ of formula (2) and R"⁹ to R"¹³ of formula (1B) are independently the same as R"⁹ to R"¹³ of formula (4);
wherein if the reactant is a spiropyran of formula (5),
the obtained spiropyran of formula (1) is represented by formula (1A), wherein R"¹ to R"⁸ of formula (1A) are independently the same as R"¹to R"⁸ of formula (5) and R'⁹ to R'¹³ of formula (1A) are independently the same as R'⁹ to R'¹³ of formula (2), or
the obtained spiropyran of formula (1) is represented by formula (1B), wherein R'¹ to R'⁸ of formula (1B) are independently the same as R'¹ to R'⁸ of formula (2) and R"⁹ to R"¹³ of formula (1B) are independently the same as R"⁹ to R"¹³ of formula (5);
wherein X is selected from S, C, or N; if X is S, then R⁶, R⁷, R'⁶, R'⁷, R"⁶, R"⁷ may not be present accordingly; if X is N, then R⁷, R'⁷, R"⁷ may not be present accordingly,
wherein Y is selected from O, S, or N; where Y is N, the substituent contains the atoms necessary to complete a cyclic structure with R¹³ selected from the group consisting of benzimidazole, indoline, indole, dihydroquinoline, and tetrahydroquinoline;
wherein Z is selected from N or C;
wherein if present A is selected from O, S, or Se;
wherein if present B is selected from H or D;
wherein if present Hal⁻ is a halogen anion or an anionic compound;
wherein if present R¹ to R¹³, R'¹ to R'¹³, and R"¹ to R"¹³ are independently selected from the group consisting of H; D; halogen; NO₂; CN; OH; SH; substituted or unsubstituted C₁-C₂₀-alkyl; substituted or unsubstituted C₃-C₂₀-cycloalkyl; substituted or unsubstituted C₆-C₄₈-aryl; substituted or unsubstituted C₂-C₄₂-heteroaryl; substituted or unsubstituted C₂-C₄₉-alkyl acyl; substituted or unsubstituted C₂-C₄₉-aryl acyl; substituted or unsubstituted C₁-C₂₀-alkoxy; substituted or unsubstituted C₆-C₄₈-aryloxy; NH₂; substituted or unsubstituted C₁-C₂₀-alkyl ester; substituted or unsubstituted C₆-C₄₈-aryl ester; substituted or unsubstituted C₁-C₂₀ alkyl amide; substituted or unsubstituted C₆-C₄₈-aryl amide; NR'₂; SiR'₃; -O-SiR'₃,
wherein R' is independently selected from the group consisting of substituted or unsubstituted C₁-C₂₀-alkyl and substituted or unsubstituted C₆-C₄₈-aryl, two R' may form a ring structure; substituted or unsubstituted carboxylic acids and salts thereof; substituted or unsubstituted sulfonic acids and salts thereof; substituted or unsubstituted sulfonic esters; substituted or unsubstituted sulfonic amides; formyl; ether, thioether; carbonate; carbonate ester; sulfates; boronic acids; boronic esters; phosphonic acids; phosphonic esters; phosphines; phosphates; peroxycarbonic acids; thiocarbonic acids; sulfinic acids; sulfinic esters; sulfonates; thiolesters, sulfoxides; sulfones; alkylsulfones; hydrazides; thioaldehydes; ketones; thioketones; oximes; hydrazines; nitroso; azo; diazo; diazonium; isocyanides; cyanate; isocyanate; thiocyanate; isothiocyanate; hydroperoxide; peroxide; acetals; ketal; orthoester; orthocarbonate esters; ammonium; imines; imides; azide; nitrate; isonitrile; nitrosoxy; substituted or unsubstituted carbamates; substituted or unsubstituted ethers; substituted or unsubstituted polyether carbamates; substituted or unsubstituted arylazo; substituted or unsubstituted C₂-C₂₀-alkynyl and substituted or unsubstituted C₂-C₂₀-alkenyl;
wherein the one or more substituents if present in one or more of R¹ to R¹³, are independently selected from the group consisting of D; halogen; NO₂; CN, C₂-C₄₉-alkyl acyl; substituted or unsubstituted C₁-C₂₀-alkoxy; substituted or unsubstituted C₆-C₄₈-aryloxy; substituted or unsubstituted C₂-C₄₉-aryl acyl; (meth)acrylate; tosyl; sulfonic acid or salts thereof; carboxylic acid or salts thereof; boronic acid or salts thereof; phosphonic acid or salts thereof; NR'₃⁺, wherein R' is independently selected from the group consisting of H, D, substituted or unsubstituted C₁-C₂₀-alkyl and substituted or unsubstituted C₆-C₄₈-aryl, two R' may form a ring structure; NH₂; and OH;
wherein if present two adjacent groups of R² to R⁵, R'² to R'⁵, R"² to R"⁵, R¹⁰ to R¹³; R'¹⁰ to R'¹³ and R"¹⁰ to R"¹³ may be independently linked to each other to form a fused ring structure; and
wherein if present R"⁴ to R"^{B} are independently selected from H and D.

A second aspect of the embodiment is a process for the manufacture of a precursor represented by the following formula (2): 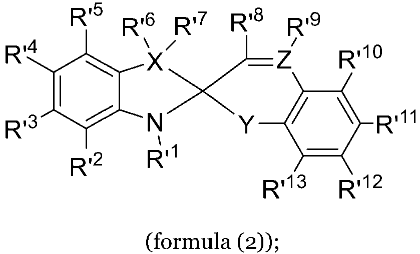
wherein R'¹, R'⁶ to R'⁹ are independently selected from the group consisting of H, D, substituted or unsubstituted C₁-C₁₀-alkyl, preferably methyl; substituted or unsubstituted C₆-C₃₂-aryl, preferably phenyl; substituted or unsubstituted C₂-C₂₀-alkynyl and substituted or unsubstituted C₂-C₂₀-alkenyl and benzyl;
X is C;
Z is C;
Y is O;
R'² to R'⁵ and R'¹⁰ to R'¹³ are independently selected from the group consisting of H, D, F, Cl, Br, substituted or unsubstituted C₁-C₁₀-alkyl, substituted or unsubstituted C₆-C₃₂-aryl, substituted or unsubstituted C₁-C₂₀-alkoxy, substituted or unsubstituted C₆-C₄₈-aryloxy, CF₃, CN; wherein two adjacent groups of may be linked to each other to form a fused ring structure, preferably, a fused aromatic C₆-ring; and a substituent of the following formula: 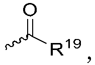
wherein R¹⁹ is defined as it is defined in the description;
wherein at least one of R'² to R'⁵ and R'¹⁰ to R'¹³ is a substituent of the following formula: 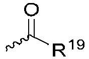
the process comprising the steps of:
   - providing a reactant, wherein the reactant is a spiropyran represented by the following formula (2A): 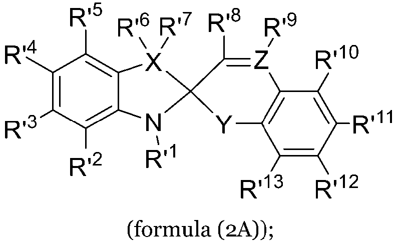
      wherein at least one of R'² to R'⁵ and R'¹⁰ to R'¹³ is a halogen atom selected from the group consisting of Cl, Br and I; and the rest of the substituents are the same as in the precursor of formula (2);
         - reacting the halogen atom of the reactant in a metal-halogen exchange reaction, preferably in a metal-halogen exchange reaction with an organolithium reagent or Grignard reagent, to obtain a metal-spiropyran species,
         - subsequently reacting the metal-spiropyran species with a Weinreb-amide of the following formula: 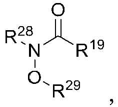
            wherein R²⁸ and R²⁹ are selected from the group consisting of substituted or unsubstituted C₁-C₁₀-alkyl; substituted or unsubstituted C₆-C₃₂-aryl;
            substituted or unsubstituted C₂-C₂₀-alkynyl and substituted or unsubstituted C₂-C₂₀-alkenyl, preferably are R²⁸ and R²⁹ are each methyl,
            to obtain the precursor of formula (2), wherein preferably the precursor of formula (2) is obtained after an acidic aqueous work-up.

A third aspect of the invention is a spiropyran represented by the following formula (1): 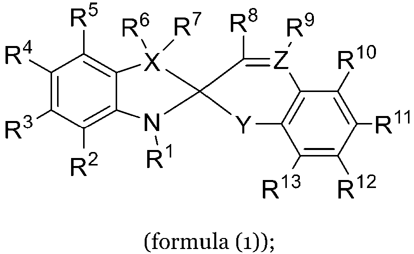
wherein X is selected from S, C, or N; if X is S, then R⁶, R⁷, R'⁶, R'⁷, R"⁶, R"⁷ may not be present accordingly; if X is N, then R⁷, R'⁷, R"⁷ may not be present accordingly,
wherein Y is selected from O, S, or N; where Y is N, the substituent contains the atoms necessary to complete a cyclic structure with R¹³ selected from the group consisting of benzimidazole, indoline, indole, dihydroquinoline, and tetrahydroquinoline;
wherein Z is selected from N or C;
wherein R¹ to R¹³ are independently selected from the group consisting of H; D; halogen; NO₂; CN; OH; SH; CF₃; substituted or unsubstituted C₁-C₂₀-alkyl; substituted or unsubstituted C₃-C₂₀-cycloalkyl; substituted or unsubstituted C₆-C₄₈-aryl; substituted or unsubstituted C₂-C₄₂-heteroaryl; substituted or unsubstituted C₂-C₄₉-alkyl acyl; substituted or unsubstituted C₂-C₄₉-aryl acyl; substituted or unsubstituted C₁-C₂₀-alkoxy; substituted or unsubstituted C₆-C₄₈-aryloxy; NH₂; substituted or unsubstituted C₁-C₂₀-alkyl ester; substituted or unsubstituted C₆-C₄₈-aryl ester; substituted or unsubstituted C₁-C₂₀ alkyl amide; substituted or unsubstituted C₆-C₄₈-aryl amide; NR'₂; SiR'₃; -O-SiR'₃, wherein R' is independently selected from the group consisting of substituted or unsubstituted C₁-C₂₀-alkyl and substituted or unsubstituted C₆-C₄₈-aryl, two R' may form a ring structure; substituted or unsubstituted carboxylic acids and salts thereof; substituted or unsubstituted sulfonic acids and salts thereof; substituted or unsubstituted sulfonic esters; substituted or unsubstituted sulfonic amides; formyl; ether; thioether; carbonate; carbonate ester; sulfates; boronic acids; boronic esters; phosphonic acids; phosphonic esters; phosphines; phosphates; peroxycarbonic acids; thiocarbonic acids; sulfinic acids; sulfinic esters; sulfonates; thiolesters, sulfoxides; sulfones; alkylsulfones; hydrazides; thioaldehydes; ketones; thioketones; oximes; hydrazines; nitroso; azo; diazo; diazonium; isocyanides; cyanate; isocyanate; thiocyanate; isothiocyanate; hydroperoxide; peroxide; acetals; ketal; orthoester; orthocarbonate esters; ammonium; imines; imides; azide; nitrate; isonitrile; nitrosoxy; substituted or unsubstituted carbamates; substituted or unsubstituted ethers; substituted or unsubstituted polyether carbamates; substituted or unsubstituted arylazo; substituted or unsubstituted C₂-C₂₀-alkynyl and substituted or unsubstituted C₂-C₂₀-alkenyl; wherein two adjacent groups of may be linked to each other to form a fused ring structure, preferably, a fused aromatic C₆-ring;
wherein the one or more substituents, if present in one or more of R¹to R¹³, are independently selected from the group consisting of D; halogen; NO₂; CN, C₂-C₄₉-alkyl acyl; substituted or unsubstituted C₁-C₂₀-alkoxy; substituted or unsubstituted C₆-C₄₈-aryloxy; substituted or unsubstituted C₂-C₄₉-aryl acyl; (meth)acrylate; tosyl; sulfonic acid or salts thereof; carboxylic acid or salts thereof; boronic acid or salts thereof; phosphonic acid or salts thereof; NR'₃⁺, wherein R' is independently selected from the group consisting of H, D, substituted or unsubstituted C₁-C₂₀-alkyl and substituted or unsubstituted C₆-C₄₈-aryl, two R' may form a ring structure; NH₂; and OH; wherein if present two adjacent groups of R¹⁰ to R¹³, and R² to R⁵ may be independently linked to each other to form a fused ring structure;
   and
wherein at least one substituent for R² to R⁵ and R¹⁰ to R¹³ is selected from one of the groups consisting of chlorine; bromine; iodine; formyl; carbonate; carbonate ester; ester; amide; CF₃; substituted or unsubstituted C₂-C₄₉-alkyl acyl; substituted or unsubstituted C₂-C₄₉-aryl acyl; ketone; acyl; oxime; aldehyde, NO₂; CN; (meth)acrylate; sulfones; alkylsulfones; sulfonamides; SO₂Me; SO₂NH₂; methoxy; and tosyl; or of the following formulae: 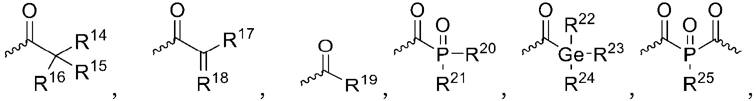 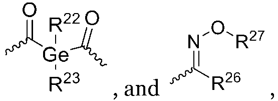
wherein R¹⁴ to R²⁷ are independently selected from the group consisting of H; D; halogen; NO₂; CN; OH; SH; CF₃; substituted or unsubstituted C₁-C₂₀-alkyl; substituted or unsubstituted C₃-C₂₀-cycloalkyl; substituted or unsubstituted C₆-C₄₈-aryl; substituted or unsubstituted C₂-C₄₂-heteroaryl; substituted or unsubstituted C₂-C₄₉-alkyl acyl; substituted or unsubstituted C₂-C₄₉-aryl acyl; substituted or unsubstituted C₁-C₂₀-alkoxy; substituted or unsubstituted C₆-C₄₈-aryloxy, and NH₂; substituted or unsubstituted C₁-C₂₀-alkyl ester; substituted or unsubstituted C₆-C₄₈-aryl ester; substituted or unsubstituted C₁-C₂₀ alkyl amide; substituted or unsubstituted C₆-C₄₈-aryl amide; NR'₂, SiR'₃, -O-SiR'₃ wherein R' is independently selected from the group consisting of substituted or unsubstituted C₁-C₂₀-alkyl and substituted or unsubstituted C₆-C₄₈-aryl, two R' may form a ring structure; substituted or unsubstituted carboxylic acids and salts thereof; substituted or unsubstituted sulfonic acids and salts thereof; substituted or unsubstituted sulfonic esters; substituted or unsubstituted sulfonic amides; formyl; ether, thioether; carbonate; carbonate ester; sulfates; boronic acids; boronic esters; phosphonic acids; phosphonic esters; phosphines; phosphates; peroxycarbonic acids; thiocarbonic acids; sulfinic acids; sulfinic esters; sulfonates; thiolesters, sulfoxides; sulfones; alkylsulfones; hydrazides; thioaldehydes; ketones; thioketones; oximes; hydrazines; nitroso; azo; diazo; diazonium; isocyanides; cyanate; isocyanate; thiocyanate; isothiocyanate; hydroperoxide; peroxide; acetals; ketal; orthoester; orthocarbonate esters; ammonium; imines; imides; azide; nitrate; isonitrile; nitrosoxy; substituted or unsubstituted carbamates; substituted or unsubstituted ethers; substituted or unsubstituted polyether carbamates; substituted or unsubstituted arylazo; substituted or unsubstituted C₂-C₂₀-alkynyl and substituted or unsubstituted C₂-C₂₀-alkenyl;
wherein the one or more substituents, if present in one or more of R¹⁴-R²⁷, are independently selected from the group consisting of D; halogen; NO₂; CN, C₂-C₄₉-alkyl acyl; substituted or unsubstituted C₁-C₂₀-alkoxy; substituted or unsubstituted C₆-C₄₈-aryloxy; substituted or unsubstituted C₂-C₄₉-aryl acyl; (meth)acrylate; tosyl; sulfonic acid or salts thereof, carboxylic acid or salts thereof, boronic acid or salts thereof, phosphonic acid or salts thereof, NR'₃⁺, wherein R' is independently selected from the group consisting of H, D, substituted or unsubstituted C₁-C₂₀-alkyl and substituted or unsubstituted C₆-C₄₈-aryl, two R' may form a ring structure; NH₂; and OH; and R¹⁵ and R¹⁶ may be linked to each other to form a unsubstituted or substituted ring structure,
   and
wherein at least one other substituent for R² to R⁵ and R¹⁰ to R¹³ is selected from the group consisting of chlorine; bromine; iodine; formyl; carbonate; carbonate ester; ester; amide; CF₃; substituted or unsubstituted C₂-C₄₉-alkyl acyl; substituted or unsubstituted C₂-C₄₉-aryl acyl; ketone; acyl; oxime; aldehyde, NO₂; CN; (meth)acrylate; sulfones; alkylsulfones; sulfonamides; SO₂Me; SO₂NH₂; methoxy; and tosyl;
   and/or
wherein at least one other substituent for R² to R⁵ and R¹⁰ to R¹³ is selected from sulfonic acid or salts thereof; carboxylic acid or salts thereof; boronic acid or salts thereof; phosphonic acid or salts thereof; NR'₃⁺, wherein R' is independently selected from the group consisting of H, D, substituted or unsubstituted C₁-C₂₀-alkyl and substituted or unsubstituted C₆-C₄₈-aryl, two R' may form a ring structure; substituted or unsubstituted sulfonic esters; substituted or unsubstituted sulfonic amides; phosphonic esters; phosphines; phosphates; sulfinic acids; sulfinic esters; sulfonates; sulfoxides; sulfones; alkylsulfones; oximes; isocyanides; cyanate; isocyanate; thiocyanate; isothiocyanate; ammonium; substituted or unsubstituted carbamates; (meth)acrylate; tosyl; NH₂; and OH.
Furthermore, the spiropyrans of the present invention may be photoinitiators, preferably dual color photoinitiators, or used as such. Further embodiments are subject to dependent claims.

In a further aspect of the present invention, a formulation may contain the spiropyran of formula (1), which may be a photoinitiator.

Another aspect of the present invention is a process for locally polymerizing a starting material by using a spiropyran according to the present invention as a photoinitiator and irradiating the spiropyran with two light sources of different wavelengths.

One aspect of the present invention is a process for locally polymerizing a starting material
by dual color photopolymerization, comprising:
- providing a polymerizable starting material containing photoinitiator molecules wherein the photoinitiator molecule is a spiropyran according to the present invention, which can be converted by sequential optical excitation into a reactive state in which the photoinitiator molecules locally trigger polymerization of the starting material; and
- photopolymerizing the starting material in a local volume by irradiating light of a first wavelength and light of a second wavelength, different from the first wavelength, into the local volume, whereby in the local volume
   - the photoinitiator molecules are converted, due to the absorption of the light of the first wavelength, from an initial state in which the photoinitiator molecules substantially do not absorb the light of the second wavelength, into an intermediate state with changed optical properties compared to the initial state, such that the photoinitiator molecules in the intermediate state absorb the light of the second wavelength; and
- the photoinitiator molecules are transferred from the intermediate state to the reactive state due to the absorption of the light of the second wavelength, which triggers the polymerization locally; and/ or
- the photoinitiator molecules may be transferred from the intermediate state to the initial state spontaneously in a thermal reaction.

One further aspect of the present invention is a process for the formation of shaped body by
dual color photopolymerization, comprising:
- providing a polymerizable starting material containing photoinitiator molecules wherein the photoinitiator molecule is a spiropyran as it is defined in the present invention, which can be converted by sequential optical excitation into a reactive state in which the photoinitiator molecules locally trigger polymerization of the starting material; and
   - photopolymerizing the starting material in a local volume by irradiating light of a first wavelength and light of a second wavelength, different from the first wavelength, into the local volume, whereby in the local volume
   - the photoinitiator molecules are converted, due to the absorption of the light of the first wavelength, from an initial state in which the photoinitiator molecules substantially do not absorb the light of the second wavelength, into an intermediate state with changed optical properties compared to the initial state, such that the photoinitiator molecules in the intermediate state absorb the light of the second wavelength; and
- the photoinitiator molecules are transferred from the intermediate state to the reactive state due to the absorption of the light of the second wavelength, which triggers the polymerization locally; and
- the photoinitiator molecules may be transferred from the intermediate state to the initial state spontaneously in a thermal reaction.

Yet another aspect of the present invention is a process for locally polymerizing a starting
material by dual color photopolymerization and forming a shaped body, comprising the following steps:
- providing a container which is at least partially filled with a polymerizable starting material containing photoinitiator molecules, which can be converted by sequential optical excitation into a reactive state in which the photoinitiator molecules locally trigger polymerization of the starting material; and
   - photopolymerizing the starting material in the container by irradiating light of a first wavelength and light of a second wavelength, different from the first wavelength, into the container, whereby in the container
   - the photoinitiator molecules are converted, due to the absorption of the light of the first wavelength, from an initial state in which the photoinitiator molecules substantially do not absorb the light of the second wavelength, into an intermediate state with changed optical properties compared to the initial state, such that the photoinitiator molecules in the intermediate state absorb the light of the second wavelength; and
- the photoinitiator molecules are transferred from the intermediate state to the
   reactive state due to the absorption of the light of the second wavelength, which triggers the polymerization locally to form the shaped body; and
   - wherein the initial state of the photoinitiator molecules in the photopolymerizable material has an extinction coefficient which is lower than 5000 Lmol⁻¹cm⁻¹.

In yet another aspect, the present invention discloses a process for locally polymerizing a
starting material by dual color photopolymerization and forming a shaped body, comprising the following steps:
- providing a container which is at least partially filled with a polymerizable starting material containing photoinitiator molecules, which can be converted by sequential optical excitation into a reactive state in which the photoinitiator molecules locally trigger polymerization of the starting material; and
   - photopolymerizing the starting material in a container by irradiating light of a first wavelength and light of a second wavelength, different from the first wavelength, into the container, whereby in the container
   - the photoinitiator molecules are converted, due to the absorption of the light of the first wavelength, from an initial state in which the photoinitiator molecules substantially do not absorb the light of the second wavelength, into an intermediate state with changed optical properties compared to the initial state, such that the photoinitiator molecules in the intermediate state absorb the light of the second wavelength; and
- the photoinitiator molecules are transferred from the intermediate state to the
   reactive state due to the absorption of the light of the second wavelength, which triggers the polymerization locally to form a shaped body;
   - wherein the photopolymerizable material has an absorbance at the first wavelength in a range between 1 and 0.07 absorbance units.

### Detailed description

### 1. General process for the manufacture of Spiropyran of formula (1)

The spiropyran of formula (1) may be obtained by a reversible exchange reaction.

Without being bound by any theory, the inventors provide a plausible mechanism of the present reversible exchange reaction in the following outlined scheme which may occur in the reaction mixture: A spiropyran opens to the merocyanine form. Water can undergo a Michael Addition to the merocyanine, which reacts further to the free salicylaldehyde and the 2-methyleneindoline. The 2-methyleneindoline is a strong nucleophile which can add to another merocyanine in a reversible fashion. In further steps, one 2-methyleneindoline is released from the adduct, leaving a new merocyanine which can close to the respective spiropyran. The thereby released 2-methyleneindoline can undergo a spiropyran condensation with salicylaldehyde which has been liberated earlier from a spiropyran (shown in the scheme below) or a different salicylaldehyde which has been added to the reaction mixture (not shown in the scheme below). Vice versa, the released salicylaldehyde can undergo a spiropyran condensation with 2-methyleneindoline which has been liberated earlier from a spiropyran (shown in the scheme below) or a different 2-methyleneindoline which has been added to the reaction mixture (not shown in the scheme below).

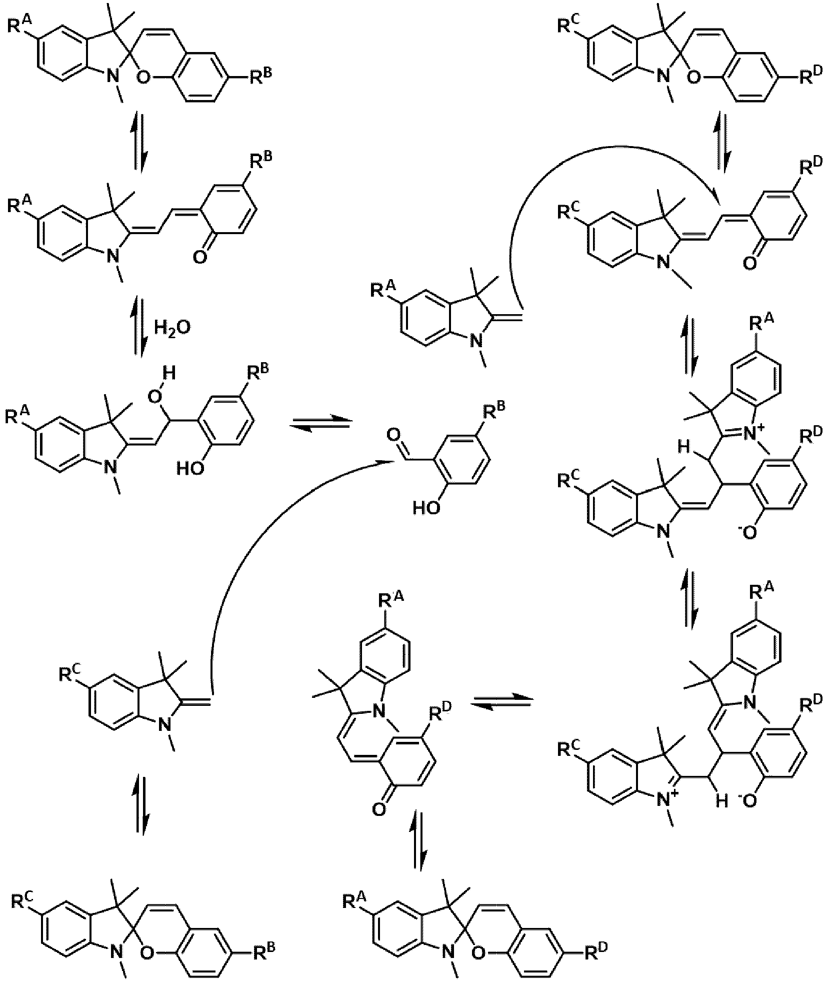

Many nucleophiles could undergo the described Michael Addition on the merocyanine. In some cases, it may be advantageous to treat the precursor spiropyran with a nucleophile, such as methylamine, and pre-activate it for the reaction (shown in scheme below). This activation may lead to the formation of the respective imine/iminium/salicylaldehyde and 2-methyleneindoline derivatives and may be more efficient than with water. The respective salicylaldehyde and 2-methyleneindoline may be separated, e.g. by basic extraction, prior to addition of the reactant.

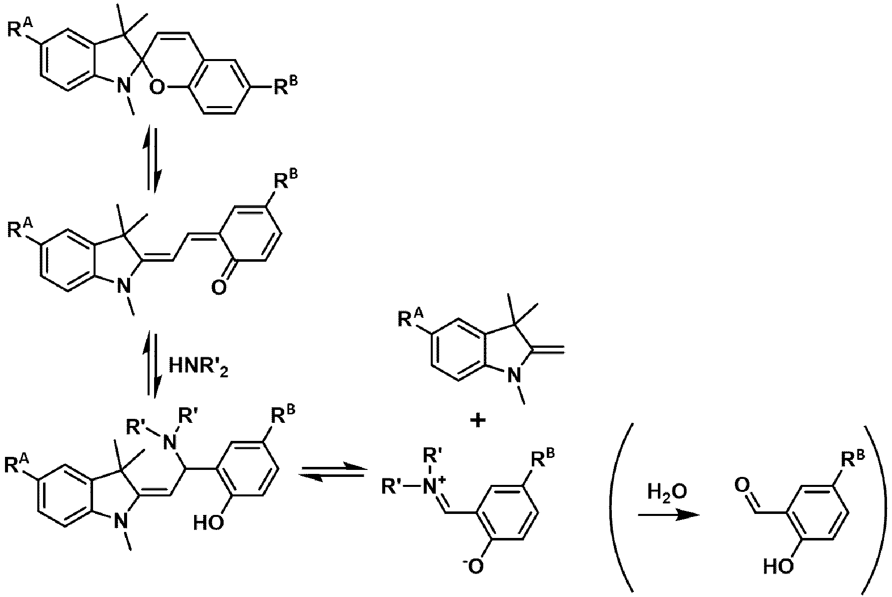

Another option to accelerate the exchange of two spiropyrans may be to add a catalytic amount of a 2-methyleneindoline, so that there may always be some nucleophile in slight excess present. In some embodiments, the efficiency of the reaction may be enhanced by substitution of the indole with an electron donating moiety and/or by substitution of the salicylaldehyde with an electron withdrawing moiety. Such a substitution pattern may facilitate the opening to the merocyanine and the attack of the 2-methyleneindoline on the merocyanine. In another embodiment, the equilibrium may be shifted towards the product side by substitution of R^{8'} in the precursor of formula (2) with an electron donating moiety, such as methyl. Such substituent may influence the basicity und nucleophilicity of the corresponding 2-methyleneindoline derivative.

Accordingly, a mixture of two or more spiropyrans may exchange their respective components which originate from the 2-methyleneindoline and salicylaldehyde, respectively. The spiropyrans may be dissolved in a suitable solvent and heated until the equilibrium may be reached. Starting from two spiropyrans, with different 2-methyleneindoline and salicylaldehyde components, four different spiropyrans in the mixture may be found, when the thermodynamic equilibrium may be reached. The number of possible spiropyrans may increase according to the possible combinations. From the number of starting spiropyrans originating from different 2-methyleneindolines I and different salicylaldehydes S, the maximum number of different derivatives in the mixture P may be deduced according to the formula P=S*I.

As stated above, in one aspect, a process for the manufacture of a spiropyran represented by the following formula (1): 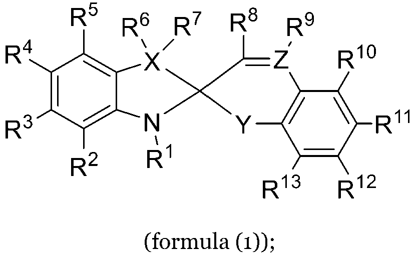 is provided. The process comprises the steps of
- providing a precursor, wherein the precursor is a spiropyran represented by the following formula (2): 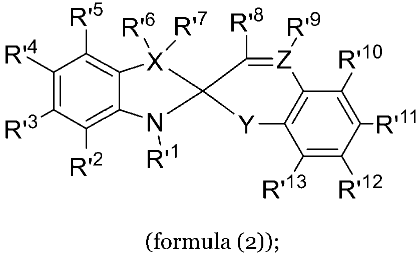
- providing a reactant, wherein the reactant is an indolenium salt represented by the following formula (3):

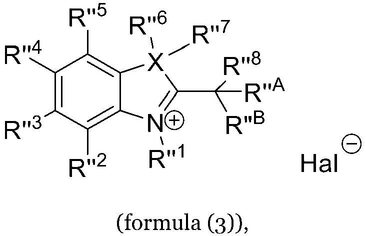

or the corresponding 2-methyleneindoline compound; or as an alternative
a salicylaldehyde represented by the following formula (4): 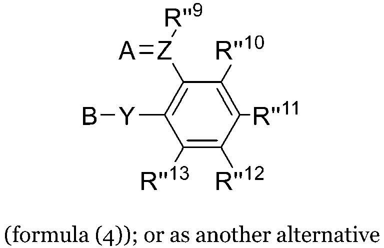
a spiropyran represented by the following formula 5:

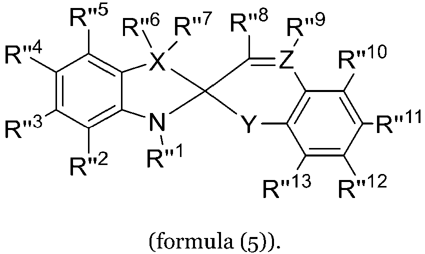

Optionally, the precursor is pre-activated. A reaction mixture comprising the precursor or optionally a preactivated precursor is provided, as well as the reactant to obtain said spiropyran of formula (1). Thereby, the spiropyran, the precursor, and the reactant are different from each other. If the reactant is an indolenium salt of formula (3), the obtained spiropyran of formula (1) is represented by the following formula (1A): 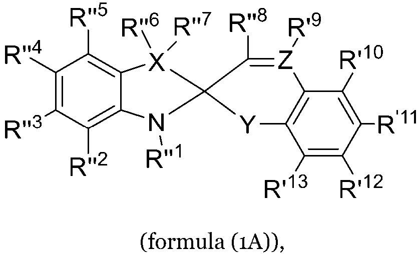 wherein R"¹ to R"⁸ of formula (1A) are independently the same as R"¹ to R"⁸ of formula (3) and R'⁹ to R'¹³ of formula (1A) are independently the same as R'⁹ to R'¹³ of formula (2);

If the reactant is a salicylaldehyde of formula (4), the obtained spiropyran of formula (1) is represented by the following formula (1B): 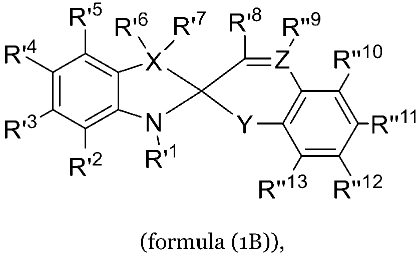 wherein R'¹ to R'⁸ of formula (1B) are independently the same as R'¹ to R'⁸ of formula (2) and R"⁹ to R"¹³ of formula (1B) are independently the same as R"⁹ to R"¹³ of formula (4).

In a further aspect of the invention, the present invention solves the problem of providing a methyleneindolin.

The skilled person is aware of that a side product of the spiropyran of formula (2) and a methyleneindoline of formula (3) may be a methyleneindoline of the following formula (3B):

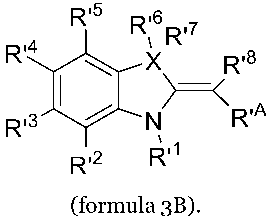

Therein, the substituents of R'¹, R'², R'³, R'⁴, R'⁵, R'⁶, R'⁷ and R'⁸, as well as X, are the same as in formula (2), from which the methyleneindoline of formula (3B) derives from. The methyleneindoline of (3B) may be protonated as the corresponding indolenium salt. In a preferred embodiment of methyleneindoline of formula (3B), X is C and / or R'^{A} is H. The skilled person will realize that the process for the manufacture of a spiropyran of the present invention may be also used to provide methyleneindolines, like methyleneindolines of formula (3B), that may have been challenging to access or may not have been accessible at all by alternative conventional synthetic routes. The methyleneindoline of formula (3B) may be used as a precursor for a different reaction, for example for the synthesis of another spiropyran.

In a further aspect of the invention, the present invention solves the problem of providing salicylaldehyde. The salicylaldehyde may contain substituents and / or a substituent pattern that may not be accessible by conventional synthetic routes.

The skilled person is aware of that a side product of the spiropyran of formula (2) and salicylaldehyde of formula (4) may be a salicylaldehyde of the following formula (4B):

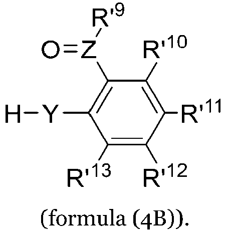

The salicylaldehyde of formula (4b) may be a side product, which may be obtained as a side product of spiropyran of formula (2) which may was pre-activated with a nucleophile, like an amine, as it is described further below.

Therein, the substituents of R'9, R'¹⁰, R'¹¹, R'¹² and R'¹³, as well as Y and Z, are the same as in formula (2), from which the salicylaldehyde of formula (4B) derives from. **In** a preferred embodiment of salicylaldehyde of formula (4B), Y is O, Z is C and / or R'⁹ is H. The salicylaldehyde of formula (4B) may be a compound wherein at least one of the substituents R'¹⁰, R'¹¹, R'¹² and R'¹³ are not directly accessible from salicylaldehyde. Consequently, the skilled person will realize that the process for the manufacture of a spiropyran of the present invention may be also used to provide salicylaldehydes, like salicylaldehydes of formula (4B), that may have been challenging to access or may not have been accessible at all by alternative conventional synthetic routes.

Such salicylaldehydes may be accessed by utilizing the spiropyran of formula (2) as a protecting group for the salicylaldehyde of formula (4B) through which substituents R'¹⁰, R'¹¹, R'¹² and R'¹³ can be modified by the methods known in the art. Subsequently, the desired salicylaldehyde of formula (4B) may be "deprotected" by reacting the spiropyran of formula (2) with a nucleophile, for example an amine as it is further specified under pre-activation of the precursor of formula (2) or salicylaldehyde of formula (4) by using the method to manufacture a spiropyran of formula (1B). The salicylaldehyde of formula (4B) may be used as a precursor for a different reaction, for example for the synthesis of another spiropyran.

If the reactant is a spiropyran of formula (5), the obtained spiropyran of formula (1) is represented by formula (1A), wherein R"¹ to R"⁸ of formula (1A) are independently the same as R"¹ to R"⁸ of formula (5) and R'⁹ to R'¹³ of formula (1A) are independently the same as R'⁹ to R'¹³ of formula (2), or
the obtained spiropyran of formula (1) is represented by formula (1B), wherein R'¹ to R'⁸ of formula (1B) are independently the same as R'¹ to R'⁸ of formula (2) and R"⁹ to R"¹³ of formula (1B) are independently the same as R"⁹ to R"¹³ of formula (5);
X is selected from S, C, or N; if X is S, then R⁶, R⁷, R'⁶, R'⁷, R"⁶, R"⁷ may not be present accordingly; if X is N, then R⁷, R'⁷, R"⁷ may not be present accordingly, preferably X is C.
Y is selected from O, S, or N; where Y is N, the substituent contains the atoms necessary to complete a cyclic structure with R¹³ selected from the group consisting of benzimidazole, indoline, indole, dihydroquinoline, and tetrahydroquinoline, preferably Y is O.
Z is selected from N or C, preferably Z is C.

If present A is selected from O, S, or Se, preferably A is O.

If present B is selected from H or D, preferably B is H.

If present Hal⁻ is a halogen anion or an anionic compound, preferably Hal⁻ is Cl⁻, Br⁻, I⁻, more preferably Hal⁻ is I⁻.

If present R¹ to R¹³, R'¹ to R'¹³, and R"¹ to R"¹³ are independently selected from the group consisting of H; D; halogen; NO₂; CN; OH; SH; substituted or unsubstituted C₁-C₂₀-alkyl; substituted or unsubstituted C₃- C₂₀-cycloalkyl; substituted or unsubstituted C₆-C₄₈-aryl; substituted or unsubstituted C₂-C₄₂-heteroaryl; substituted or unsubstituted C₂-C₄₉-alkyl acyl; substituted or unsubstituted C₂-C₄₉-aryl acyl; substituted or unsubstituted C₁-C₂₀-alkoxy; substituted or unsubstituted C₆-C₄₈-aryloxy; NH₂; substituted or unsubstituted C₁-C₂₀-alkyl ester; substituted or unsubstituted C₆-C₄₈-aryl ester; substituted or unsubstituted C₁-C₂₀ alkyl amide; substituted or unsubstituted C₆-C₄₈-aryl amide; NR'₂; SiR'₃; -O-SiR'₃, wherein R' is independently selected from the group consisting of substituted or unsubstituted C₁-C₂₀-alkyl and substituted or unsubstituted C₆-C₄₈-aryl, two R' may form a ring structure; substituted or unsubstituted carboxylic acids and salts thereof; substituted or unsubstituted sulfonic acids and salts thereof; substituted or unsubstituted sulfonic esters; substituted or unsubstituted sulfonic amides; formyl; ether, thioether; carbonate; carbonate ester; sulfates; boronic acids; boronic esters; phosphonic acids; phosphonic esters; phosphines; phosphates; peroxycarbonic acids; thiocarbonic acids; sulfinic acids; sulfinic esters; sulfonates; thiolesters, sulfoxides; sulfones; alkylsulfones; hydrazides; thioaldehydes; ketones; thioketones; oximes; hydrazines; nitroso; azo; diazo; diazonium; isocyanides; cyanate; isocyanate; thiocyanate; isothiocyanate; hydroperoxide; peroxide; acetals; ketal; orthoester; orthocarbonate esters; ammonium; imines; imides; azide; nitrate; isonitrile; nitrosoxy; substituted or unsubstituted carbamates; substituted or unsubstituted ethers; substituted or unsubstituted polyether carbamates; substituted or unsubstituted arylazo; substituted or unsubstituted C₂-C₂₀-alkynyl and substituted or unsubstituted C₂-C₂₀-alkenyl;

If in one or more of R¹ to R¹³ one or more substituents are present, they are independently selected from the group consisting of D; halogen; NO₂; CN, C₂-C₄₉-alkyl acyl; substituted or unsubstituted C₁-C₂₀-alkoxy; substituted or unsubstituted C₆-C₄₈-aryloxy; substituted or unsubstituted C₂-C₄₉-aryl acyl; (meth)acrylate; tosyl; sulfonic acid or salts thereof; carboxylic acid or salts thereof; boronic acid or salts thereof; phosphonic acid or salts thereof; NR'₃⁺, wherein R' is independently selected from the group consisting of H, D, substituted or unsubstituted C₁-C₂₀-alkyl and substituted or unsubstituted C₆-C₄₈-aryl, two R' may form a ring structure; NH₂; and OH.

If two adjacent groups of R² to R⁵, R'² to R'⁵, R"² to R"⁵, R¹⁰ to R¹³; R'¹⁰ to R'¹³ and R"¹⁰ to R"¹³ are present, they may be independently linked to each other to form a fused ring structure; and if R"^{A} to R"^{B} are present, the may be independently selected from H and D.

Preferably, at least one of R² to R⁵ and R¹⁰ to R¹³ in formula (1) is a substituent selected from the group consisting of chlorine; bromine; iodine; formyl; carbonate; carbonate ester; ester; amide; CF₃; substituted or unsubstituted C₂-C₄₉-alkyl acyl; substituted or unsubstituted C₂-C₄₉-aryl acyl; ketone; acyl; oxime; aldehyde, NO₂; CN; (meth)acrylate; sulfones; alkylsulfones; sulfonamides; SO₂Me; SO₂NH₂; methoxy; and tosyl; or one of the following formulae:

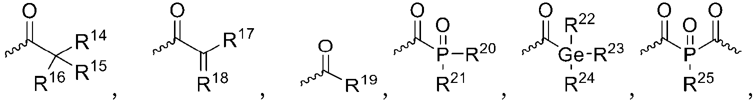

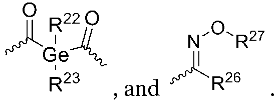

More preferably, at least one of R¹⁰ and R¹² to R¹³ is a substituent selected from the group consisting of chlorine; bromine; iodine; formyl; carbonate; carbonate ester; ester; amide; CF₃; substituted or unsubstituted C₂-C₄₉-alkyl acyl; substituted or unsubstituted C₂-C₄₉-aryl acyl; ketone; acyl; oxime; aldehyde, NO₂; CN; (meth)acrylate; sulfones; alkylsulfones; sulfonamides; SO₂Me; SO₂NH₂; methoxy; and tosyl; or one of the following formulae: 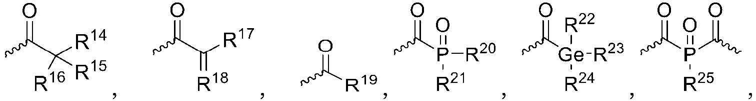 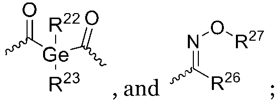 wherein R¹⁴ to R²⁷ are defined above.

More preferably, at least one of R² to R⁵ and additionally at least one of R¹⁰ to R¹³, like preferably R¹⁰ and R¹² to R¹³, in formula (1) are substituents selected from the group consisting of carbonyl; chlorine; bromine; iodine; formyl; carbonate; carbonate ester; ester; amide; CF₃; substituted or unsubstituted C₂-C₄₉-alkyl acyl; substituted or unsubstituted C₂-C₄₉-aryl acyl; ketone; acyl; oxime; aldehyde, NO₂; CN; (meth)acrylate; sulfones; alkylsulfones; sulfonamides; SO₂Me; SO₂NH₂; methoxy; and tosyl; or one of the following formulae: 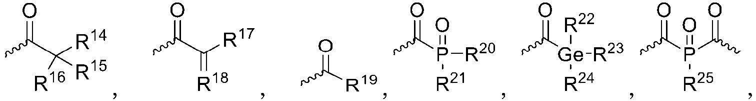 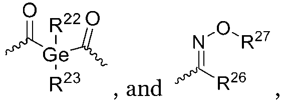 so that the spiropyran of formula (1) contains at least two of such substituents.

A spiropyran with one of the above substituents may be beneficial as a photoinitiator, preferably dual color photoinitiator. On the other hand, spiropyrans containing one of those substituents, may be obtained by a limited or none manufacturing methods, since organometal reagents, like organolithium reagents, may rather attack the carbonyl of the substituent leading to undesired side products. The process for the manufacture of spiropyrans of formula (1) may avoid the use of organometal reagents in the presence sensitive moieties, so that surprisingly the process may provide spiropyrans containing the above substituents, i.e. carbonyl moieties, with higher efficiency.

R¹⁴ to R²⁷ may be independently selected from the group consisting of H; D; halogen; NO₂; CN; OH; SH; substituted or unsubstituted C₁-C₂₀-alkyl; substituted or unsubstituted C₃-C₂₀-cycloalkyl; substituted or unsubstituted C₆-C₄₈-aryl; substituted or unsubstituted C₂-C₄₂-heteroaryl; substituted or unsubstituted C₂-C₄₉-alkyl acyl; substituted or unsubstituted C₂-C₄₉-aryl acyl; substituted or unsubstituted C₁-C₂₀-alkoxy; substituted or unsubstituted C₆-C₄₈-aryloxy; NH₂; substituted or unsubstituted C₁-C₂₀-alkyl ester; substituted or unsubstituted C₆-C₄₈-aryl ester; substituted or unsubstituted C₁-C₂₀ alkyl amide; substituted or unsubstituted C₆-C₄₈-aryl amide; NR'₂; SiR'₃; -O-SiR'₃, wherein R' is independently selected from the group consisting of substituted or unsubstituted C₁-C₂₀-alkyl and substituted or unsubstituted C₆-C₄₈-aryl, two R' may form a ring structure; substituted or unsubstituted carboxylic acids and salts thereof; substituted or unsubstituted sulfonic acids and salts thereof; substituted or unsubstituted sulfonic esters; substituted or unsubstituted sulfonic amides; formyl; ether; thioether; carbonate; carbonate ester; sulfates; boronic acids; boronic esters; phosphonic acids; phosphonic esters; phosphines; phosphates; peroxycarbonic acids; thiocarbonic acids; sulfinic acids; sulfinic esters; sulfonates; thiolesters, sulfoxides; sulfones; alkylsulfones; hydrazides; thioaldehydes; ketones; thioketones; oximes; hydrazines; nitroso; azo; diazo; diazonium; isocyanides; cyanate; isocyanate; thiocyanate; isothiocyanate; hydroperoxide; peroxide; acetals; ketal; orthoester; orthocarbonate esters; ammonium; imines; imides; azide; nitrate; isonitrile; nitrosoxy; substituted or unsubstituted carbamates; substituted or unsubstituted ethers; substituted or unsubstituted polyether carbamates; substituted or unsubstituted arylazo; substituted or unsubstituted C₂-C₂₀-alkynyl and substituted or unsubstituted C₂-C₂₀-alkenyl; wherein the one or more substituents, if present in one or more of R¹⁴-R²⁷, are independently selected from the group consisting of D; halogen; NO₂; CN; C₂-C₄₉-alkyl acyl; substituted or unsubstituted C₁-C₂₀-alkoxy; substituted or unsubstituted C₆-C₄₈-aryloxy; substituted or unsubstituted C₂-C₄₉-aryl acyl; (meth)acrylate; tosyl; sulfonic acid or salts thereof; carboxylic acid or salts thereof; boronic acid or salts thereof; phosphonic acid or salts thereof; NR'₃⁺, wherein R' is independently selected from the group consisting of H, D, substituted or unsubstituted C₁-C₂₀-alkyl and substituted or unsubstituted C₆-C₄₈-aryl, two R' may form a ring structure; NH₂; and OH; and R¹⁵ and R¹⁶ may be linked to each other to form a unsubstituted or substituted ring structure.

Preferably, at least one of R² to R⁵ and R¹⁰ to R¹³ in formula (1) is a substituent selected from the group consisting of chlorine; bromine; iodine; formyl; carbonate; carbonate ester; ester; amide; CF₃; substituted or unsubstituted C₂-C₄₉-alkyl acyl; substituted or unsubstituted C₂-C₄₉-aryl acyl; ketone; acyl; oxime; aldehyde, NO₂; CN; (meth)acrylate; sulfone; alkylsulfone; sulfonamide; SO₂Me; SO₂NH₂; dicyanovinylene and tosyl. More preferably, at least one of R² to R⁵ and R¹⁰ to R¹³ in formula (1) is a substituent selected from the group consisting of chlorine; bromine; iodine, preferably bromine or iodine.

A halide such as chlorine, bromine or iodine may be transformed to a carbonyl functionality in a subsequent reaction, as outlined in the background of the art. Halides can be provided on every position of a salicylaldehyde of formula (4) and on every position of a 2-methyleneindoline of formula (3) allowing for transformation to a carbonyl group on every position. Thereby, bromides can also be introduced by reaction of N-bromosuccinimide with salicylaldehydes which carry electron withdrawing groups such as trifluoromethyl. Therefore, halogenated spiropyrans may be prepared as precursors for carbonyl substituted spiropyrans. To give an example, one way to introduce the carbonyl is by metal-halide exchange of a bromine on spiropyran using n-butyl lithium and reaction of the formed organo-lithium species with a Weinreb amide. The new spiropyran containing a carbonyl can be subjected to an exchange reaction as disclosed in the invention, allowing substitution patterns which may have not been accessible by conventional methods of the prior art.

More preferably, at least one, preferably at least two, of R² to R⁵ and R¹⁰ to R¹³ in formula (1) is a substituent selected from the group consisting of CN, F, Cl, OCF₃, NO₂, ester groups, ketone, formyl, acyl groups, SO₂R, such as SO₂CF₃, SO₂Me, SO₂Ph, or SO₂NH₂, SF₅, NR₃⁺, pyridinium, halogen, and fluorinated alkyls or aryls, such as CF₃. Even more preferably, a substituent selected from the group consisting of acyl, substituted or unsubstituted benzoyl, CN, F, Cl, OCF₃, NO₂, and CF₃, most preferably acyl, substituted or unsubstituted benzoyl, and CF₃.

In one embodiment R² to R⁵ are independently selected from the group consisting of H and electron withdrawing groups, and one of R¹⁰ to R¹³ is selected from unsubstituted or substituted C₆-C₄₉-aryl acyl; or unsubstituted or substituted C₂-C₄₉-alkyl acyl. Typical electron withdrawing groups may be CN, F, Cl, OCF₃, NO₂, ester groups, formyl, acetyl, benzoyl, substituted benzoyl, acyl groups, SO₂R, such as SO₂CF₃, SO₂Me, SO₂Ph, or SO₂NH₂, SF₅, NR₃⁺, pyridinium, halogen, and fluorinated alkyls or aryls, such as CF₃.

It has been surprisingly found, that in the spiropyran of formula (1), the electron withdrawing groups may be decoupled. Thus, if the spiropyran of formula (1\) is used as a photoinitiator, preferably dual color photoinitiator, UV-light is absorbed, and the spiropyran of formula (1) opens to the corresponding merocyanine. The ring opening of the spiropyran to the corresponding merocyanine may be more efficient, the more electron withdrawing groups are present in R² to R⁵ and R¹⁰ to R¹³ in formula (1) as substituents. Hence, in a preferred embodiment multiple electron withdrawing groups in R² to R⁵ and R¹⁰ to R¹³, for example two or more than two CF₃ or other electron withdrawing groups are substituted at R² to R⁵ and R¹⁰ to R¹³. Without being bound by any theory, in the merocyanine form, the acceptors are in conjugation, decreasing the HOMO and LUMO of the merocyanine. When the electron deficient merocyanine absorbs visible light, the excited state is formed, which is strongly oxidizing and has a long lifetime.

In one embodiment at least one of R^{2'} to R^{5'} of spiropyran of formula (2) is independently selected from the group consisting of electron donating groups. Typical electron donating groups may be SH, SR, OH, OR, NH₂, NHR, and NR₂. Such a substitution pattern may facilitate the opening to the merocyanine and the attack of the 2-methyleneindoline on the merocyanine. Preferably, R⁴ is OR, more preferably methoxy. In a further embodiment it may be provided that R¹ is selected from the group consisting of C₁-C₂₀-alkyl and C₆-C₄₈-aryl, alternatively C₁-C₈-alkyl and C₆-C₁₈-aryl, alternatively C₁-C₄-alkyl and C₆-C₁₂-aryl, preferably methyl, benzyl and phenyl.

In an embodiment, the photoinitiator of formula (1) may be linked to a polymerizable group. The polymerizable group may be selected from the group consisting of (meth)acrylate, acrylamide, vinylether, and vinylester, preferably (meth)acrylate by any of R¹ to R¹³.

In a further embodiment, at least one of R¹ to R¹³ in the photoinitiator of formula (1\) may comprise at least one structural motif selected from the group of thioxanthone, acenaphtylene-1,2-dione, thiochroman-4-on, 9-fluorenone, anthraquinone, benzanthrone, 9,10-phenanthrenequinone, xanthone, 1,3-indanedione, chromone, 1,4-naphthoquinone, coumarin, benzil, benzophenone, and acetophenone.

In a further embodiment, at least one of R¹ to R¹³ in the photoinitiator of formula (1) may be substituted or unsubstituted C₆-C₄₈-aryl or substituted or unsubstituted C₁-C₂₀-alkyl, wherein the substituent comprises at least one structural motif selected from the group consisting of thioxanthone, acenaphtylene-1,2-dione, thiochroman-4-on, 9-fluorenone, anthraquinone, benzanthrone, 9,10-phenanthrenequinone, xanthone, 1,3-indanedione, chromone, 1,4-naphthoquinone, coumarin, benzil, benzophenone, and acetophenone.

In another embodiment, two or more spiropyran of formula (1) may be linked to each other by a linker group. The chemical bond(s) to the linker group may be independently established by any of R¹ to R¹³, preferably by R¹.

Preferably, at least one of R² to R⁵ and R¹⁰ to R¹³ in formula (1) is a substituent selected from the group consisting of sulfonic acid or salts thereof; carboxylic acid or salts thereof; boronic acid or salts thereof; phosphonic acid or salts thereof; NR'₃⁺, wherein R' is independently selected from the group consisting of H, D, substituted or unsubstituted C₁-C₂₀-alkyl and substituted or unsubstituted C₆-C₄₈-aryl, two R' may form a ring structure; substituted or unsubstituted sulfonic esters; substituted or unsubstituted sulfonic amides; phosphonic esters; phosphines; phosphates; sulfinic acids; sulfinic esters; sulfonates; sulfoxides; sulfones; alkylsulfones; oximes; isocyanides; cyanate; isocyanate; thiocyanate; isothiocyanate; ammonium; substituted or unsubstituted carbamates; (meth)acrylate; tosyl; NH₂ and OH.

Preferably, R⁶ and R⁷ are independently substituted or unsubstituted C₁-C₂₀-alkyl. Two adjacent groups of R⁶ and R⁷ may be linked to each other to form a fused cycloalkyl ring structure, preferably, a fused C₄-C₈-cycloalkyl, even more preferably cyclohexyl or cyclopentyl. Preferably, the substituted C¹-C²⁰-alkyl may be substituted with at least one substituent select of the group consisting of terminal sulfonic acids or salts thereof, carboxylic acid or salts thereof, and ammonium salts. or alternatively looped to form C₄-C₈ cycloalkyl ring.

Preferably, R¹ is selected from the group consisting of H, D, substituted or unsubstituted C₁-C₆-alkyl, -CH₂-CH₂-OH, -CH₂-COOH, -CH₂-CH₂-COOH, -CH₂-CH₂-CH₂-NMe₃⁺, -CH₂-CH₂-CH₂-SO₃⁻, phenyl and benzyl. More preferably, R¹ is methyl, -CH₂-CH₂-OH, phenyl or benzyl, -CH₂-COOH, -CH₂-CH₂-COOH, -CH₂-CH₂- CH₂-NMe₃⁺ and -CH₂-CH₂- CH₂-SO₃⁻.

Preferably, R⁸ may be selected from H, D, substituted or unsubstituted C₁-C₆-alkyl, and substituted or unsubstituted phenyl. More preferred, R⁸ is H or methyl.

Preferably, R² to R⁵ may be independently selected from H, D, substituted or unsubstituted C₁-C₆-alkyl, carboxylic acid and salts thereof, sulfonic acid and salts thereof, phosphonic acid and salts thereof, fluorine, bromine, chlorine, iodine, substituted or unsubstituted C₂-C₄₉-alkyl acyl; substituted or unsubstituted C₁-C₂₀-alkoxy; substituted or unsubstituted C₂-C₄₉-aryl acyl, CN, NO₂, aldehyde, ketone, sulfones, sulfonamides, SO₂Me, SO₂Ph, SO₂NH₂, CF₃.

More preferably, at least one of R², R³, R⁵ is substituted or unsubstituted C₂-C₄₉-alkyl acyl; substituted or unsubstituted C₂-C₄₉-aryl acyl, CN, aldehyde, or ketone.

More preferably, at least one of R² to R⁵ is substituted C₂-C₄₉-alkyl acyl; substituted C₂-C₄₉-aryl acyl, where the substituent is chosen from the group of electron withdrawing groups.

Preferably, R¹⁰ to R¹³ may be independently selected from H, D, substituted or unsubstituted C₁-C₆-alkyl, carboxylic acid and salts thereof, sulfonic acid and salts thereof, phosphonic acid and salts thereof, fluorine, bromine, chlorine, iodine, substituted or unsubstituted C₂-C₄₉-alkyl acyl; substituted or unsubstituted C₁-C₂₀-alkoxy; substituted or unsubstituted C₂-C₄₉-aryl acyl, CN, NO₂, aldehyde, ketone, sulfone, sulfonamide, SO₂Me, SO₂Ph, SO₂NH₂, CF₃, OCF₃.

More preferably, at least one of R¹⁰, R¹¹, R¹³ is substituted or unsubstituted C₂-C₄₉-alkyl acyl; substituted or unsubstituted C₂-C₄₉-aryl acyl, CN, aldehyde, or ketone.

In another embodiment, R¹¹ may be selected from the group consisting of CF₃, H, D, substituted or unsubstituted alkoxy, substituted or unsubstituted, more preferably alkyl, methyl, tert-butyl, methoxy.

In one preferred embodiment, R¹¹ may be H, D, substituted or unsubstituted alkoxy, or substituted or unsubstituted alkyl, more preferably methyl, tert-butyl or methoxy. The introduction of R¹¹ being one of the selected substituents has the advantage to provide improved dual color photoinitiators.

More preferably, at least one of R¹⁰ to R¹³ is substituted C₂-C₄₉-alkyl acyl; substituted C₂-C₄₉-aryl acyl, where the substituent is chosen from electron withdrawing groups.

In one embodiment, R¹² and R¹³, or R¹¹ and R¹² may be linked together to form a fused 5- or 6-membered ring. Preferably, the fused 5- or 6-membered ring may contain at least one heteroatom. The heteroatom may be selected from the group consisting of O, S and N. The formed fused 5- or 6-membered ring may be a derivative of flavone, xanthone, thioxanthone, coumarin, or naphthoquinone. Even more preferably, the spiropyran of formula (1) is selected from the group consisting of:

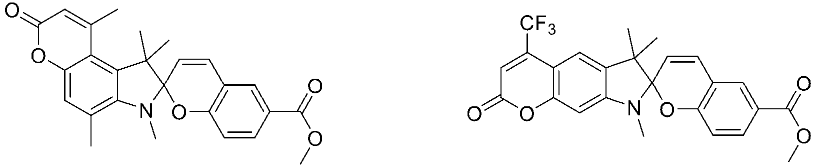

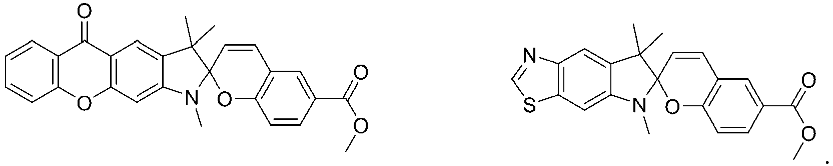

Preferably, R¹⁴ may be selected from H, methyl, halogen, more preferably R¹⁴, R¹⁵, and R¹⁶ may be independently selected from H, methyl, halogen. More preferably R¹⁴, R¹⁵, and R¹⁶ are chlorine.

Preferably, R¹⁴, R¹⁵, and R¹⁶ may be independently selected from H, D, CN, substituted or unsubstituted C₁-C₁₀-alkyl; substituted or unsubstituted C₃-C₁₀-cycloalkyl; substituted or unsubstituted C₆-C₃₂-aryl; substituted or unsubstituted C₂-C₂₈-heteroaryl, more preferably, R¹⁴, R¹⁵, and R¹⁶ may be selected from methyl, phenyl, or substituted phenyl.

In a further preferred embodiment, R¹⁴ may be NR'₂, wherein R' may be independently selected from the group consisting of H, D, substituted or unsubstituted C₁-C₁₀-alkyl and substituted or unsubstituted C₆-C₃₂-aryl, and two R' may form a ring structure; R¹⁵ and R¹⁶ may be independently selected from H, D, CN, substituted or unsubstituted C₁-C₁₀-alkyl; substituted or unsubstituted C₃-C₁₀-cycloalkyl; substituted or unsubstituted C₆-C₃₂-aryl; substituted or unsubstituted C₂-C₂₈-heteroaryl. More preferably, R¹⁴ may be NR'₂, wherein R' may be independently selected from the group consisting of substituted or unsubstituted C₁-C₁₀-alkyl, two R' may form a ring structure; R¹⁵ and R¹⁶ may be independently selected from substituted or unsubstituted C₁-C₁₀-alkyl; substituted or unsubstituted C₃-C₁₀-cycloalkyl; substituted or unsubstituted C₆-C₃₂-aryl. Most preferably, R¹⁴ may be NR'₂, wherein R' may be methyl, ethyl, or two R' completing a morpholine; R¹⁵ and R¹⁶ are independently selected from methyl, ethyl, and benzyl.

In another preferred embodiment, R¹⁴ may be OR', wherein R' is selected from the group consisting of H, D, substituted or unsubstituted C₁-C₁₀-alkyl; substituted or unsubstituted C₃-C₁₀-cycloalkyl; substituted or unsubstituted C₆-C₃₂-aryl; substituted or unsubstituted C₂-C₂₈-heteroaryl, SiR"₃, wherein R" is independently selected from the group consisting of substituted or unsubstituted C₁-C₁₀-alkyl and substituted or unsubstituted C₆-C₃₂-aryl, R¹⁵ and R¹⁶ may be independently selected from H, D, CN, substituted or unsubstituted C₁-C₁₀-alkyl; substituted or unsubstituted C₃-C₁₀-cycloalkyl; substituted or unsubstituted C₆-C₃₂-aryl; substituted or unsubstituted C₂-C₂₈-heteroaryl. More preferably, R¹⁴ may be OR', wherein R' is independently selected from the group consisting of H, D, substituted or unsubstituted C₁-C₁₀-alkyl, substituted or unsubstituted C₃-C₁₀-cycloalkyl, SiR"₃, wherein R" is independently selected from the group consisting of substituted or unsubstituted C₁-C₁₀-alkyl and substituted or unsubstituted C₆-C₃₂-aryl. R¹⁵ and R¹⁶ may be independently selected from substituted or unsubstituted C₁-C₁₀-alkyl; substituted or unsubstituted C₃-C₁₀-cycloalkyl; substituted or unsubstituted C₆-C₃₂-aryl. Most preferably, R¹⁴ is OR', wherein R' is methyl, ethyl, benzyl, or trimethylsilyl; and R¹⁵ and R¹⁶ are independently selected from methyl, ethyl, phenyl, and benzyl.

In a further preferred embodiment, R¹⁴ and R¹⁵ may be OR', wherein R' is independently selected from the group consisting of H, D, substituted or unsubstituted C₁-C₁₀-alkyl; substituted or unsubstituted C₃-C₁₀-cycloalkyl; substituted or unsubstituted C₆-C₃₂-aryl; substituted or unsubstituted C₂-C₂₈-heteroaryl, R¹⁶ may be selected from H, D, CN, substituted or unsubstituted C₁-C₁₀-alkyl; substituted or unsubstituted C₃-C₁₀-cycloalkyl; substituted or unsubstituted C₆-C₃₂-aryl; substituted or unsubstituted C₂-C₂₈-heteroaryl. More preferably, R¹⁴ and R¹⁵ may be OR', wherein R' is independently selected from the group consisting of H, D, substituted or unsubstituted C₁-C₁₀-alkyl, substituted or unsubstituted C₃-C₁₀-cycloalkyl; and R¹⁶ may be selected from substituted or unsubstituted C₁-C₁₀-alkyl; substituted or unsubstituted C₃-C₁₀-cycloalkyl; substituted or unsubstituted C₆-C₃₂-aryl.

Most preferably, R¹⁴ and R¹⁵ may be OR', wherein R' is H, methyl, ethyl, or benzyl; and R¹⁶ may be selected from methyl, ethyl, phenyl, and benzyl.

Preferably, R¹⁷ may be selected from substituted or unsubstituted C₁-C₁₀-alkyl; substituted or unsubstituted C₃-C₁₀-cycloalkyl; substituted or unsubstituted C₆-C₃₂-aryl; substituted or unsubstituted C₂-C₂₈-heteroaryl, substituted or unsubstituted C₂-C₂₀-alkynyl and substituted or unsubstituted C₂-C₂₀-alkenyl, OR', wherein R' is selected from the group consisting of H, D, substituted or unsubstituted C₁-C₁₀-alkyl; substituted or unsubstituted C₃-C₁₀-cycloalkyl; substituted or unsubstituted C₆-C₃₂-aryl; substituted or unsubstituted C₂-C₂₈-heteroaryl, the substituent may contain the atoms necessary to complete a cyclic structure with one of R⁵-R⁸ or R¹⁰-R¹³ forming a phenanthrenequinone. More preferably, R¹⁷ is selected from substituted or unsubstituted C₁-C₁₀-alkyl; substituted or unsubstituted C₆-C₃₂-aryl; OR', wherein R' is selected from the group consisting of H, substituted or unsubstituted C₁-C₁₀-alkyl. Most preferably, R¹⁷ may be methyl, ethyl, phenyl, methoxy, or ethoxy.

Preferably, R¹⁸ may be O or NR' wherein R' is selected from substituted or unsubstituted C₁-C₂₀-alkyl ester; substituted or unsubstituted C₆-C₄₈-aryl ester. More preferably, R¹⁸ may be O or NR' wherein R' is substituted or unsubstituted C₆-C₄₈-aryl ester. Most preferably, R¹⁸ may be O or NR' wherein R' is phenylester or tolylester.

Preferably, the substituent on any of R¹⁴ to R²⁷, preferably R¹⁹, may contain the atoms necessary to complete a cyclic structure with one of R⁵-R⁸ or R¹⁰-R¹³.

Preferably, R¹⁹ may be selected from substituted or unsubstituted C₁-C₁₀-alkyl, preferably methyl or ethyl; substituted or unsubstituted C₃-C₁₀-cycloalkyl; substituted or unsubstituted C₆-C₃₂-aryl; substituted or unsubstituted C₂-C₂₈-heteroaryl, substituted or unsubstituted C₂-C₂₀-alkynyl and substituted or unsubstituted C₂-C₂₀-alkenyl, the substituent may contain the atoms necessary to complete a cyclic structure with one of R⁵-R⁸ or R¹⁰-R¹³, more preferably the substituent may contain the atoms necessary to complete a cyclic structure with one of R⁵-R⁸ or R¹⁰-R¹³ forming an anthracene, thioxanthone, fluorenone, acenaphtylene-1,2-dione, thiochroman-4-on, 9-fluorenone, anthraquinone, benzanthrone, 9,10-phenanthrenequinone, xanthone, 1,3-indanedione, chromone, 1,4-naphthoquinone, coumarin, more preferably R¹⁹ may be selected from substituted or unsubstituted phenyl or naphthyl. More preferably, if the selected substituent of R¹⁹ is additionally substituted, the additional substituent may be selected from the group consisting of H, D, methyl, alkyl, phenyl, CN, Cl, Br, F, methoxy, NMe₂, CF₃, SO₂Me, and SO₂NH₂.

In one preferred embodiment the following formula: 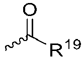 may be α-aminoacyl, α-N,N-dialkylamino-acyl, α-hydroxyacyl or α-alkoxyacyl.

If present, R'¹ or R"¹ may be the same as R¹ defined above, R'² or R"² may be the same as R² defined above, R'³ or R"³ may be the same as R³ defined above, R'⁴ or R"⁴ may be the same as R⁴ defined above, R'⁵ or R"⁵ may be the same as R⁵ defined above, R'⁶ or R"⁶ may be the same as R⁶ defined above, R'⁷ or R"⁷ may be the same as R⁷ defined above, R'⁸ or R"⁸ may be the same as R⁸ defined above, R'⁹ or R"⁹ may be the same as R⁹ defined above, R'¹⁰ or R"¹⁰ may be the same as R¹⁰ defined above, R'¹¹ or R"¹¹ may be the same as R¹¹ defined above, R'¹² or R"¹² may be the same as R¹² defined above, R'¹³ or R"¹³ may be the same as R¹³ defined above.

Here halogen may be fluorine, chlorine, bromine, iodine.

Here alkyl, alkenyl, and alkynyl may be cyclic, linear, or branched.

Here alkyl acyl has the following formula 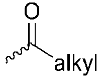 and aryl acyl has the following formula 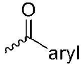 wherein the waved line represents the bond of the acyl group to the structure of formula (1).

**In** the case that one (or more) of the groups R¹-R¹³ is selected as amide, the bond can be made via the N as well as via the CO.

**In** the case that one (or more) of the groups R¹-R¹³ is selected as ester, the bond can be made via the O as well as via the CO.

**In** a further embodiment, it is provided that R³-R⁸ and R¹⁰-R¹³ are independently selected from the group consisting of H and electron withdrawing groups.

A spiropyran of formula (1) containing one or more preferred substituents above may be beneficial for the use as a photoinitiator, preferably dual color photoinitiator, or as an intermediate product to obtain a photoinitiator. On the other hand, spiropyrans containing one of those substituents, may be obtained by limited or none manufacturing methods, since organometallic reagents, such as organolithium reagents, which may be used in a conventional synthetic pathway may rather undergo metal-substituent exchange, like metal-halogen exchange leading to undesired side products. However, also other undesired side reactions are possible with the above moieties by conventional methods of the prior art. The process for the manufacture of spiropyrans of formula (1) of the present invention may avoid the use of organometal reagents and other harsh reagents in the presence of sensitive functional groups so that surprisingly the process may provide spiropyrans containing the above substituents with higher efficiency, or may be accessible for the first time.

Without being bound by any theory, the skilled person may be aware of that while the formylation of 4-hydroxybenzophenone in a Reimer-Tiemann reaction or a Casiraghi reaction may yield the respective 2-hydroxy-5-benzoyl-benzaldehyde, a similar reaction on other hydroxybenzophenone derivatives such as 2-hydroxybenzophenone or 3-hydroxybenzophenone may not be practically accessible. The process for the manufacture of spiropyrans of formula (1) of the present invention may circumvent the formylation of hydroxybenzophenone derivatives so that surprisingly the process may provide spiropyrans containing carbonyl substituents in other position than R¹¹ with higher efficiency, or may be accessible for the first time.

While electron-rich acid anhydrides can be used in Friedl-Crafts acylations to the 5-position of 2-hydroxy-benzaldehydes, this is not possible for other positions or electron deficient acid chlorides or anhydrides. It is further limited in that salicylaldehydes which are functionalized with electron withdrawing groups are not reactive enough for Friedl-Crafts acylations, thus it is very difficult to introduce more than one electron withdrawing group. **In** one aspect of the invention, the process above may be used to access spiropyrans of formula (1) with more than one electron withdrawing group, which additionally may also contain at least one carbonyl.

For that, one approach may be a process in which a halide such as chlorine, bromine or iodine may be transformed to a carbonyl functionality in a subsequent reaction. Halides can be provided on every position of a salicylaldehyde of formula (4) and on every position of a 2-methyleneindoline of formula (3) allowing for transformation to a carbonyl group on every position of a spiropyran. Thereby, bromides can also be introduced by reaction of N-bromosuccinimide with salicylaldehydes which carry electron withdrawing groups such as trifluoromethyl. Therefore, halogenated spiropyrans may be prepared as precursors for carbonyl substituted spiropyrans. The new spiropyran containing a carbonyl can be subjected to an exchange reaction as disclosed in the invention, allowing substitution patterns which may have not been accessible by conventional methods of the prior art. This process may also be applicable for the introduction of electron deficient carbonyl groups or carbonyl groups in positions which are not accessible by direct functionalization of salicylaldehydes or 2-methleneindolines. Thereby, the process may introduce two or more carbonyl functionalities when other electron withdrawing substituents are already present in the precursor of formula (2) or any reactant of formula (3), formula (4) or formula (5). Furthermore, the method of the present invention allows to introduce two identical carbonyl groups simultaneously, when a spiropyran is substituted with a halide each on the indole half and the salicylaldehyde half of the molecule. In this case two equivalents of n-butyl lithium and the respective Weinreb amide may be used.

The advantage of such a method is the simple simultaneous introduction of the necessary functional carbonyl groups leading to dual color photoinitiators with surprisingly improved properties regarding absorption spectra, switching and initiation efficiency. While methods according to the state of the art allow only the simultaneous functionalization in R⁴ and R¹¹ with electron rich carbonyls such as acyl or benzoyl, when no other electron withdrawing groups are present. The newly described method is neither restricted in the choice of positions nor in the electronic properties of the carbonyl substituent or further already present electron withdrawing substituents, particularly when R⁴ or R¹¹ is H. By installing multiple functional groups in one reaction a short synthetic approach to complex and highly functionalized spiropyrans is provided. When further functional groups which are compatible with the reaction conditions to implement the groups, such as CF₃, methyl, methoxy and others, are introduced beforehand, it is possible to introduce three or more functional groups with a surprisingly short synthetic route. These spiropyrans may be better suited as dual color photoinitiators than dual color initiator described in the state of the art.

The present invention may further be advantageous for introducing functional groups on the spiropyran, the 2-methyleneindoline or the salicylaldehyde which interfere with the reaction conditions for introducing a carbonyl functionality. This aspect may be of certain importance for the synthesis of spiropyrans which are functionalized with at least two different carbonyl groups or at least one carbonyl group and at least one interfering substituent. This includes substituents which cause insufficient solubility in solvents, such as tetrahydrofuran, like charged groups, such as sulfonate, sulfonic acid, carboxylate, carboxylic acid, quaternary ammonium salts, phenoxide, hydroxy, phosphonic acid, and phosphonate. This aspect further includes the halides, such as Cl, Br, and I which may undergo a metal-halogen exchange themselves, and functional groups which may react with organo-metallic species such as n-butyl lithium which include but are not limited to ketone, aldehyde, nitrile, ester, carboxamide, carbonate, cyanate, isocyanate, nitro, carbamate, oxime, sulfonyl, methyl sulfone, alkyl sulfones, and phosphine oxide. This aspect further includes aromatic systems which can be deprotonated by n-butyl lithium due to very electron withdrawing substituents such as two trifluoromethyl groups.

The present invention may further be advantageous for introducing different carbonyl groups on either side of the spiropyran motif with flexibility to the position, which leads to improved characteristics regarding the dual color photoinitiation properties.

### 1.1. Precursor, spiropyran of formula (2)

At least one of R'² to R'⁵ and R'¹⁰ to R'¹³, preferably at least one of R'¹⁰ to R'¹³, more preferably at least one of R'¹⁰, R'¹² and R'¹³ of the precursor of formula (2) may be a substituent selected from the group consisting of chlorine; bromine; iodine; formyl; carbonate; carbonate ester; ester; amide; CF₃; substituted or unsubstituted C₂-C₄₉-alkyl acyl; substituted or unsubstituted C₂-C₄₉-aryl acyl; ketone; acyl; oxime; aldehyde, NO₂; CN; (meth)acrylate; sulfones; alkylsulfones; sulfonamides; SO₂Me; SO₂NH₂; methoxy; and tosyl; or one of the following formulae: 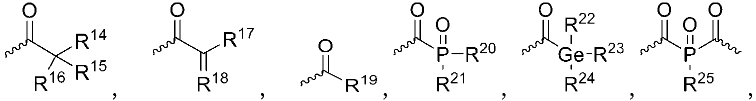 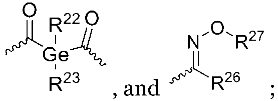 wherein R¹⁴ to R²⁷ are defined as above.

Preferably, if at least one of R'² to R'⁵ is substituted with one of the above substituents, then R'¹⁰ to R'¹³ is also substituted with one of the above substituents, which are preferably electron withdrawing groups, more preferably CF₃.

Preferably, R⁸' is selected from the group consisting of H, D or C₁-C₈-alkyl, preferably methyl.

Hence, a spiropyran of formula (1) containing at least one of the above substituents may be efficiently obtained by a precursor containing one of the substituents above. Surprisingly, such spiropyran of formula (1\) may not have been accessible by a conventional method reported in the prior art.

The precursor being the spiropyran of formula (2) may be provided with 1.0 equivalents in the reaction mixture.

The precursor may be preactivated with a nucleophile as it is disclosed above. Preferably, the nucleophile may be an amine base, preferably amine base, to obtain a pre-activated precursor prior to providing the reaction mixture. In one embodiment, the precursor may be in situ pre-activated in the reaction mixture by adding a catalytic amount of an amine base. The catalytic amount of amine base may be 0.02 to 0.2 equivalents, preferably 0.5 to 0.15 equivalents, most preferably 0.1 equivalents, based on the amount of the precursor. The formation of the pre-activated precursor may improve the formation of the spiropyran of formula (1).

In another embodiment, the precursor may be pre-activated before addition to the reaction mixture by reaction with water, an amine base and/or an acid. The amount of amine base and/or acid may be 0.5-100 equivalents, preferably 0.8 to 10 equivalents, most preferably 1 to 3 equivalents, based on the amount of the precursor. The formation of the pre-activated precursor may improve the formation of the spiropyran of formula (1). The amine base, may be preferably a primary or secondary amine base, more preferably a primary amine base. The primary amine base may be alkylamine, preferably methylamine, ethylamine, propyl amine or a mixture of those. Methylamine may be preferred, since methylamine can be easily removed from the reaction mixture. The acid may be an inorganic acid or an organic acid, preferably a carboxylic acid. More preferably, the carboxylic acid may be formic acid or acetic acid.

Alternatively, in a further embodiment a secondary amine base like piperidine may be preferred. Furthermore, the precursor may be functionalized with a group which can be reduced or oxidized. After the reduction or oxidation, the spiropyran is either subjected to an exchange reaction yielding another spiropyran or treated with a primary amine base to yield the corresponding salicylaldehyde and 2-methyleneindoline. In an embodiment R'² to R'⁵ and R'¹⁰ to R'¹³ may contain a group, which can be reduced or oxidized. Groups which can be reduced or oxidized include but are not limited to formyl, hydroxy, ketone, carboxylic acid, cyano, nitro, and amino.

In a further embodiment, the present invention may be directed to a process for the manufacture of a precursor represented by the following formula (2): 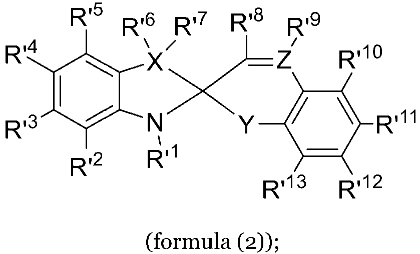
wherein R'¹, R'⁶ to R'⁹ are independently selected from the group consisting of H, D, substituted or unsubstituted C₁-C₁₀-alkyl, preferably methyl; substituted or unsubstituted C₆-C₃₂-aryl, preferably phenyl; substituted or unsubstituted C₂-C₂₀-alkynyl and substituted or unsubstituted C₂-C₂₀-alkenyl and benzyl;
X is C;
Z is C;
Y is O;
R'² to R'⁵ and R'¹⁰-R'¹³ are independently selected from the group consisting of H, D, substituted or unsubstituted C₁-C₁₀-alkyl, substituted or unsubstituted C₆-C₃₂-aryl, substituted or unsubstituted C₁-C₂₀-alkoxy, substituted or unsubstituted C₆-C₄₈-aryloxy, F, Cl, CF₃, CN; wherein two adjacent groups of may be linked to each other to form a fused ring structure, preferably, a fused aromatic C₆-ring; and a substituent of the following formula: 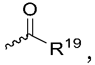
wherein R¹⁹ is defined as it is defined above in the description;
wherein at least one of R² to R⁵ and R¹⁰ to R¹³ is a substituent of the following formula: 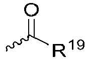 the process comprising the steps of:
   - providing a reactant, wherein the reactant is a spiropyran represented by the following formula (2A): 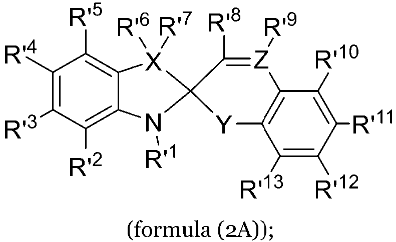
      wherein at least one of R'² to R'⁵ and R'¹⁰ to R'¹³ is a halogen atom selected from the group consisting of Cl, Br and I; and the rest of the substituents in the reactant of formula (2a) are the same as in the precursor of formula (2);
         - reacting the halogen atom of the reactant in a metal-halogen exchange reaction with an organolithium reagent or Grignard reagent, preferably organolithium reagent, to obtain a metal-spiropyran species,
         - subsequently reacting the metal-spiropyran species with a Weinreb-amide of the following formula: 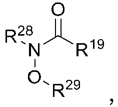 wherein R²⁸ and R²⁹ are selected from the group consisting of substituted or unsubstituted C₁-C₁₀-alkyl; substituted or unsubstituted C₆-C₃₂-aryl; substituted or unsubstituted C₂-C₂₀-alkynyl and substituted or unsubstituted C₂-C₂₀-alkenyl, preferably are R²⁸ and R²⁹ are each methyl,
      to obtain the precursor of formula (2), wherein preferably the precursor of formula (2) is obtained after an acidic aqueous work-up.

The acidic aqueous work-up may be performed after reacting the metal-spiropyran species with a Weinreb-amide to obtain the precursor of formula (2). The acidic aqueous work-up may be necessary to form the desired ketone moiety. The acidic aqueous work-up may contain at least water and an acid, like preferably an inorganic or organic acid. More preferably the acidic aqueous work-up may contain at least water and an acid selected from group consisting of hydrochloric acid, acetic acid or formic acid.

Preferably, at least one of R'² to R'⁵ of the reactant of formula (2a) is a halogen atom selected from the group consisting of Cl, Br and I and at least one of R'¹⁰ to R'¹³ of the reactant of formula (2a) is also a halogen atom selected from the group consisting of Cl, Br and I, and wherein at least two or more halogen atoms react in a metal-halogen exchange reaction and with a Weinreb-amide to obtain a precursor according to formula (2). Hence, the obtained precursor of formula (2) may contain at least two, preferably two, substituents of the following formula:

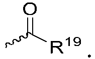

Preferably, the organolithium reagent is an alkyl organolithium reagent or an aryl organolithium reagent, more preferably, the organolithium reagent is an alkyl organolithium reagent. An alkyl organolithium reagent is preferably selected from the group consisting of n-BuLi, sec-BuLi, tert-BuLi, preferably n-BuLi.

Preferably, the halogen atom in the reactant of formula (2) is Br.

In an especially preferred embodiment of the present invention, a precursor of formula (2) may be obtained according to the following reaction scheme: 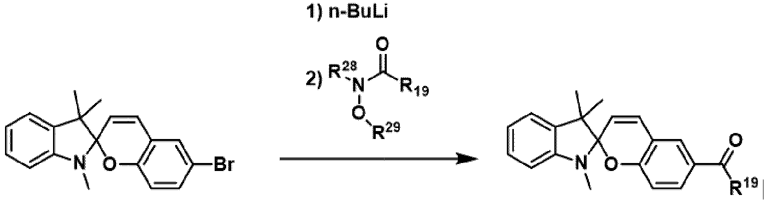 wherein the substitutents are the same as defined above. The skilled person knows that the spriopyran reactant and product in the reaction scheme may contain additional substitutents, which were left out in the reaction scheme for the sake of improved readability. Hence, if necessary, the skilled person also knows that the bromine substituent can be provided on any other position of the aryls as well, leading to the respective isomer, where also the carbonyl substituent is provided on the respective position.

Preferably, the process for the manufacture of the precursor of formula (2) is followed by the process for the manufacture of the spiropyran of formula (1).

For the process to manufacture the precursor of formula (2) the skilled person knows without being bound by any theory, that halogenated spiropyrans may be preferred for reactions with metal-organic reagents, since the spiropyran motif itself may be quite inert. Methods known from the state of the art may utilize either the combination of n-butyl lithium + acid chloride/anhydride or *n*-butyl lithium + nitrile. The combination of n-BuLi with acid chloride/anhydride may have the disadvantage that the carbonyl may be directly formed, so that a second lithiated spiropyran may add to the carbonyl. Furthermore, the reaction mixture may be strongly basic, which may promote aldol condensation of the already formed products. Nitriles with n-BuLi may have a lower reactivity compared to acid chlorides, which may have the disadvantage that they require prolonged reaction times or higher temperatures, than the typically applied -78 °C. This may lead to unspecific side reactions and lower yields.

Hence, the present invention surprisingly solves the problem of the state of the art by providing a method of functionalizing a precursor spiropyran, which may then be used in the exchange reaction. A new method to introduce the carbonyl on the spiropyran is by metal-halide exchange of a halide on spiropyran using an organolithium species, like n-butyl lithium, and reacting the formed organo-lithium species with a Weinreb amide. The reaction works surprisingly well, with high yields, preferably quantitative yields. The only side products of the metal halogen exchange reaction and the subsequent carbonyl introduction may be a few percent, like less than 5 mol%, of de-halogenated spiropyran of the overall yield. The low amount of side products has the surprising effect, that a simplified purification of the desired spiropyran may be accessible so that the manufacture beyond lab scale, like industrial scale is possible. The new method may allow for the first time to introduce more than one carbonyl moiety to the spiropyran by providing a spiropyran precursor with two halogen atoms. The person skilled in the art will notice, that the reaction may be very efficient as the carbonyls cannot be introduced stepwise. The reaction may tolerate several functional groups substituted to spiropyran including alkyl, alkoxy, CF₃, and aryl groups substituted with these substituents, etc. as it is defined above.

### 1.1.A Indolenium salt of formula (3) and spiropyran of formula (2)

**In** a further aspect, the invention may provide a process for the manufacture of a spiropyran represented by the following formula (1): 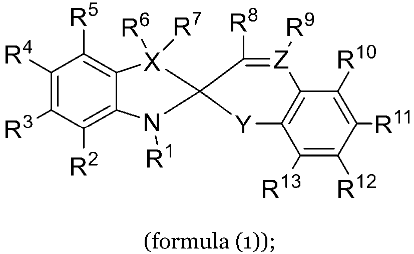
wherein the process comprises the steps of
   - providing a precursor, wherein the precursor is a spiropyran represented by the following formula (2): 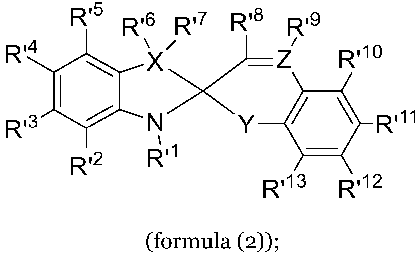
   - providing a reactant, wherein the reactant is
      an indolenium salt represented by the following formula (3): 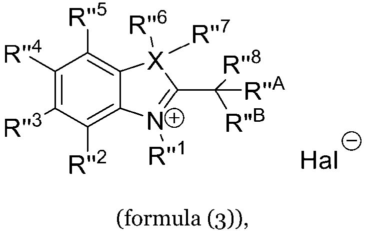
or the corresponding 2-methyleneindoline compound;
   - optionally pre-activating the precursor
   - providing a reaction mixture comprising the precursor or optionally a preactivated precursor, and the reactant to obtain said spiropyran of formula (1);
wherein the spiropyran, the precursor, and the reactant are different from each other;
wherein if the reactant is an indolenium salt of formula (3), the obtained spiropyran of formula (1) is represented by the following formula (1A): 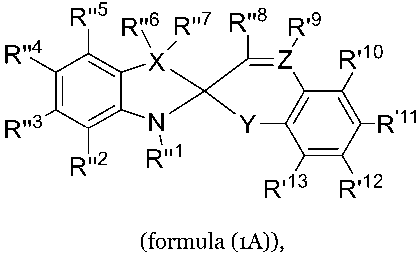
wherein R"¹ to R"⁸ of formula (1A) are independently the same as R"¹ to R"⁸ of formula (3) and R'⁹ to R'¹³ of formula (1A) are independently the same as R'⁹ to R'¹³ of formula (2);

Preferably, the reactant is the indolenium salt of formula (3) wherein X, Hal, R"^{A}, R"^{B} and R"¹ to R"⁸ are substituents as defined above.

Thereby, R'⁹, R'¹⁰, R'¹¹, R'¹² and R'¹³ of the precursor being the spiropyran of formula (2), and the spiropyran of formula (1A) correspond to R⁹, R¹⁰, R¹¹, R¹² and R¹³ of the spiropyran of formula (1). Hence, the precursor being the spiropyran of formula (2), and the spiropyran of formula (1A) may contain the same substituents for R'⁹, R'¹⁰, R'¹¹, R'¹² and R'¹³, as the spiropyran of formula (1) for R⁹, R¹⁰, R¹¹, R¹² and R¹³ above as preferred embodiments.

Thereby, R"¹, R"², R"³, R"⁴, R"⁵, R"⁶, R"⁷ and R"⁸ of the indolenium salt of formula (3), and the spiropyran of formula (1A) correspond to R¹, R², R³, R⁴, R⁵, R⁶, R⁷ and R⁸ of spiropyran of formula (1). Hence, indolenium salt of formula (3), and the spiropyran of formula (1A) may contain the same substituents for R"¹, R"², R"³, R"⁴, R"⁵, R"⁶, R"⁷ and R"⁸, as the spiropyran of formula (1) for R¹, R², R³, R⁴, R⁵, R⁶, R⁷ and R⁸ above as preferred embodiments.

More preferably the amount of electron withdrawing substituents in R"² to R"⁵ of formula (3) is higher than the amount of electron withdrawing substituents in R'² to R'⁵ of formula (2).

Even more preferably, the substituent in R"4 of formula (3) is stronger electron withdrawing than the substituent in R'4 of formula (2), and/or the substituent in R"4 of formula (3) is chosen from the group consisting of carbonyl; chlorine; bromine; iodine; formyl; carbonate; carbonate ester; ester; amide; CF₃; substituted or unsubstituted C₂-C₄₉-alkyl acyl; substituted or unsubstituted C₂-C₄₉-aryl acyl; ketone; acyl; acetyl; benzoyl; oxime; aldehyde, NO₂; CN; (meth)acrylate; sulfones; alkylsulfones; sulfonamides; SO₂Me; SO₂NH₂.

The amount of electron withdrawing substituents may have an effect on the acidity of the compound. This may have the beneficial effect, that the newly formed 2-methyleneindoline is formed as a side product, which may be directly protonated to the corresponding indolenium salt. As a consequence, the newly formed 2-methyleneindoline may not be available for a second condensation reaction, so that the equilibrium is shifted to the formation of the desired spiropyran of formula (1) and the back reaction in the equilibrium is hindered.

Furthermore, if substituents in R'² to R'⁵ of formula (2) render the newly formed 2-methyleneindole more basic, the acid-base equilibrium may force the reaction to the formation of the corresponding indolenium salt substituted with R'² to R'⁵ as defined.

It has been surprisingly found that the equilibrium of the reaction may be independent of the nature of the salicylaldehyde, which makes the reaction very versatile and spiropyrans of formula (1) may be accessible by the present invention for the first time. Furthermore, the process in which the indolenium salt of formula (3) is used, has been found to be surprisingly mild, when the salicylaldehyde is substituted with electron withdrawing groups so that the process may be applicable in the presence of most substituents, which may be acid or base sensitive.

Preferably, the indolenium salt of formula (2) is formed from the corresponding 2-methyleneindoline compound and an acid, wherein preferably the acid is an organic or inorganic acid, more preferably the acid is selected from the group consisting of hydrochloric acid, acetic acid or formic acid.

Prior to mixing the precursor with the reactant in a reaction mixture, the reactant may be pre-activated by converting the indolenium salt of formula (3) into a corresponding 2-methyleneindoline compound, which is then added to the reaction mixture. The corresponding 2-metyhleneindoline compound may have the following formula (3A): 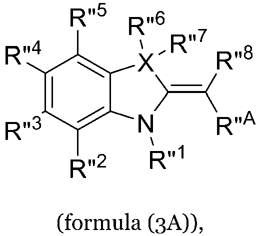 wherein the substituents are defined as in the indolenium salt of formula (3). Hence, instead of adding an indolenium salt of formula (3), the corresponding 2-methyleneindoline compound may be used.

The reactant, i.e. the indolenium salt of formula (3), may be activated with a base, more preferably the reactant is activated with an alkylamine, hydroxide, or carbonate. Hence, the indolenium salt may first be pre-activated by deprotonation of the indolenium salt to form 2-methyleneindoline as pre-activated reactant. The indolenium salt may be deprotonated with a base. The indolenium salt may be deprotonated in situ. Preferably, the indolenium salt is *in situ* deprotonated with a base to form a 2-methyleneindoline compound. The pre-activated reactant of formula (3), i.e. the corresponding 2-methyleneindoline compound, may be provided in the reaction mixture to obtain a spiropyran of formula (1A). Thereby, a different 2-methyleneindoline compound will be newly formed as a side product.

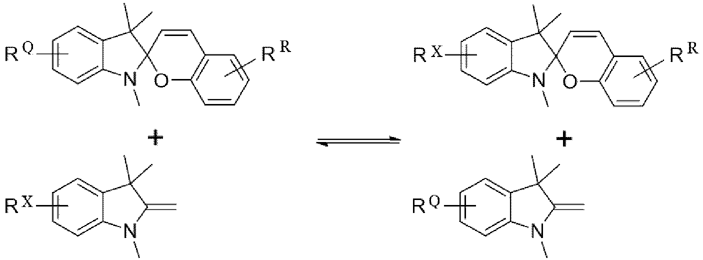

The indolenium salt of formula (3) may be provided to the reaction mixture with an excess of more than 1.1 equivalents, more preferably in range of 1.1 to 1.5 equivalents, more preferably in a range of 1.1 to 1.2 equivalents, most preferably 1.2 equivalents, based on the amount of the precursor of formula (2). If the indolenium salt of formula (3) or the corresponding 2-methyleneindoline is applied in excess, it may add to the spiropyran a second time and undergo another addition or condensation. Hence, the reaction may be carried out with an indolenium salt of formula (3) in slight excess.

Preferably, the concentration of acid or base may be adjusted to provide an overall amount 0.1 equivalents of deprotonated 2-methyleneindoline derivatives in the overall reaction mixture.

The following indolenium salts may be especially preferred:

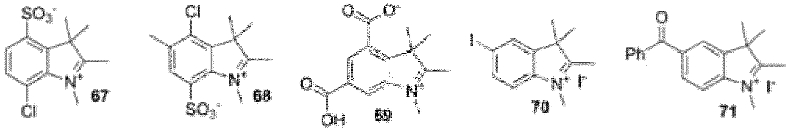

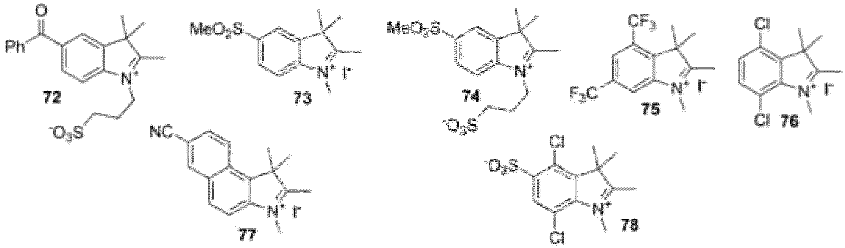

In a further aspect of the invention, the indolenium salt of formula (3) or the corresponding 2-methyleneindoline compound may be provided with an excess of at least 2 equivalents, preferably in the range of 2.5 to 100 equivalents, more preferably in the range of 3 to 10 equivalents, most preferably in the range of 3.5 to 5 equivalents to the reaction mixture containing a spiropyran of formula (2). Where an indolenium salt is applied an amine base may be provided to the reaction mixture in a range of 1 to 3 equivalents. Where a 2-methyleneindoline is applied, acid may be provided in a range of 1 to 2 equivalents. Given these conditions an adduct of the product spiropyran with the following formula 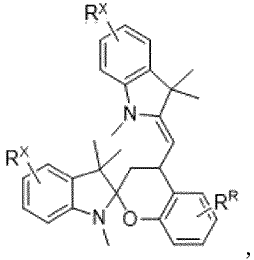 may be obtained from indolenium salt or 2-methyleneindoline compound as reactants. Therein, R^{x} may represent the corresponding substituents R'² to R'⁵ of formula (2) independently from each other, which may be the same or different, as described above. R^{R} may represent substituents R'¹⁰ to R'¹³ of formula (2) independently from each other, which may be the same or different.

This adduct may be easily separated (purified) from the reaction mixture, preferably by precipitation. In a further reaction the adduct may be treated with an acid in a polar solvent to provide the product spiropyran. The acid is applied in the second reaction with 1 to 50%, preferably 5 to 20%, more preferably 10%. The acid may be an inorganic acid or an organic acid, preferably the acid is chosen from hydrochloric acid, sulfuric acid, a carboxylic acid, methane sulfonic acid, toluene sulfonic acid, or trifluoroacetic acid, more preferably the acid is formic acid or acetic acid. The polar solvent may be chosen from THF, nitromethane, MTBE, methanol, ethanol, 2-propanol, 1-butanol, ethylene glycol, preferably the solvent is ethanol or methanol. The temperature of the second reaction may be 0 to 150°C, preferably 15 to 100°C, more preferably 20 to 70°C, most preferably 25°C. Hence, the formation of the adduct may have the advantage that it can be surprisingly easily separated from the reaction mixture and can be further reacted to obtain the desired spiropyran of formula (1).

The reaction mixture may further comprise a catalytic amount of a base, preferably a secondary amine base. The secondary amine base may be preferably piperidine. The catalytic amount of base may preferably be in a range of 0.02 to 0.2 equivalents, more preferably in a range of 0.05 to 0.15 equivalents, most preferably 0.1 equivalents based on the precursor.

In one embodiment, piperidine may be used between and 1 and 1.3 equivalents to pre-activate the indolenium salt prior adding to the reaction mixture, so that the corresponding 2-methyleneindoline is formed, which is then added to the reaction mixture with the precursor of formula (2).

In one embodiment the reaction mixture comprising the indolenium salt of the corresponding 2-methyleneindoline may be free of base.

Preferably, at least one, more preferably each, of R'² to R^{'5} of the precursor of formula (2) may be hydrogen or deuterium, and at least one of R"² to R^{"5} of the indolenium salt of formula (3) and the corresponding 2-methyleneindoline may be an electron withdrawing group, preferably benzoyl or methyl sulfone (SO₂Me). Alternatively, if R"² to R^{"5} of the indolenium salt of formula (3) and the corresponding 2-methyleneindoline may not be an electron withdrawing group, R'² to R^{'5} of the precursor of formula (2) is an electron donating substituent as defined above, preferably methoxy. For the formation of the desired spiropyran of formula (1A) it may be advantageous to accelerate the reaction if at least one of R'¹⁰ to R'¹³ of formula (2) may be independently selected from substituted or unsubstituted C₁-C₆-alkyl, carboxylic acid and salts thereof, sulfonic acid and salts thereof, phosphonic acid and salts thereof, fluorine, bromine, chlorine, iodine, substituted or unsubstituted C₂-C₄₉-alkyl acyl; substituted or unsubstituted C₁-C₂₀-alkoxy; substituted or unsubstituted C₂-C₄₉-aryl acyl, CN, NO₂, aldehyde, ketone, sulfone, alkylsulfone; sulfonamide, SO₂Me, SO₂Ph, SO₂NH₂, CF₃, OCF₃. Preferably, R'¹¹ of formula (2) is CF₃. By having an electron-withdrawing group for R'¹⁰ to R'¹³ of formula (2) it has been surprisingly found, that the reaction with the indolenium salt of formula (3) and the corresponding 2-methyleneindoline can facilitated, so that a higher variety of substituents at the indolenium salt of formula (3) and the corresponding 2-methyleneindoline is tolerated for the formation of spiropyran of formula (1A).

A polar solvent, preferably alcohol, most preferably n-butanol or ethanol, may be added to the reaction mixture. A polar solvent may be THF, water, acetic acid, nitromethane, formic acid, methanol, ethanol, isopropanol. Alcohol may be methanol, ethanol, isopropanol, n-butanol, ethylene glycol. The reaction mixture may be stirred at above 40°C, preferably in a range of 40 °C to 150 °C, more preferably in range of 70 °C to 120°C. The reaction mixture may be stirred for more than 1 hour, preferably in a range of 0.5 hours to 48 hours, more preferably in a range of 1 hour to 12 hours, most preferably in the range of 1 hour to 2 hours. The process of the present invention may yield more than 80% yield, like 80% to 100% yield, preferably 90% to 99% yield of the spiropyran of formula (2), if an indolenium salt of formula (3) is used as a reactant.

In a preferred embodiment, R'² to R^{'5} of the precursor of formula (2) may be hydrogen and at least one of R"² to R^{"5} of the indolenium salt of formula (3) may be substituted or unsubstituted benzoyl, the indolenium salt of formula (3) may be used in 0.9 to 1.2 equivalents, preferably 1.0 to 1.1 equivalents, based on the amount of the precursor, piperidine may be used as a catalyst in 0.1 equivalents and ethanol may be used as a solvent. After stirring the reaction mixture at 70 °C for 1 to 2 hours, the desired product, i.e. spiropyran of formula (1) may be obtained with more than 80% yield next to a newly formed indolenium salt as a further product. If R"² to R^{"5} of the indolenium salt of formula (3) is not substituted by a strong electron withdrawing group, preferably R'² to R^{'5} of the precursor of formula (2) may be selected to be an alkoxy, preferably methoxy, so that a similar high yield of more than 80% may be obtained.

A scavenger, like a template, may be added to the reaction mixture. The scavenger may provide a favorable interaction with the product, i.e. the spiropyran of formula (1), or respective merocyanine or the newly formed 2-Methyleneindoline or the corresponding indolenium salt which may be obtained as a further product. Thereby, the equilibrium of the reaction may be shifted to the product side, so that more of the desired spiropyran of formula (1) may be obtained. A non-limiting example for a scavenger is a proton, which may trap the formed 2-Methyleneindoline as the corresponding indolenium salt. A proton may be obtained if the scavenger is an acid.

### 1.1.B Salicylaldehyde of formula (4) and spiropyran of formula (2)

**In** a preferred aspect, the invention may provide a process for the manufacture of a spiropyran represented by the following formula (1): 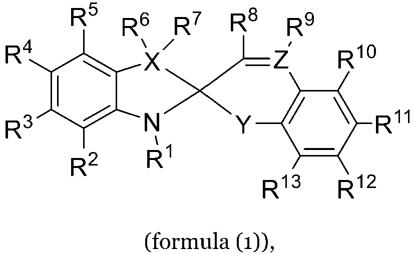
wherein the process comprises the steps of providing a precursor, wherein the precursor is a spiropyran represented by the following formula (2): 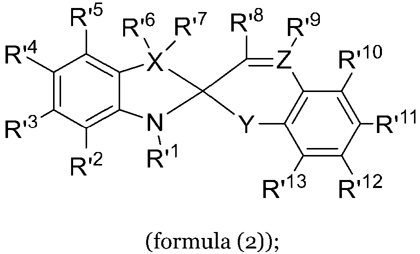
and providing a salicylaldehyde represented by the following formula (4): 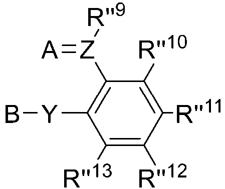 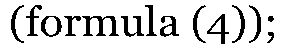
optionally pre-activating the precursor, and then
providing a reaction mixture comprising the precursor or optionally a preactivated precursor, and the reactant to obtain said spiropyran of formula (1);
wherein the spiropyran, the precursor, and the reactant are different from each other;
wherein if the reactant is a salicylaldehyde of formula (4), the obtained spiropyran of formula (1) is represented by the following formula (1B): 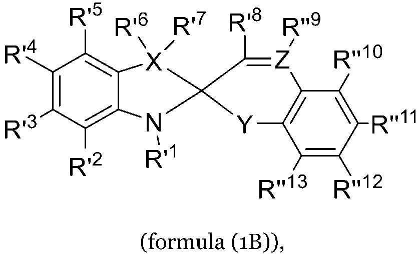
wherein R'¹ to R'⁸ of formula (1B) are independently the same as R'¹ to R'⁸ of formula (2) and R"⁹ to R"¹³ of formula (1B) are independently the same as R"⁹ to R"¹³ of formula (4);

Thereby, R'¹, R'², R'³, R'⁴, R'⁵, R'⁶, R'⁷ and R'⁸ of the precursor being spiropyran of formula (2), and the spiropyran of formula (1B) correspond to R¹, R², R³, R⁴, R⁵, R⁶, R⁷ and R⁸ of spiropyran of formula (1). Hence, the precursor being spiropyran of formula (2), and the spiropyran of formula (1B) may contain the same substituents for R'¹, R'², R'³, R'⁴, R'⁵, R'⁶, R'⁷ and R'⁸, as the spiropyran of formula (1) for R¹, R², R³, R⁴, R⁵, R⁶, R⁷ and R⁸ above as preferred embodiments.

Thereby, additionally, R"⁹, R"¹⁰, R"¹¹, R"¹² and R"¹³ of the salicylaldehyde of formula (4) and the spiropyran of formula (1B) correspond to R⁹, R¹⁰, R¹¹, R¹² and R¹³ of the spiropyran of formula (1). Hence, salicylaldehyde of formula (4) and the spiropyran of formula (1B) may contain the same substituents for R"⁹, R"¹⁰, R"¹¹, R"¹² and R"¹³, as the spiropyran of formula (1) for R⁹, R¹⁰, R¹¹, R¹² and R¹³ above as preferred embodiments.

Preferably, the reactant is a salicylaldehyde of formula (4), wherein A, B, Z, Y, and R"⁹ to R"¹³ are substituents as defined above. More preferably R"⁹ is H or D; and R"¹⁰ to R"¹³ are independently selected from the group consisting of H; D; halogen; tosyl; formyl; carbonate; carbonate ester; ester; amide; CF₃; substituted or unsubstituted C₂-C₄₉-alkyl acyl; substituted or unsubstituted C₂-C₄₉-aryl acyl; ketone; acyl; oxime; aldehyde, NO₂; CN, (meth)acrylate; sulfone; alkylsulfone; sulfonamide; SO₂Me; SO₂NH₂; sulfonic acid or salts thereof; carboxylic acid or salts thereof; boronic acid or salts thereof; phosphonic acid or salts thereof; NR'₃⁺, wherein R' is independently selected from the group consisting of H, D, substituted or unsubstituted C₁-C₂₀-alkyl and substituted or unsubstituted C₆-C₄₈-aryl, two R' may form a ring structure; substituted or unsubstituted sulfonic esters; substituted or unsubstituted sulfonic amides; phosphonic esters; phosphines; phosphates; sulfinic acids; sulfinic esters; sulfonates; sulfoxides; sulfones; alkylsulfones; oximes; isocyanides; cyanate; isocyanate; thiocyanate; isothiocyanate; ammonium; substituted or unsubstituted carbamates; NH₂; OH; substituted or unsubstituted C₁-C₂₀-alkyl; substituted or unsubstituted C₃-C₂₀-cycloalkyl; substituted or unsubstituted C₆-C₄₈-aryl; substituted or unsubstituted C₁-C₂₀-alkoxy; substituted or unsubstituted C₆-C₄₈-aryloxy; substituted or unsubstituted C₁-C₂₀-alkyl ester; substituted or unsubstituted C₆-C₄₈-aryl ester; SiR'₃, -O-SiR'₃ wherein R' is independently selected from the group consisting of substituted or unsubstituted C₁-C₂₀-alkyl and substituted or unsubstituted C₆-C₄₈-aryl, two R' may form a ring structure.

Thereby, it has been surprisingly found that a spiropyran of formula (2) may be treated with a salicylaldehyde of formula (4) of low acidity to exchange and thereby release a new salicylaldehyde from the spiropyran.

The salicylaldehyde of formula (4) may be used in excess, preferably in more than 1.1 equivalents, more preferably in a range of 1.1 to 2.0 equivalents based on the amount of the precursor. As consequence, the formation of a spiropyran of formula (1) which may have a low solubility, may be enhanced by shifting the equilibrium to the product side. Thereby, larger quantities of a new salicylaldehyde may be released as a side product.

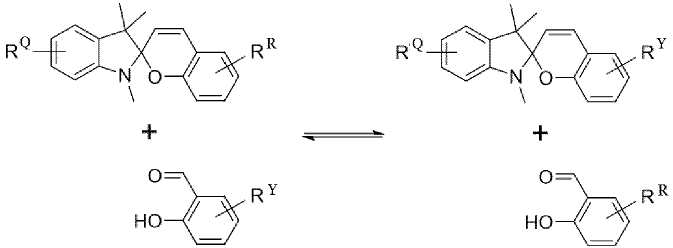

The reaction may be preferably performed in a polar solvent as defined above, more preferably alcohol as defined above, more preferably ethanol. The substituents R^{Y} is preferably selected from non-polar groups. R^{Y}, i.e. R"¹⁰-R"¹³ in salicylaldehyde of formula (4), may be more preferably selected from H, *tert*-butyl, bromine, or chlorine.

Preferably the precursor of formula (2) has been pre-activated with a nucleophile. Preferably, the nucleophile is an amine base, as it is defined above in regard to the precursor.

A scavenger, like a template, may be added to the reaction mixture. The scavenger may provide a favorable interaction with the product, i.e. the spiropyran of formula (1), or respective merocyanine or the newly formed salicylaldehyde as a side product. Thereby the equilibrium of the reaction may be shifted to the product side, so that more of the desired spiropyran of formula (1) containing the substituents R^{Q} and R^{Y} may be obtained.

### 1.1.C Spiropyran of formula (2) and spiropyran of formula (5) in an exchange reaction

In another aspect, the invention may provide a process for the manufacture of a spiropyran represented by the following formula (1): 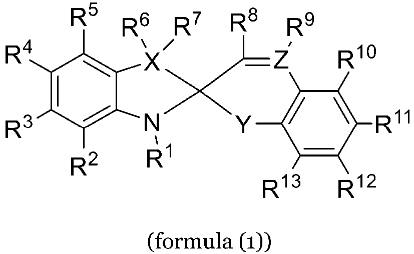
wherein the process comprises the steps of providing a precursor, wherein the precursor is a spiropyran represented by the following formula (2): 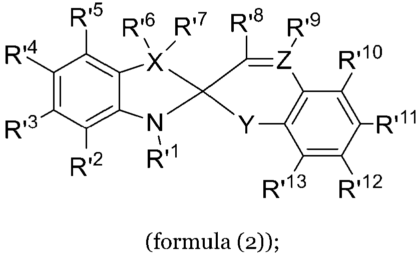
and a spiropyran represented by the following formula (5): 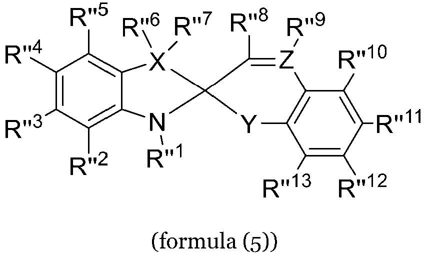
providing a reaction mixture comprising the precursor or optionally a preactivated precursor, and the reactant to obtain said spiropyran of formula (1), wherein the spiropyran, the precursor, and the reactant are different from each other;
wherein if the reactant is a spiropyran of formula (5),
the obtained spiropyran of formula (1) is represented by formula (1A), wherein R"¹ to R"⁸ of formula (1A) are independently the same as R"¹ to R"⁸ of formula (5) and R'⁹ to R'¹³ of formula (1A) are independently the same as R'⁹ to R'¹³ of formula (2), or
the obtained spiropyran of formula (1) is represented by formula (1B), wherein R'¹ to R'⁸ of formula (1B) are independently the same as R'¹ to R'⁸ of formula (2) and R"⁹ to R"¹³ of formula (1B) are independently the same as R"⁹ to R"¹³ of formula (5).

The preferred embodiments for the substituents of spiropyran of formula (1) may also apply for the substituents of the spiropyran of formula (1A) and spiropyran of formula (1B) as defined above.

Preferably, the reactant is a spiropyran of formula (5), wherein the reaction mixture may comprise a further catalyst. More preferably, the catalyst may be an amine base. Even more preferably the catalyst may be a primary or secondary amine base. Still more preferably, the catalyst may be a C1-C6 alkylamine, and most preferably, piperidine, ethylamine or methylamine. In an alternative preferred embodiment, the catalyst may be a 2-methyleneindoline. The catalyst may be added in a catalytic amount, like 0.02 to 0.2 equivalents, preferably 0.05 to 0.15, based on the amount of the precursor of formula (2). The catalyst may pre-activate the precursor.

The reaction mixture of the process may comprise a solvent, preferably a polar solvent as defined above, more preferably alcohol as defined above, even more preferably ethanol or n-butanol. The reaction may be carried out at a temperature from 0 to 200 °C, 10 to 180°C, 20 to 170°C, 30 to 160°C, 40 to 140°C or 60 to 100°C. The reaction may be carried out at a pressure from 0.01 to 100 atm, 0.1 to 10 atm or 0.5. to 1.5 atm, such as normal pressure (= 1 atm corresponsding to 101325 Pa). In a further embodiment, the reaction may be carried out in an alcohol at elevated temperature, preferably in a range of 40 °C to 140°C, more preferably in a range of 60° to 100 °C. In a further preferred embodiment, the molar ratio of the precursor (that is, a precursor of formula (2)) to the reactant (that is, a reactant of formula (3) or of formula (4) or of formula (5)) may be from 1:100 to 100:1, from 1:50 to 50:1, from 1:20 to 20:1 or from1:10 to 10:1. In a further preferred embodiment, the concentration of the precursor (that is, a precursor of formula (2)) in the reaction mixture may be from 0.00001 M to 100 M, from 0.001 to 1 M, or from 0.001 to 0.1 M. In a further preferred embodiment, the concentration of the reactant (that is, a reactant of formula (3) or of formula (4) or of formula (5)) in the reaction mixture may be from 0.00001 M to 100 M, from 0.001 to 1 M, or from 0.001 to 0.1 M. In an especially preferred embodiment, the solvent is ethanol and the elevated temperature may be 70 °C, or n-butanol and the elevated temperature may be 120 °C. In another preferred embodiment, after providing a reaction mixture comprising the precursor or optionally a preactivated precursor, and the reactant, said spiropyran of formula (1) may be obtained after 24 hours, preferably in a range of 24 hours and 10 days, more preferably in a range of 6 to 8 days, most preferably 7 days. Without being bound by any theory it is believed that the following equilibrium may be obtained following the above conditions. Thereby, it is desired, that the equilibrium is on the product side, i.e. right side of the equation.

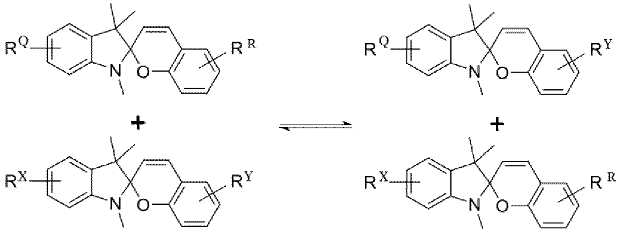

For obtaining the desired spiropyran, the desired spiropyran may have a lower solubility compared to the precursor in said solvent. Hence, the equilibrium may be on the product side. If the reaction mixture may contain a polar solvent, like alcohol, R^{Q} and R^{Y}, i.e. R"²-R"⁵ and R'¹⁰-R'¹³, or R²-R⁵ and R¹⁰-R¹³, respectively, may be one or more non-polar substituent, preferably aromatic systems, like substituted or unsubstituted C₆-C₄₈-aryl, alkyl groups, preferably substituted or unsubstituted C₁-C₂₀-alkyl; substituted or unsubstituted C₃-C₂₀-cycloalkyl; or halides, preferably bromine. Having one or more non-polar substituent in formula (I) in a polar solvent, may have the effect that the spiropyran of formula (I) may precipitate out in the reaction mixture, so that the equilibrium is shifted to the product side, giving control over the reaction. Furthermore, one of the formed spiropyrans may be thermodynamically strongly favored over the corresponding merocyanine. This may mainly lead to the formation of the stable spiropyrans and may also shift the equilibrium to the product side. For example, if the unsubstituted spiropyran contains R^{Q}, R^{Y} =H, the formation of higher substituted spiropyrans with R^{R} and R^{X} being substituents other than H, may be improved. Hence, the equilibrium may also be shifted to the product side.

On the other hand, the reaction mixture may further comprise a scavenger, i.e. template, and said scavenger may be able to bind to the obtained spiropyran, or to bind to an obtained side product in the reaction mixture so that the equilibrium may be shifted to the product side. In other words, this scavenger may be a template being added to the reaction mixture, providing favorable interaction with at least one of the product spiropyrans or respective merocyanines and thereby providing a shift of the equilibrium to the product side.

### 2. Spiropyran of formula (1)

Another aspect of the present invention is a spiropyran represented by the following formula 1: 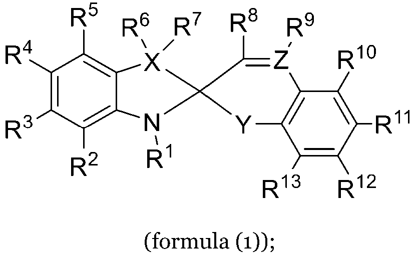
wherein X is selected from S, C, or N, X is selected from S, C, or N; if X is S, then R⁶, R⁷, R'⁶, R'⁷, R"⁶, R"⁷ may not be present accordingly; if X is N, then R⁷, R'⁷, R"⁷ may not be present accordingly,
wherein Y is selected from O, S, or N; where Y is N, the substituent contains the atoms necessary to complete a cyclic structure with R¹³ selected from the group consisting of benzimidazole, indoline, indole, dihydroquinoline, and tetrahydroquinoline, preferably Y is O;
wherein Z is selected from N or C, preferably Z is C;
wherein R¹ to R¹³ are independently selected from the group consisting of H; D; halogen; NO₂; CN; OH; SH; substituted or unsubstituted C₁-C₂₀-alkyl; substituted or unsubstituted C₃-C₂₀-cycloalkyl; substituted or unsubstituted C₆-C₄₈-aryl; substituted or unsubstituted C₂-C₄₂-heteroaryl; substituted or unsubstituted C₂-C₄₉-alkyl acyl; substituted or unsubstituted C₂-C₄₉-aryl acyl; substituted or unsubstituted C₁-C₂₀-alkoxy; substituted or unsubstituted C₆-C₄₈-aryloxy; NH₂; substituted or unsubstituted C₁-C₂₀-alkyl ester; substituted or unsubstituted C₆-C₄₈-aryl ester; substituted or unsubstituted C₁-C₂₀ alkyl amide; substituted or unsubstituted C₆-C₄₈-aryl amide; NR'₂; SiR'₃; -O-SiR'₃, wherein R' is independently selected from the group consisting of substituted or unsubstituted C₁-C₂₀-alkyl and substituted or unsubstituted C₆-C₄₈-aryl, two R' may form a ring structure; substituted or unsubstituted carboxylic acids and salts thereof; substituted or unsubstituted sulfonic acids and salts thereof; substituted or unsubstituted sulfonic esters; substituted or unsubstituted sulfonic amides; formyl; ether; thioether; carbonate; carbonate ester; sulfates; boronic acids; boronic esters; phosphonic acids; phosphonic esters; phosphines; phosphates; peroxycarbonic acids; thiocarbonic acids; sulfinic acids; sulfinic esters; sulfonates; thiolesters, sulfoxides; sulfones; hydrazides; thioaldehydes; ketones; thioketones; oximes; hydrazines; nitroso; azo; diazo; diazonium; isocyanides; cyanate; isocyanate; thiocyanate; isothiocyanate; hydroperoxide; peroxide; acetals; ketal; orthoester; orthocarbonate esters; ammonium; imines; imides; azide; nitrate; isonitrile; nitrosoxy; substituted or unsubstituted carbamates; substituted or unsubstituted ethers; substituted or unsubstituted polyether carbamates; substituted or unsubstituted arylazo; substituted or unsubstituted C₂-C₂₀-alkynyl and substituted or unsubstituted C₂-C₂₀-alkenyl; wherein two adjacent groups of may be linked to each other to form a fused ring structure, preferably, a fused aromatic C₆-ring;
wherein the one or more substituents, if present in one or more of R¹ to R¹³, are independently selected from the group consisting of D; halogen; NO₂; CN, C₂-C₄₉-alkyl acyl; substituted or unsubstituted C₁-C₂₀-alkoxy; substituted or unsubstituted C₆-C₄₈-aryloxy; substituted or unsubstituted C₂-C₄₉-aryl acyl; (meth)acrylate; tosyl; sulfonic acid or salts thereof; carboxylic acid or salts thereof; boronic acid or salts thereof; phosphonic acid or salts thereof; NR'₃⁺, wherein R' is independently selected from the group consisting of H, D, substituted or unsubstituted C₁-C₂₀-alkyl and substituted or unsubstituted C₆-C₄₈-aryl, two R' may form a ring structure; NH₂; and OH; wherein if present two adjacent groups of R¹⁰ to R¹³, and R² to R⁵ may be independently linked to each other to form a fused ring structure;
   and
wherein at least one substituent for R² to R⁵ and R¹⁰ to R¹³ is selected from the group consisting of chlorine; bromine; iodine; formyl; carbonate; carbonate ester; ester; amide; CF₃; substituted or unsubstituted C₂-C₄₉-alkyl acyl; substituted or unsubstituted C₂-C₄₉-aryl acyl; ketone; acyl; oxime; aldehyde, NO₂; CN; (meth)acrylate; sulfones; alkylsulfones; sulfonamides; SO₂Me; SO₂NH₂; methoxy; and tosyl; or one of the following formulae: 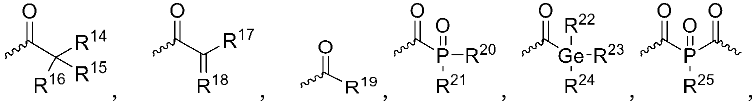 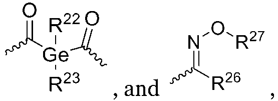
wherein R¹⁴ to R²⁷ are independently selected from the group consisting of H; D; halogen; NO₂; CN; OH; SH; substituted or unsubstituted C₁-C₂₀-alkyl; substituted or unsubstituted C₃-C₂₀-cycloalkyl; substituted or unsubstituted C₆-C₄₈-aryl; substituted or unsubstituted C₂-C₄₂-heteroaryl; substituted or unsubstituted C₂-C₄₉-alkyl acyl; substituted or unsubstituted C₂-C₄₉-aryl acyl; substituted or unsubstituted C₁-C₂₀-alkoxy; substituted or unsubstituted C₆-C₄₈-aryloxy, and NH₂; substituted or unsubstituted C₁-C₂₀-alkyl ester; substituted or unsubstituted C₆-C₄₈-aryl ester; substituted or unsubstituted C₁-C₂₀ alkyl amide; substituted or unsubstituted C₆-C₄₈-aryl amide; NR'₂, SiR'₃, -O-SiR'₃ wherein R' is independently selected from the group consisting of substituted or unsubstituted C₁-C₂₀-alkyl and substituted or unsubstituted C₆-C₄₈-aryl, two R' may form a ring structure; substituted or unsubstituted carboxylic acids and salts thereof; substituted or unsubstituted sulfonic acids and salts thereof; substituted or unsubstituted sulfonic esters; substituted or unsubstituted sulfonic amides; formyl; ether, thioether; carbonate; carbonate ester; sulfates; boronic acids; boronic esters; phosphonic acids; phosphonic esters; phosphines; phosphates; peroxycarbonic acids; thiocarbonic acids; sulfinic acids; sulfinic esters; sulfonates; thiolesters, sulfoxides; sulfones; alkylsulfones; hydrazides; thioaldehydes; ketones; thioketones; oximes; hydrazines; nitroso; azo; diazo; diazonium; isocyanides; cyanate; isocyanate; thiocyanate; isothiocyanate; hydroperoxide; peroxide; acetals; ketal; orthoester; orthocarbonate esters; ammonium; imines; imides; azide; nitrate; isonitrile; nitrosoxy; substituted or unsubstituted carbamates; substituted or unsubstituted ethers; substituted or unsubstituted polyether carbamates; substituted or unsubstituted arylazo; substituted or unsubstituted C₂-C₂₀-alkynyl and substituted or unsubstituted C₂-C₂₀-alkenyl;
wherein the one or more substituents, if present in one or more of R¹⁴-R²⁷, are independently selected from the group consisting of D; halogen; NO₂; CN, C₂-C₄₉-alkyl acyl; substituted or unsubstituted C₁-C₂₀-alkoxy; substituted or unsubstituted C₆-C₄₈-aryloxy; substituted or unsubstituted C₂-C₄₉-aryl acyl; (meth)acrylate; tosyl; sulfonic acid or salts thereof, carboxylic acid or salts thereof, boronic acid or salts thereof, phosphonic acid or salts thereof, NR'₃⁺, wherein R' is independently selected from the group consisting of H, D, substituted or unsubstituted C₁-C₂₀-alkyl and substituted or unsubstituted C₆-C₄₈-aryl, two R' may form a ring structure; NH₂; and OH; and R¹⁵ and R¹⁶ may be linked to each other to form a unsubstituted or substituted ring structure,
   and
wherein at least one other substituent for R² to R⁵ and R¹⁰ to R¹³ is selected from the group consisting of chlorine; bromine; iodine; formyl; carbonate; carbonate ester; ester; amide; CF₃; substituted or unsubstituted C₂-C₄₉-alkyl acyl; substituted or unsubstituted C₂-C₄₉-aryl acyl; ketone; acyl; oxime; aldehyde, NO₂; CN; (meth)acrylate; sulfones; alkylsulfones; sulfonamides; SO₂Me; SO₂NH₂; methoxy; and tosyl;
   and/or
wherein at least one other substituent for R² to R⁵ and R¹⁰ to R¹³ is selected from sulfonic acid or salts thereof; carboxylic acid or salts thereof; boronic acid or salts thereof; phosphonic acid or salts thereof; NR'₃⁺, wherein R' is independently selected from the group consisting of H, D, substituted or unsubstituted C₁-C₂₀-alkyl and substituted or unsubstituted C₆-C₄₈-aryl, two R' may form a ring structure; substituted or unsubstituted sulfonic esters; substituted or unsubstituted sulfonic amides; phosphonic esters; phosphines; phosphates; sulfinic acids; sulfinic esters; sulfonates; sulfoxides; sulfones; alkylsulfones; oximes; isocyanides; cyanate; isocyanate; thiocyanate; isothiocyanate; ammonium; substituted or unsubstituted carbamates; (meth)acrylate; tosyl; NH₂; and OH.

Preferably, at least one of R² to R⁵ is selected from one of the following formulae: 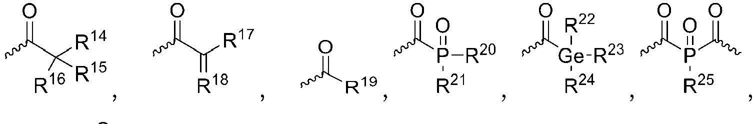 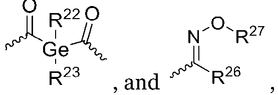 wherein R¹⁴ to R²⁷ are defined above. Additionally, at least one of R¹⁰ to R¹³ may be selected from the group consisting of chlorine; bromine; iodine; formyl; carbonate; carbonate ester; ester; amide; CF₃; substituted or unsubstituted C₂-C₄₉-alkyl acyl; substituted or unsubstituted C₂-C₄₉-aryl acyl; ketone; acyl; oxime; aldehyde; NO₂; CN; (meth)acrylate; sulfones; alkylsulfones; sulfonamides; SO₂Me; SO₂NH₂; methoxy; and tosyl.

On the other hand, at least one other substituent for R¹⁰ to R¹³ may be alternatively or additionally added, selected from sulfonic acid or salts thereof; carboxylic acid or salts thereof; boronic acid or salts thereof; phosphonic acid or salts thereof; NR'₃⁺, wherein R' is independently selected from the group consisting of H, D, substituted or unsubstituted C₁-C₂₀-alkyl and substituted or unsubstituted C₆-C₄₈-aryl, two R' may form a ring structure; substituted or unsubstituted sulfonic esters; substituted or unsubstituted sulfonic amides; phosphonic esters; phosphines; phosphates; sulfinic acids; sulfinic esters; sulfonates; sulfoxides; sulfones; alkylsulfones; oximes; isocyanides; cyanate; isocyanate; thiocyanate; isothiocyanate; ammonium; substituted or unsubstituted carbamates; (meth)acrylate; tosyl; NH₂; and OH.

Furthermore, preferably, at least one of R¹⁰ to R¹³; more preferably at least one of R¹⁰ and R¹² to R¹³, is selected from one of the following formulae: 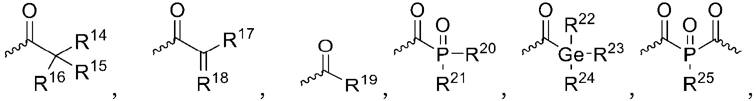 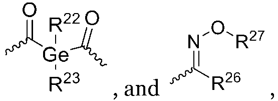 wherein R¹⁴ to R²⁷ are defined above. Additionally, at least one of R² to R⁵ may be selected from chlorine; bromine; iodine; formyl; carbonate; carbonate ester; ester; amide; CF₃; substituted or unsubstituted C₂-C₄₉-alkyl acyl; substituted or unsubstituted C₂-C₄₉-aryl acyl; ketone; acyl; oxime; aldehyde; NO₂; CN; (meth)acrylate; sulfones; alkylsulfones; sulfonamides; SO₂Me; SO₂NH₂; methoxy; and tosyl.

On the other hand, at least one other substituent for R² to R⁵ may be alternatively or additionally added, selected from sulfonic acid or salts thereof; carboxylic acid or salts thereof; boronic acid or salts thereof; phosphonic acid or salts thereof; NR'₃⁺, wherein R' is independently selected from the group consisting of H, D, substituted or unsubstituted C₁-C₂₀-alkyl and substituted or unsubstituted C₆-C₄₈-aryl, two R' may form a ring structure; substituted or unsubstituted sulfonic esters; substituted or unsubstituted sulfonic amides; phosphonic esters; phosphines; phosphates; sulfinic acids; sulfinic esters; sulfonates; sulfoxides; sulfones; alkylsulfones; oximes; isocyanides; cyanate; isocyanate; thiocyanate; isothiocyanate; ammonium; substituted or unsubstituted carbamates; (meth)acrylate; tosyl; NH₂; and OH.

Preferably, at least one of R² to R⁵ and additionally at least one of R¹⁰ to R¹³ are substituents selected from one of the following formulae: 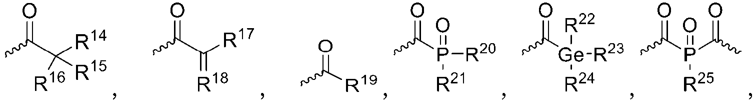 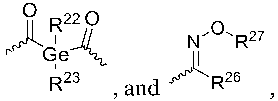 wherein R¹⁴ to R²⁷ are defined above.

Preferably, the selected substituent at one R² to R⁵ is different from the selected substituent at one of R¹⁰ to R¹³.

In one embodiment, at least two substituents of R², R³, R⁵, and R¹⁰-R¹³ are independently selected from the group consisting of 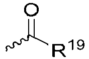 or an electron withdrawing group.

In a further embodiment, at least two substituents of R²-R⁵, R¹⁰, R¹², R¹³ are independently selected from the group consisting of 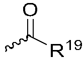 or an electron withdrawing group.

In yet another embodiment, one of R²-R⁵ is 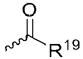 and one of R¹⁰-R¹³ is 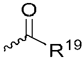 wherein R19 is the same.

In one embodiment, one of R²-R⁵ is 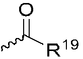 and one of R¹⁰-R¹³ is 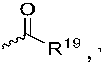 wherein R¹⁹ is different.

In one embodiment, at least one of R²-R⁵ is an electron withdrawing group, and / or at least one of R⁴, R¹⁰, R¹², R¹³ is 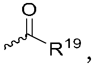 preferably at least one of R¹², R¹³ is 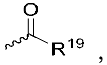 more preferably R¹³ is

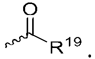

In one embodiment, at least one of R²-R⁵ and/ or R¹⁰-R¹³ is a substituted arylacyl and contains an electron withdrawing group as a substituent, preferably the substituent is selected from the group consisting of CN, CF₃, F, Cl, Br, I, OCF₃, substituted or unsubstituted alkylester, substituted or unsubstituted aryl ester, SO₂Me and SO₂NH₂.

In one embodiment, at least one of R²-R⁵ is a substituted or unsubstituted arylacyl and at least one of R¹⁰-R¹³ is a substituted or unsubstituted alkylacyl.

In one embodiment, R⁴ is an electron withdrawing group and R¹³ is

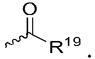

In one embodiment, R¹¹ is H or an electron withdrawing group.

In one embodiment, R¹³ is

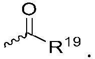

In one embodiment, at least one of R¹⁰-R¹³ is a substituted arylacyl and contains an electron withdrawing group as a substituent, preferably the substituent is selected from the group consisting of CN, CF₃, F, Cl, Br, I, OCF₃, substituted or unsubstituted alkylester, substituted or unsubstituted aryl ester, SO₂Me and SO₂NH₂.

In a preferred embodiment the substituted or unsubstituted aryl acyl as mentioned above is preferably selected from the group consisting of phenyl acyl having the following formula 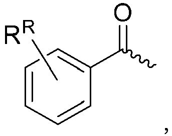 1-naphthyl acyl having the following formula 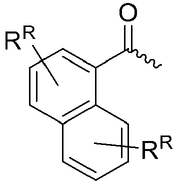 and 2-naphthyl having the following formula 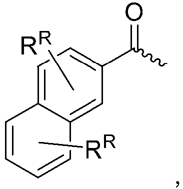 wherein R^{R} are independently selected from the group consisting of hydrogen, deuterium, methyl, tert-butyl, substituted and unsubstituted phenyl, methoxy, CN, CF₃, F, Cl, Br, I, OCF₃, C₁-C₂₀-alkyl ester, C₆-C₄₈-aryl ester, alkyl acyl, aryl acyl, acetyl, benzoyl, NMe₂, SO₂Me, SO2NH₂.

In a preferred embodiment, C₂-C₄₉-alkyl acyl is preferably C₂-C₈-alkyl acyl, more preferably methyl acyl or ethyl acyl.

In a preferred embodiment, the group of electron withdrawing substituents or electron withdrawing groups contains substituents which are more electron withdrawing in reference to hydrogen (H). The skilled person may refer to: "A survey of Hammett substituent constants and resonance and field parameters", Chem. Rev. 1991, 91, 2, 165-195, which lists electron withdrawing groups and electron withdrawing substituents.

Preferably, (substituted or unsubstituted) C₁-C₂₀-alkyl ester is methyl ester or ethyl ester. Preferably, (substituted or unsubstituted) C₆-C₄₈-aryl ester is phenyl ester.

Further preferred embodiments of spiropyran of formula (1) may be stated above in the process for the manufacture of the spiropyran of formula (1).

The following compounds are especially preferred:

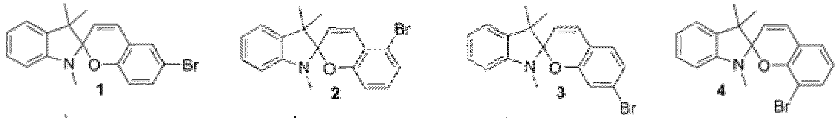

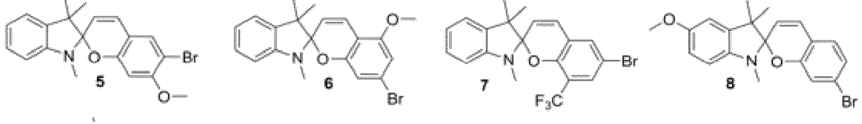

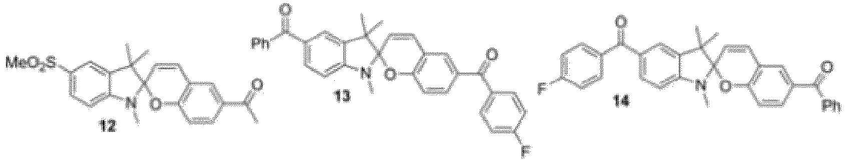

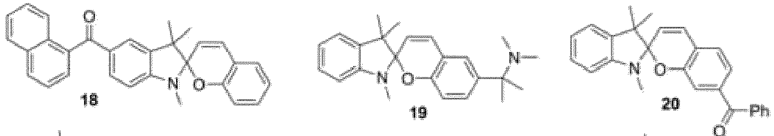

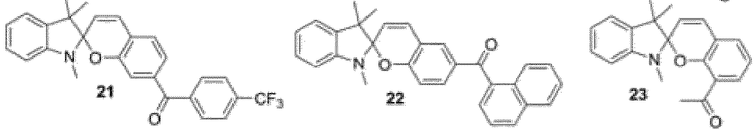

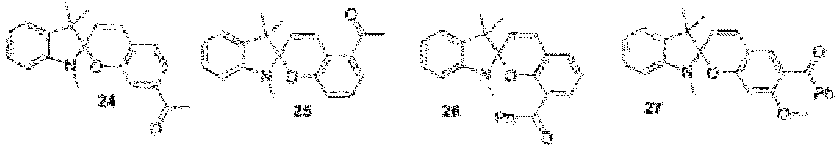

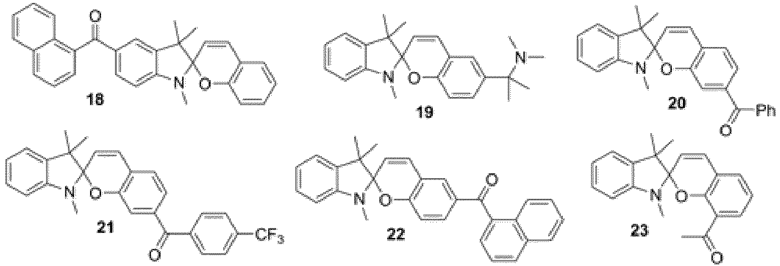

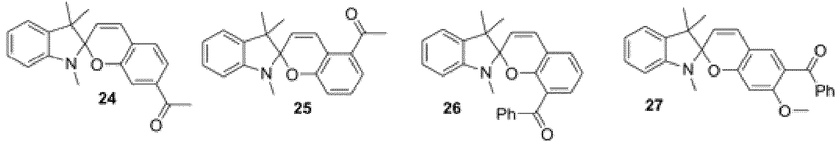

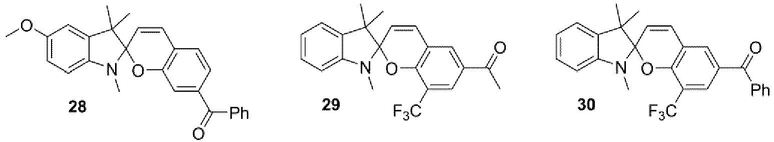

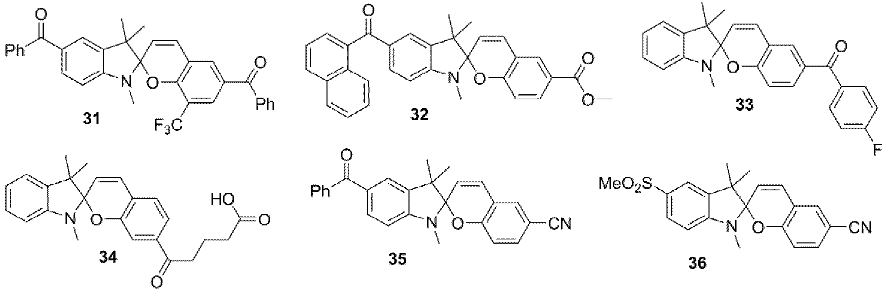

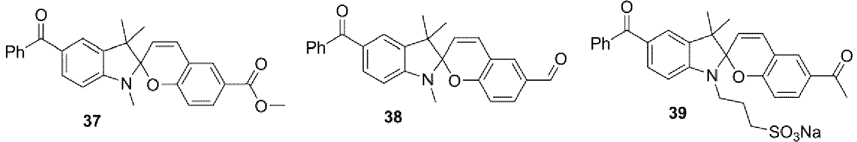

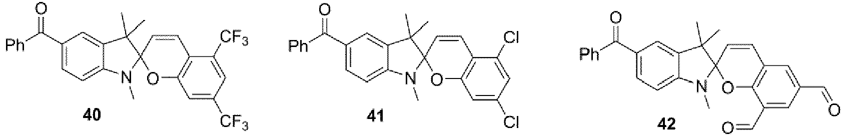

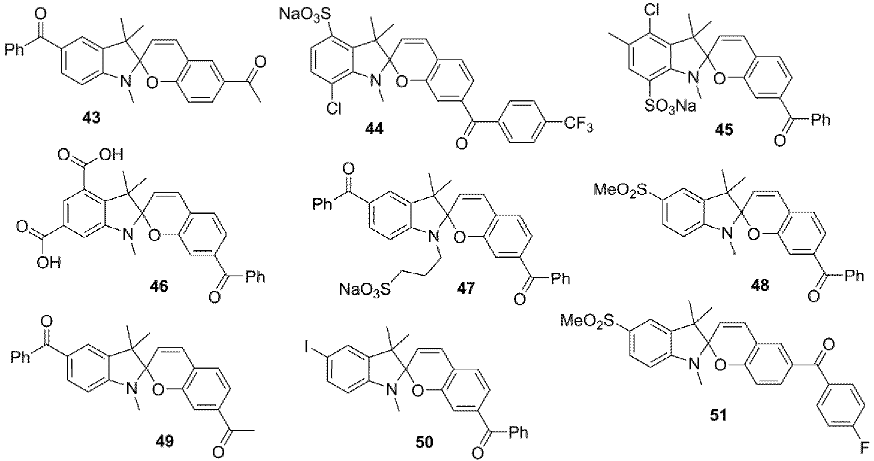

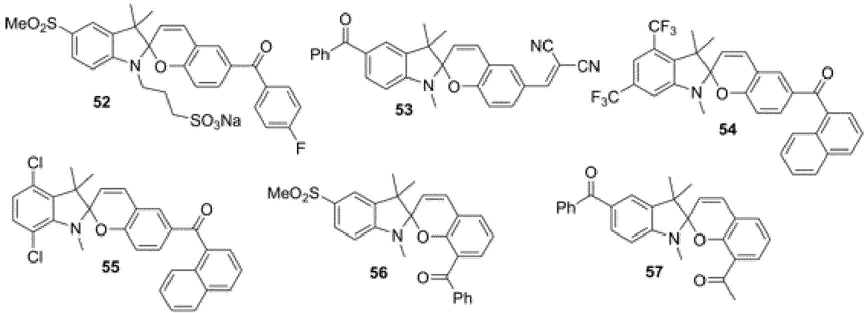

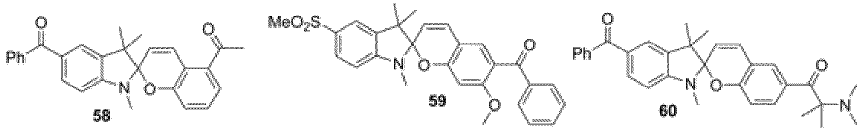

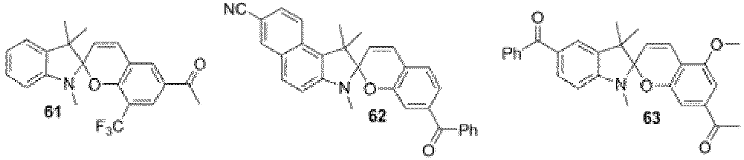

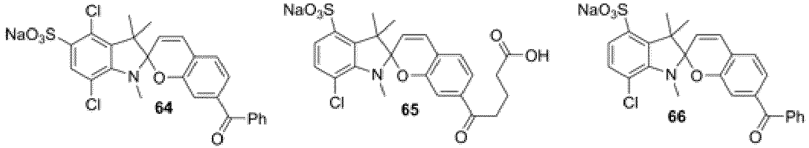

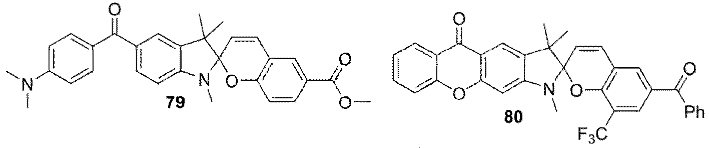

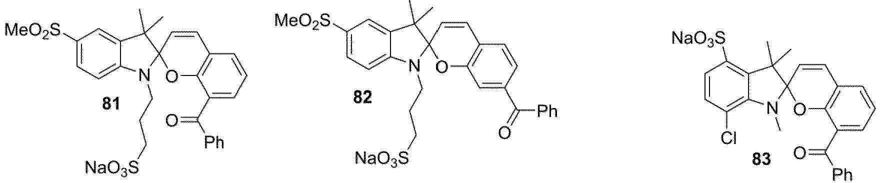

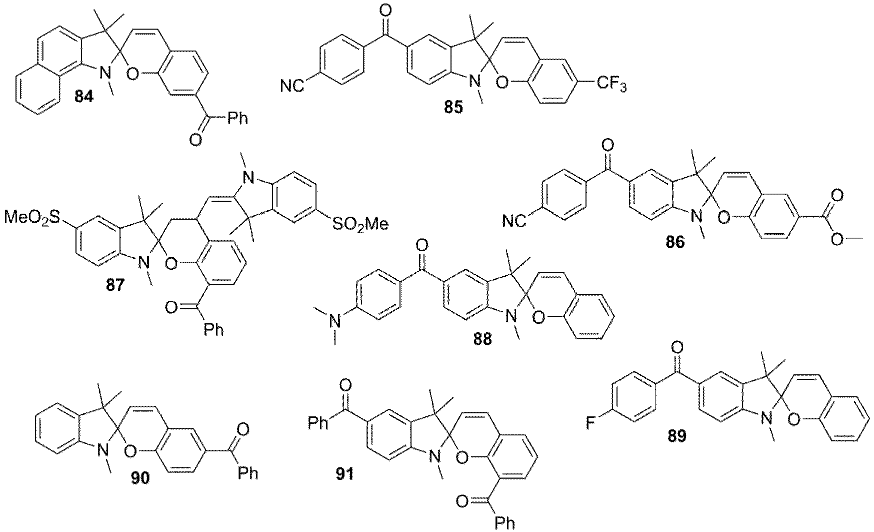

### 3. Dual color photoinitiators

In a further aspect of the present invention, the spiropyran of formula (1) may be a photoinitiator, preferably a dual color photoinitiator.

The photoinitiator molecule, and its necessary function can be produced in different ways. One example provides the following:

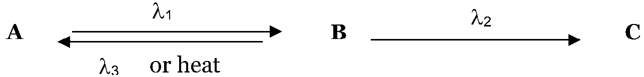

The photoinitiator can exist in three different states, which may be characterized as follows:
Initial state (A):
   - Without light irradiation the photoinitiator molecules are present in this state. The spiropyran of formula (1) may be the initial state (A).
Intermediate state (B):
   - The B state is an electronic ground state. The corresponding merocyanine form of spiropyran of formula (1) may be the intermediate state B.
   - The intermediate state is created from the initial state A by absorption of light of wavelength λ₁.
   - The photoinitiator molecules have a new or more intense absorption band for light of wavelength λ₂.
   - Alternatively, the absorption band for λ₁ disappears. The photoinitiator molecule returns to the initial state A spontaneously in the absence of light or by absorption of light of wavelength λ₃.
Reactive state (C):
   - The reactive state is generated from the intermediate state B by absorption of light of wavelength λ₂.
   - The reactive state initiates a polymerization reaction in the immediate vicinity of the molecule.
   - A back reaction to B is not intended.

In another aspect, the present invention discloses a process for locally polymerizing a starting material by using a spiropyran as a photoinitiator and irradiating the spiropyran with light of at least one wavelength, preferably with light of two different wavelengths.

In a special embodiment, the light of at least one wavelength may have a first wavelength provided by the simultaneous absorption of two photons of a second wavelength.

The light of the second wavelength may be provided by a pulsed laser source, which may be of sufficiently high intensity to induce a two-photon-absorption event of the dual color photoinitiator in the initial state. Without being bound by any theory, the photoinitiator may switch to the intermediate state, where it absorbs at least a single photon of the second wavelength and forms the reactive state. The reactive state may initiate a polymerization reaction, which may be used for the manufacture of a shaped body, especially of a shaped body with high resolution. The second wavelength of the pulsed laser source may be in the range of 500-1000 nm, preferably 600-800 nm. Preferably, the first wavelength and the second wavelength may be the same.

The light of the first wavelength may be absorbed by the photoinitiator resulting in the formation of the intermediate state. The intermediate state may have a higher extinction coefficient at the first wavelength. Due to the formation of the intermediate state the absorbance at the first wavelength may increase, thereby limiting the penetration of the light of the first wavelength into the resin. The intermediate state absorbs the light of the first wavelength and forms the reactive state. The reactive state initiates a polymerization. Such an embodiment may be preferably used for 3D printing with stereolithography, digital light processing or similar techniques. Due to the increasing absorption at the first wavelength, the penetration of the light into the resin may be limited. The limited penetration may be advantageous for a special embodiment to reduce the amount of absorber in the resin or to improve the z-resolution of the printed article.

In a preferred embodiment, the process is for locally polymerizing a starting material by using a spiropyran as a photoinitiator and irradiating the spiropyran with light of two different wavelengths.

In one embodiment, the present invention discloses a process for locally polymerizing a starting material by using a spiropyran as a photoinitiator and irradiating the spiropyran with at least one light source of at least one wavelength, preferably with two light sources of different wavelengths, according to the disclosure above.

In yet another aspect, the present invention discloses a process for locally polymerizing a
starting material by dual color photopolymerization, comprising:
- providing a polymerizable starting material containing photoinitiator molecules wherein the photoinitiator molecule is a spiropyran according to the present invention which can be converted by sequential optical excitation into a reactive state in which the photoinitiator molecules locally trigger polymerization of the starting material; and
   - photopolymerizing the starting material in a local volume by irradiating light of a first wavelength and light of a second wavelength, different from the first wavelength, into the local volume, whereby in the local volume
   - the photoinitiator molecules are converted, due to the absorption of the light of the first wavelength, from an initial state in which the photoinitiator molecules substantially do not absorb the light of the second wavelength, into an intermediate state with changed optical properties compared to the initial state, such that the photoinitiator molecules in the intermediate state absorb the light of the second wavelength; and
- the photoinitiator molecules are transferred from the intermediate state to the reactive state due to the absorption of the light of the second wavelength, which triggers the polymerization locally, and / or
- the photoinitiator molecules may be transferred from the intermediate state to the initial state spontaneously in a thermal reaction.

Dual color photoinitiators, which respond to electromagnetic radiation of a first wavelength by switching from a thermodynamically stable state A to a metastable state B. B is able to absorb electromagnetic radiation of a second wavelength, thereby forming C, that can initiate a polymerization reaction with or without a co-initiator. B can undergo a fast thermal reverse reaction to the form A and may then be deactivated for electromagnetic radiation of the second wavelength. Application of such dual color photoinitiators may allow the curing of a photopolymerizable resin in any volume, where electromagnetic radiation of both wavelengths intersects, for example where an image may be projected on a light sheet.

The disclosed dual color photoinitiators may be characterized in a way that a polymerization may be induced where electromagnetic radiation of two different wavelengths interact with the same volume of polymerizable material in a simultaneous or consecutive fashion. In volumes which interact with electromagnetic irradiation of only one wavelength, no polymerization may be induced.

Due to the absorption of the light of the second wavelength, the photoinitiator molecules can be converted into a reactive state which triggers a radical polymerization in the local volume.

A light beam of light of the first wavelength and a light beam of light of the second wavelength can be irradiated in the local volume at least partially overlapping.

The starting material can be polymerized in several local volumes by means of photo polymerization and thus a three-dimensional shaped body can be produced in the starting material.

Without being bound by any theory, a dual color photoinitiator in the thermodynamically stable form A may absorb a photon from electromagnetic radiation of the first wavelength which induces an isomerization reaction to the metastable form B. The dual color photoinitiator in the metastable form B may absorb a photon from electromagnetic radiation of the second wavelength, which results in an excited state C and may further cause the formation of radicals by hydrogen abstraction from a co-initiator followed by electron transfer or decomposition into radicals, by electron transfer which is followed by hydrogen abstraction or decomposition into radicals, or homolytic bond cleavage which can be preceded or followed by other rearrangement reactions to form radicals. Dual color photoinitiators in the metastable state B which have not absorbed a photon from electromagnetic radiation of the second wavelength, may return spontaneously via a thermal process to the thermodynamically stable state A.

The dual color photoinitiators carry carbonyl functions which are triplet sensitizers for spiropyrans and cause efficient switching to the merocyanine form via the triplet state and therefore may not show curing with UV light alone. Electromagnetic radiation of the first wavelength causes excitation of the initiator in form A and switching to the initiator in form B. The efficient ring opening reaction of the photoswitch motif may prevent the dual color photoinitiators from radical formation by irradiation with the first wavelength alone. The merocyanine type form B can act as an internal triplet sensitizer upon irradiation with the second wavelength, which may cause the carbonyl group to abstract a hydrogen atom from a co-initiator, undergo an electron transfer reaction, or undergo homolytic bond cleavage. Alternatively, the merocyanine type form B can undergo an electron transfer reaction with the co-initiator from the singlet excited state. The substituents may be selected in a way to minimize or extinguish the absorption of the merocyanine type form B at the first wavelength and that form B is thermodynamically destabilized to ensure a fast thermal back reaction from B to A. Substituents are also chosen in a way to adjust the photo redox potential to the respective co-initiator if present. Furthermore, the dual color photoinitiators may benefit from an exceptionally low or neglectable quantum yield for the competing photoreaction from B to A and high extinction coefficients of the B form, where form A does not absorb. The merocyanine form B may typically have a broad absorption in the visible region, which allows for high intensities over a broad range of wavelengths.

Spiropyran of formula (1) that are used as dual color photoinitiators can be functionalized with several strong electron withdrawing groups for R²-R⁵ and R¹⁰-R¹³. In one preferred embodiment, at least one electron withdrawing group is substituted for R¹⁰-R¹³ and at least another electron withdrawing group which may be the same or different may be substituted for R²-R⁵ of spiropyran of formula (1). The electron withdrawing group can be the same or different and include but are not limited to cyano, formyl, keto, nitro, ester, trifluoromethyl, dicyanovinylene, methylsulfone, sulfonamide, fluoro, chloro, bromo, and iodo. In the spiropyran form, the electron withdrawing groups are decoupled. When the spiropyran absorbs UV-light, it opens to the merocyanine, since the ring opening reaction is very efficient. In the merocyanine form, the acceptors are in conjugation, decreasing the HOMO and LUMO of the merocyanine. When the electron deficient merocyanine absorbs visible light, the excited state is formed, which is strongly oxidizing and has a long lifetime. An electron is transferred from a co-initiator to the merocyanine, which is followed by a proton transfer. The hereby formed starting radicals can initiate a polymerization.

In one aspect, the intermediate state may return thermally at the printing temperature in the printing resin to the initial state. Preferably, the intermediate state may return thermally at the printing temperature to the initial state in a mechanism with one or more rate constants with the highest rate constant higher than k=0.01 s⁻¹. Especially preferably, at least one rate constant for the thermal back reaction is higher than 0.02 s⁻¹, more preferably, higher than 0.05 s⁻¹, even more preferably higher than 0.08 s⁻¹, most preferably higher than 0.25 s⁻¹, but optionally not higher than 0.65 s⁻¹. Hence, the rate constant may be in the range of 0.1 s⁻¹ and 0.65 s⁻¹, or any other range which may be formed from the values above.

The spiropyran of formula (1) shown above may be used in connection with the process for locally polymerizing a starting material by dual color photopolymerization. The same applies to the process for 3D-printing of the molded body or the method for volumetric printing of a shaped body. A detailed description in which spiropyran are used in a formulation for dual color photopolymerization in volumetric printing may be found in PCT application [Garmshausen et al., WO2020245456A1], which demonstrates the successful application of spiropyrans as photoinitiators, especially dual color photoinitiators for volumetric printing.

Formulations suitable for volumetric printing may contain the following parts by weight:
- ¹ -99.9999 wt%, preferably 5-99.99 wt%, more preferably 20-99.9 wt% of photopolymerizable compound(s), e.g. monomer(s);
- 0-99wt%, preferably 1-50 wt%, more preferably 3-20 wt% co-initiator, when the co-initiator contains photopolymerizable groups, e.g. acrylates;
- 0-50wt%, preferably 1-40 wt%, more preferably 3-10 wt% of co-initiator, when the co-initiator does not contain photopolymerizable groups;
- 0.0001-20 wt%, preferably 0.001-10 wt%, more preferably 0.01-5 wt%, most preferably 0.1-1 wt% of a spiropyran of formula (1) which may be used a dual color photoinitiator;
- 0-20 wt%, preferably 1-10 wt%, more preferably 3-5 wt% of acids or bases;
- 0-90 wt%, preferably 1-70 wt%, more preferably 5-50 wt%, most preferably 10-30 wt% of other additives, such as organic or inorganic fillers, optical brighteners, inhibitors, chain transfer agents and others;
- 0- 90 wt%, preferably 5-50 wt%, more preferably 10-30 wt% of solvent; and
- 0-99 wt%, preferably 5-95 wt%, more preferably 20-80 wt%, even more preferably 30-70 wt% of water.

All weight ratios are given with respect to the weight of the total formulation.

In a preferred embodiment the dual color photoinitiator may be lyophilized or freeze dried prior to addition to the formulation.

In another preferred embodiment, prior to addition to the formulation, the dual color photoinitiator may be precipitated as a powder by adding a solution of the dual color photoinitiator to a solvent wherein the dual color photoinitiator has a lower solubility.

Typical curing parameters which are suitable for volumetric printing may be:
- any setup which employs light of two different wavelengths;
- a temperature of -20 °C to +100 °C; preferably 0 °C to +60 °C, more preferably +20 °C to + 60 °C
- a first wavelength of: 250 nm to 500 nm; preferably, 300 nm to 450 nm and
- a second wavelength of: 350 nm to 800 nm.

The co-initiator can be of high molecular weight, such as >1000 g/mol, or bound to a polymer to prevent migration in the cured object. The co-initiator can contain polymerizable groups, such as acrylates or methacrylates which are built into the polymer network during curing to prevent later migration of the co-initiator. A typical example is the following:

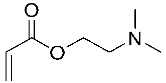

Alternatively, the co-initiator can be a derivative of ethanolamine, preferably a derivative of diethanolamine, more preferably an N-alkyldiethanolamin, alternatively N-methyldiethanolamin, N-phenyldiethanolamin, triethanolamine, 4-(2-hydroxyethyl)morpholine, *N*-(2-hydroxypropyl)morpholine, *N-tert*-butyldiethanolamine, *N*-butyldiethanolamine, *N*-(3-aminopropyl)diethanolamine, *N,N*-di(2-hydroxyethyl)glycine, 1-[bis(2-hydroxyethyl)amino]-2-propanol, *N*-phenyldiethanolamine, *m*-tolyldiethanolamine, *p*-tolyldiethanolamine, *N*-benzyldiethanolamine, bis(2-hydroxyethyl)aminotris(hydroxymethyl)methane, *N,N*-bis(2-hydroxypropyl)aniline, *N,N-*bis(2-hydroxyethyl)-2-aminoethanesulfonic Acid, *N,N*-bis(2-hydroxyethyl)-3-chloroaniline, *N,N,N',N'*-tetrakis(2-hydroxyethyl)ethylenediamine, 3*-*[*N,N*-bis(2-hydroxyethyl)amino]-2-hydroxypropanesulfonic acid, *N*-lauryldiethanolamine, *N,N,N',N'*-tetrakis(2-hydroxypropyl)ethylenediamine, *N,N,N',N",N"*-pentakis(2-hydroxypropyl)diethylenetriamine, ethyl 4-(dimethylamino)benzoate, isoamyl 4-(dimethylamino)benzoate, 2-butoxyethyl 4-(dimethylamino)benzoate, 2-ethylhexyl 4-(dimethylamino)benzoate.

Any combinations of embodiments, preferred ranges and/or moieties, in particular, preferred 76 moieties of the invention are particularly preferred.

In one aspect, the present invention discloses a process for locally polymerizing a starting
material by dual color photopolymerization and forming a shaped body, comprising:
- providing a container which is at least partially filled with a polymerizable starting material containing photoinitiator molecules, which can be converted by sequential optical excitation into a reactive state in which the photoinitiator molecules locally trigger polymerization of the starting material; and
   - photopolymerizing the starting material in a container by irradiating light of a first wavelength and light of a second wavelength, different from the first wavelength, into the container, whereby in the local volume
   - the photoinitiator molecules are converted, due to the absorption of the light of the first wavelength, from an initial state in which the photoinitiator molecules substantially do not absorb the light of the second wavelength, into an intermediate state with changed optical properties compared to the initial state, such that the photoinitiator molecules in the intermediate state absorb the light of the second wavelength; and
- the photoinitiator molecules are transferred from the intermediate state to the
   reactive state due to the absorption of the light of the second wavelength, which triggers the polymerization locally to form the shaped body; wherein
   - the initial state of the photoinitiator in the photopolymerizable material has an extinction coefficient at the first wavelength which is lower than 5000 L mol⁻¹cm⁻¹, preferably lower than 2500 L mol⁻¹cm⁻¹, more preferably lower than 1000 L mol⁻¹cm⁻¹, even more preferably lower than 500 L mol⁻¹cm⁻¹, most preferably lower than 250 L mol⁻¹cm⁻¹; and / or
   - The initial state of the photoinitiator in the photopolymerizable material may have an extinction coefficient at the first wavelength which is higher than 30 L mol⁻¹cm⁻¹, preferably higher than 70 L mol⁻¹cm⁻¹, more preferably higher than 100 L mol⁻¹cm⁻¹, even more preferably higher than 150 L mol⁻¹cm⁻¹, most preferably higher than 200 L mol⁻¹cm⁻¹, like higher than 210 L mol⁻¹cm⁻¹. Hence, the initial state of the photoinitiator in the photopolymerizable material may have an extinction coefficient at the first wavelength which is in the range of 5000 - 0 L mol⁻¹ cm⁻¹ , preferably 2500 - 10 L mol⁻¹ cm⁻¹ , more preferably 1000 - 20 L mol⁻¹ cm⁻¹ , even more preferably 500 - 50 L mol⁻¹ cm⁻¹ , most preferably 300 - 100 L mol⁻¹ cm⁻¹, like 300 - 210 L mol⁻¹ cm⁻¹.

In another aspect, the present invention discloses a process for locally polymerizing a starting
material by dual color photopolymerization and forming a shaped body, comprising:
- providing a container which is at least partially filled with a polymerizable starting material containing photoinitiator molecules, which can be converted by sequential optical excitation into a reactive state in which the photoinitiator molecules locally trigger polymerization of the starting material; and
   - photopolymerizing the starting material in the container by irradiating light of a first wavelength and light of a second wavelength, different from the first wavelength, into the container, whereby in the container
   - the photoinitiator molecules are converted, due to the absorption of the light of the first wavelength, from an initial state in which the photoinitiator molecules substantially do not absorb the light of the second wavelength, into an intermediate state with changed optical properties compared to the initial state, such that the photoinitiator molecules in the intermediate state absorb the light of the second wavelength; and
- the photoinitiator molecules are transferred from the intermediate state to the
   reactive state due to the absorption of the light of the second wavelength, which triggers the polymerization locally to form the shaped body;
wherein the photopolymerizable material has an absorbance at the first wavelength in a range between 1 and 0.05 absorbance units, preferably in a range between 1 and 0.07 absorbance units, more preferably in a range between 0.90 and 0.10 absorbance units, even more preferably in a range between 0.80 and 0.15 absorbance units when measured in a distance of 1 cm. The absorbance of the photopolymerizable material may be measured by UV/-Vis spectrometry using a Cary60 UV-Vis spectrophotometer supplied by Agilent Technologies. Acetonitrile may be used as a reference to determine the absorbance. The first wavelength may be 250 nm to 500 nm; preferably 300 nm to 450 nm.

In an additional aspect, the present invention discloses a process for locally polymerizing a starting material by dual color photopolymerization and forming a shaped body, comprising:
- providing a container which is at least partially filled with a polymerizable starting material containing photoinitiator molecules, which can be converted by sequential optical excitation into a reactive state in which the photoinitiator molecules locally trigger polymerization of the starting material; and
   - photopolymerizing the starting material in a container by irradiating light of a first wavelength and light of a second wavelength, different from the first wavelength, into the container, whereby in the local volume
   - the photoinitiator molecules are converted, due to the absorption of the light of the first wavelength, from an initial state in which the photoinitiator molecules substantially do not absorb the light of the second wavelength, into an intermediate state with changed optical properties compared to the initial state, such that the photoinitiator molecules in the intermediate state absorb the light of the second wavelength; and
- the photoinitiator molecules are transferred from the intermediate state to the
   reactive state due to the absorption of the light of the second wavelength, which triggers the polymerization locally to form the shaped body; wherein
   - the initial state of the photoinitiator in the photopolymerizable material has an extinction coefficient at the first wavelength which is lower than 5000 L mol⁻¹cm⁻¹, preferably lower than 2500 L mol⁻¹cm⁻¹, more preferably lower than 1000 L mol⁻¹cm⁻¹, even more preferably lower than 500 L mol⁻¹cm⁻¹, most preferably lower than 250 L mol⁻¹cm⁻¹; and / or
   - The initial state of the photoinitiator in the photopolymerizable material may have an extinction coefficient at the first wavelength which is higher than 30 L mol⁻¹cm⁻¹, preferably higher than 70 L mol⁻¹cm⁻¹, more preferably higher than 100 L mol⁻¹cm⁻¹, even more preferably higher than 150 L mol⁻¹cm⁻¹, most preferably higher than 200 L mol⁻¹cm⁻¹. Hence, the initial state of the photoinitiator in the photopolymerizable material may have an extinction coefficient at the first wavelength which is in the range of 5000 - 0 L mol⁻¹ cm⁻¹ , preferably 3000 - 10 L mol⁻¹ cm⁻¹ , more preferably 2000 - 20 L mol⁻¹ cm⁻¹, even more preferably 1000 - 50 L mol⁻¹ cm⁻¹ , most preferably 500 - 100 L mol⁻¹ cm⁻¹; and /or
   - wherein the photopolymerizable material has an absorbance at the first wavelength in a range between 1 and 0.05 absorbance units, preferably in a range between 0.90 and 0.10 absorbance units, more preferably in a range between 0.80 and 0.15 absorbance units when measured in a distance of 1 cm. The absorbance of the photopolymerizable material may be measured by UV/-Vis spectrometry using a Cary60 UV-Vis spectrophotometer supplied by Agilent Technologies. Acetonitrile may be used as a reference. An extinction coefficient for a specific wavelength may be calculated according to the Beer-Lambert law from the absorbance of a resin with known concentration of photoinitiator, using the same resin without photoinitiator as a reference. The first wavelength may be 250 nm to 500 nm; preferably 300 nm to 450 nm.

Using the specified extinction coefficients and absorbances units may have the surprising effect of an improved dual color effect, so that hardening at the walls of the local volume, preferably container, is minimized, while a high degree of polymerization at the intersection of the first and second wavelength is assured.

Preferably, the photoinitiator molecule is a spiropyran as disclosed in the present invention. Preferably, a local volume may be container.

Preferably, the container may be at least partially transparent. Hence, the container is configured to at least partially transmit the first wavelength and second wavelength from the source into the container so that a polymerization reaction may be locally triggered. The container may contain a rectangular surface, preferably the container has a cubic shape.

The container may cover a volume of at least 0.5 cm x 0.5 cm x 0.5 cm, preferably at least 1 cm x 1 cm x 1 cm, more preferably at least 1.5 cm x 1.5 cm x 1.5 cm, even more preferably at least 3 cm x 3 cm x 3 cm, most preferably at least 5 cm x 5 cm x 5 cm.

Preferably, the light of the first wavelength and the light of the second wavelength are simultaneously irradiated into the local volume.

In an alternative embodiment, the light of the second wavelength may be irradiated into the local volume after the irradiation of the light of the first wavelength in the local volume has ended, while the light of the second wavelength may be irradiated before the end of a decay time of the intermediate state of the photoinitiator molecules.

Furthermore, preferably, the photoinitiator molecules in the intermediate state substantially do not absorb the light of the first wavelength.

Preferably, the photoinitiator molecules in the initial state have an extinction coefficient at the second wavelength which is lower than 2000 L mol⁻¹ cm⁻¹, more preferably lower than 1000 L mol⁻¹ cm⁻¹, even more preferably lower than 500 L mol⁻¹ cm⁻¹, still more preferably lower than 200 L mol⁻¹ cm⁻¹, most preferably lower than 100 L mol⁻¹ cm⁻¹.

Preferably, the photoinitiator molecules may be converted into a reactive state due to the sequential absorption of light of the first and second wavelength which may trigger a polymerization in the local volume.

Preferably, the photoinitiator molecules are converted into a reactive state due to the absorption of the light of the second wavelength, which may trigger a radical polymerization in the local volume.

Preferably, the light of the first wavelength is irradiated as a light beam and / or the light of the second wavelength is irradiated as a light beam. A light beam may have a diameter of 2 cm or less, preferably 1 cm or less, more preferably 0.5 cm or less, most preferably 1 mm or less. The light beam of the light of the first wavelength and the light beam of the light of the second wavelength may at least partially overlap in the local volume, preferably container, when irradiated.

Preferably, the light of the first wavelength is irradiated as a light sheet and the light of the second wavelength is irradiated as a projection of a 2D-image. The light sheet of the light of the first wavelength and the light projection of the light of the second wavelength may at least partially overlap in the local volume, preferably container, when irradiated.

Preferably, the starting material is polymerized in several local volumes by means of photopolymerization and thus a three-dimensional shaped body is produced in the starting material.

A further aspect of the present invention is a process for 3D-printing a shaped body, wherein the shaped body is produced by means of a process as it is disclosed above.

### 4. Objects with reduced color

It is generally difficult to remove chromophores from the inside of a printed three-dimensional object in volumetric 3d-printing processes. As an example, when a photoinitiator fragment or photoinitiator radical initiates a polymerization or reacts with a radical chain end, the resulting chromophore is bound to the polymer structure of the printed three-dimensional object and cannot be removed e.g. by extraction or washing.

Below illustrated is an example of a way of how a photoinitiator radical, PIH·, and a co-initiator radical, CI·, can be formed from a photoinitiator, PI, and a co-initiator CIH:

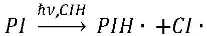

Below illustrated is an example of how a co-initiator fragment, CI, and a photoinitiator, PI, can form part of an exemplary acrylate polymer structure on basis of a radical reaction:

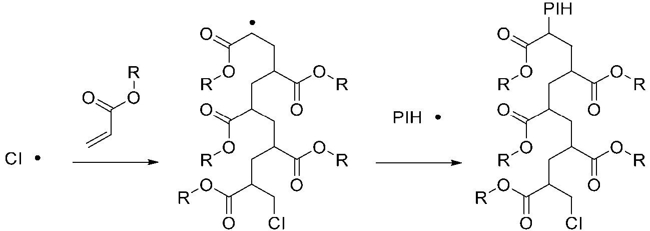

As a consequence, three-dimensional objects manufactured with volumetric 3d-printing processes typically have specific optical properties, namely absorption properties in the visible wavelength range, which result in a color or coloring, respectively. A color or coloring of the three-dimensional objects is not always desired and can indeed even be undesired for certain applications.

As such, there is a need to improve methods for manufacturing three-dimensional objects with respect to the optical properties of the three-dimensional objects which can be manufactured therewith.

According to one aspect of the invention, the process for the formation of a shaped body by dual color photopolymerization comprises further a step of post-processing the shaped body comprising a thermal treatment of the shaped body and/or an optical treatment of the shaped body.

In one embodiment a three-dimensional object may be the shaped body, or a three-dimensional object may be formed from the shaped body. Preferably a three-dimensional object may be formed from a shaped body by removal of the shaped body from the polymerizable starting material.

In one embodiment the post-processing of the three-dimensional object comprises modifying the optical properties of the three-dimensional object resulting in a decrease of the absorption properties of the three-dimensional object for at least one wavelength in a wavelength range between 300 nm and 2000 nm, preferably between 350 nm and 1000 nm, more preferably between 400 nm and 900 nm, most preferably between 450 nm and 800 nm and/or in an increase of the transmissive properties of the three-dimensional object for at least one wavelength in the wavelength range between 300 nm and 2000 nm, preferably between 350 nm and 1000 nm, more preferably between 400 nm and 900 nm, most preferably between 450 nm and 800 nm.

In one embodiment the post-processing of the three-dimensional object comprises modifying the optical properties of the three-dimensional object resulting in that an average transmission or an integral of the transmission between 300 nm and 2000 nm, preferably between 350 nm and 1000 nm, more preferably between 400 nm and 900 nm, most preferably between 450 nm and 800 nm, is increased by at least 1%, preferably at least 2%, more preferably at least 3%, more preferably at least 4%, more preferably at least 5%, more preferably at least 7.5%, more preferably at least 10%, more preferably at least 15%, more preferably at least 20%, more preferably at least 25%, more preferably at least 30%, more preferably at least 35%, more preferably at least 40%, more preferably at least 45%, more preferably at least 50%, preferably relative to a state of the three-dimensional object before it has undergone the at least one method for post-processing of the three-dimensional object; and/or
the post-processing of the three-dimensional object includes modifying the optical properties of the three-dimensional object resulting in that an average absorption or an integral of the absorption between 300 nm and 2000 nm, preferably between 350 nm and 1000 nm, more preferably between 400 nm and 900 nm, most preferably between 450 nm and 800 nm, is decreased by at least 1%, preferably at least 2 %, more preferably at least 3%, more preferably at least 4%, more preferably at least 5%, more preferably at least 7.5%, more preferably at least 10%, more preferably at least 15%, more preferably at least 20%, more preferably at least 25%, more preferably at least 30%, more preferably at least 35%, more preferably at least 40%, more preferably at least 45%, more preferably at least 50%preferably relative to a state of the three-dimensional object before it has undergone the at least one method for post-processing.

In one embodiment the post-processing of the three-dimensional object comprises modifying the optical properties of the three-dimensional object resulting in an average or integrated absorption per mm of thickness of the three-dimensional object of less than 0.5, preferably less than 0.3, more preferably less than 0.2, more preferably less than 0.1, in a wavelength range between 300 nm and 2000 nm, preferably between 350 nm and 1000 nm, more preferably between 400 nm and 900 nm, most preferably between 450 nm and 800 nm.

Preferably, the absorption per mm of thickness of the three-dimensional object body or three-dimensional object is less than 0.5, preferably less than 0.3, more preferably less than 0.2, more preferably less than 0.1, for each wavelength in the wavelength range between 300 nm and 2000 nm, preferably between 350 nm and 1000 nm, more preferably between 400 nm and 900 nm, most preferably between 450 nm and 800 nm.

In one embodiment the post-processing comprises a thermal treatment of the three-dimensional object which thermal treatment comprises tempering the three-dimensional object for a specific time at least one specific temperature.

Preferably, the thermal treatment is carried out in specific time ranges between 0.1 min and 24 h, preferably between 0.5 min and 360 min, more preferably between 1 min and 60 min.

Preferably, athe thermal treatment is carried out in specific temperature ranges between 50°C and 150°C, preferably between 75°C and 125°C.

In one embodiment the post-processing comprises an optical treatment of the three-dimensional object which optical treatment comprises irradiating the three-dimensional object with light of at least one specific wavelength for a specific time with a specific light intensity.

Preferably, the optical treatment is carried out in a time ranges between 0.1 min and 24 h, preferably between 1 min and 360 min, more preferably between 5 min and 60 min.

Preferably, an optical treatment of the three-dimensional object is carried out with a specific light intensity, wherein the specific light intensity can range between 0.0001 - 1000 W/cm², preferably between 0.001 - 100 W/cm², more preferably between 0.1 - 30 W/cm², most preferably between 1-10 W/cm2.

Preferably, the optical treatment is carried out, wherein the at least one specific wavelength ranges between 350 nm and 1000 nm, preferably between 400 nm and 800 nm, more preferably between 350 nm and 500 nm or between 420 nm and 800 nm.

In one embodiment, the optical treatment is carried out by irradiating light with an intensity and a wavelength by which the three-dimensional object is not removed from the polymerizable material. Preferably, the optical treatment is carried out, wherein the three-dimensional object is irradiated for the specific time and light intensity with the light of the at least one specific wavelength without removing the three-dimensional object from the surrounding polymerizable material.

Preferably, the optical treatment is carried out, wherein the three-dimensional object is subject to tempering after irradiating the three-dimensional object for the specific time with light of the at least one specific wavelength, wherein the tempering preferably comprises heating the three-dimensional object to a temperature ranging between 50°C and 150°C for a time ranging between 1 min and 360 min, preferably 5 min and 60 min.

### Claim-like Clause

Item (33) A process for locally polymerizing a starting material by dual color photopolymerization and forming a shaped body, comprising the following steps:
   - providing a container which is at least partially filled with a polymerizable starting material containing photoinitiator molecules, which can be converted by sequential optical excitation into a reactive state in which the photoinitiator molecules locally trigger polymerization of the starting material; and
      - photopolymerizing the starting material in the container by irradiating light of a first wavelength and light of a second wavelength, different from the first wavelength, into the container, whereby in the container
      - the photoinitiator molecules are converted, due to the absorption of the light of the first wavelength, from an initial state in which the photoinitiator molecules substantially do not absorb the light of the second wavelength, into an intermediate state with changed optical properties compared to the initial state, such that the photoinitiator molecules in the intermediate state absorb the light of the second wavelength; and
   - the photoinitiator molecules are transferred from the intermediate state to the
      reactive state due to the absorption of the light of the second wavelength, which triggers the polymerization locally to form the shaped body; and
      - wherein the initial state of the photoinitiator molecules in the photopolymerizable material has an extinction coefficient at the first wavelength which is lower than 5000 L mol⁻¹cm⁻¹.
Item (34) A process for locally polymerizing a starting material by dual color
   photopolymerization and forming a shaped body, comprising the following steps:
   - providing a container which is at least partially filled with a polymerizable starting material containing photoinitiator molecules, which can be converted by sequential optical excitation into a reactive state in which the photoinitiator molecules locally trigger polymerization of the starting material; and
      - photopolymerizing the starting material in a container by irradiating light of a first wavelength and light of a second wavelength, different from the first wavelength, into the container, whereby in the container
      - the photoinitiator molecules are converted, due to the absorption of the light of the first wavelength, from an initial state in which the photoinitiator molecules substantially do not absorb the light of the second wavelength, into an intermediate state with changed optical properties compared to the initial state, such that the photoinitiator molecules in the intermediate state absorb the light of the second wavelength; and
   - the photoinitiator molecules are transferred from the intermediate state to the
      reactive state due to the absorption of the light of the second wavelength, which triggers the polymerization locally to form a shaped body;
      - wherein the photopolymerizable material has a absorbance at the first wavelength in a range between 1 and 0.07 absorbance units.
Item (35) A process according to item (33) or item (34), wherein the photoinitiator molecule is the spiropyran as it is defined in the description.

### EXAMPLES

Hereinafter, the action and effect of the invention will be described in detail through specific examples of the invention. However, the examples are provided only to illustrate the present invention, and the scope of the invention is not limited thereto.

### A. Synthesis

### General synthetic methods

### Method A (Reaction of indolenium salt or respective 2-methylenindoline with a salicylaldehyde, examples 1-12, 35-43, 51, 52, 79, 86)

2-Methyleneindoline (1 mmol), the respective salicylaldehyde derivative (1 mmol), and piperidine (0.1 mmol) are dissolved in 10 mL EtOH and heated to 70 °C until no further formation of the spiropyran derivative is observed, typically 1 h to 12 h. The mixture is cooled to room temperature and worked up as described below.

When an indolenium salt derivative is used instead of 2-methyleneindoline, the reaction is carried out with 1.1 mmol piperidine.

When the product precipitates, the mixture is filtered, and the solid residue is recrystallized from ethanol.

Where the spiropyran is not water soluble, water and ethyl acetate are added to the mixture and the organic phase is washed with water. The organic phase is dried over anhydrous MgSO₄ and evaporated under reduced pressure. The residue is either crystallized or purified by silica-gel column chromatography using petroleum ether - acetone mixtures.

Where the spiropyran is water soluble, the reaction mixture is directly subjected to C-18 functionalized silica-gel and purified by MPLC using methanol - water mixtures. After evaporation of the solvent, the product can be dissolved in acetonitrile or methanol and an ion-exchange resin is added. The mixture is placed on a shaker for 1-3h and filtered. The last step is repeated until the exchange is complete. The solvent is removed under reduced pressure to yield the product spiropyran derivative.

### Method B (Modification of halogenated spiropyran, examples 18-31, 33, 34, 61, 84, 85, 88)

The halogenated spiropyran (1.5 mmol) is dissolved in dry tetrahydrofuran (25 mL) under an argon atmosphere and cooled to -78°C. n-Butyl lithium (1.65 mmol, 2.5 M in hexanes) is added dropwise. After stirring for 15 min at -78°C, a Weinreb amide (2 mmol) is added, and the reaction mixture is allowed to warm to room temperature. The reaction is stirred until no further conversion is observed and 1 M aqueous hydrochloric (10 mL) acid is added. The mixture is stirred for 10 min, 1 M aqueous NaOH solution is added (20 mL) and the mixture is extracted with ethyl acetate. The combined organic layers are dried over anhydrous MgSO₄ and evaporated under reduced pressure. The residue is either crystallized or purified by silica-gel column chromatography using petroleum ether - acetone mixtures.

For derivative **34** glutaric anhydride was used instead of a Weinreb amide and the reaction mixture was neutralized before extraction.

For derivative **19** 2-dimethylamino-2-methylpropionitrile was used instead of a Weinreb amide.

For derivative **31** and **91** the reaction was performed with 2.2. eq of n-BuLi and 2.8 eq of Weinrebamide.

### Method C (Reaction of indolenium salt and catalytic amount of base, examples 15-17, 44-50, 54-60, 62-66, 80-83)

An indolenium salt derivative (1.2 mmol), a spiropyran derivative (1 mmol), and piperidine (0.1 mmol) are dissolved in 20 mL ethanol and stirred at 70 °C until no further formation of the product spiropyran derivative is observed, typically 30 min to 12 h. The reaction mixture is cooled to room temperature and worked up as described below.

When the product precipitates, the mixture is filtered, and the solid residue is recrystallized from ethanol.

Where the spiropyran is not water soluble, water and ethyl acetate are added to the mixture and the organic phase is washed with water. The organic phase is dried over anhydrous MgSO₄ and evaporated under reduced pressure. The residue is either crystallized or purified by silica-gel column chromatography using petroleum ether - acetone mixtures.

Where the spiropyran is water soluble, the reaction mixture is directly subjected to C-18 functionalized silica-gel and purified by MPLC using methanol - water mixtures. After evaporation of the solvent, the product can be dissolved in acetonitrile or methanol and an ion-exchange resin is added. The mixture is placed on a shaker for 1-3h and filtered. The last step is repeated until the exchange is complete. The solvent is removed under reduced pressure to yield the product spiropyran derivative.

In all reactions according to method C the respective 2-methyleneindoline is obtained as well. In reactions where the 2-methyleneindoline is the desired product, the reaction may be performed as follows: 1,3,3-Trimethyl-5-nitro-2-methylen-indolin or the respective indolenium salt indolenium salt (1.2 mmol), a spiropyran derivative (1 mmol), piperidine (0.1 mmol), and acetic acid (1 mL) are dissolved in 20 mL ethanol and stirred at 70 °C until no further formation of the product 2-methyleneindoline derivative is observed, typically 30 min to 12 h. The purification is performed as outlined above.

Synthesis of **56: 73** (2.1 mmol), **26** (1 mmol), and piperidine (2 mmol) are dissolved in 20 mL ethanol and stirred at 70 °C for 24 h. The reaction mixture is cooled to room temperature, water and ethyl acetate are added to the mixture and the organic phase is washed with water. The organic phase is dried over anhydrous MgSO₄ and evaporated under reduced pressure. The residue purified by silica-gel column chromatography using petroleum ether - acetone mixtures to yield the intermediate product **87**. The intermediate **87** is dissolved in ethanol (10 mL) and acetic acid (1 mL). The mixture is stirred for 12 h. 1 M aqueous NaOH solution and ethyl acetate are added, and the mixture is washed with 1 M aqueous NaOH solution and water. The combined organic phase is dried over anhydrous MgSO₄ and evaporated under reduced pressure. The residue purified by silica-gel column chromatography using petroleum ether - acetone mixtures to yield product **56.**

### Method D (Synthesis of indolenium salts of the respective indoles, examples 67-78)

The corresponding aniline derivative (50 mmol) is dissolved in a mixture of concentrated aqueous hydrochloric acid (20 mL) and ice water (30 mL). NaNO₂ (100 mmol) in water is added at 0°C. After stirring for 30 min, SnCl₂ (28.4 g) in concentrated aqueous hydrochloric acid (35 mL) is added. The resulting mixture is stirred for 30 min, filtered, and washed with water or 1M aqueous hydrochloric acid to obtain the hydrazine hydrochloride which is used directly in the next step.

The corresponding hydrazine (as hydrochloride salt) (47.5 mmol), 3-methylbutan-2-one and concentrated aqueous sulfuric acid (7 mL) are dissolved in glacial acetic acid (68 mL). The mixture is refluxed until no further formation of the indole is observed. The mixture is cooled to room temperature and worked up as described below.

When the product is not functionalized with a water solubilizing group, the main fraction of the acetic acid is distilled of. The residue is neutralized with saturated aqueous NaHCO₃ solution. The mixture is extracted with ethyl acetate and the combined organic phases are dried over anhydrous MgSO₄, followed by evaporation of the solvent under reduced pressure. Where the indole is not of sufficient purity for the next step, silica gel column chromatography is performed using petroleum ether / acetone mixtures as eluent.

Where the product is functionalized with a water solubilizing group, ethyl acetate (1 L) is added to the mixture. After the precipitation is complete, the mixture is filtered, and the indole is directly used in the next step. Where the indole is not of sufficient purity, it is purified by MPLC using water - methanol solvent mixtures.

The obtained indole derivative (30 mmol) is dissolved in acetonitrile (150 mL) and the corresponding alkyl halide or propane sultone is added (90 mmol). The mixture is refluxed for 24 h. After cooling to room temperature, the product precipitates, is filtered and washed with acetonitrile. Where the product does not precipitate, the solvent is evaporated under reduced pressure. The indole is purified by recrystallization from mixtures of acetonitrile, acetone, and ethyl acetate.

Where the indole is not reactive enough to form the product in the above mentioned procedure, the procedure is modified as follows: The indole derivative (30 mmol) is dissolved in N,N-dimethylformamide (150 mL) and the corresponding alkyl halide or propane sultone is added (90 mmol). The mixture is heated to 120 °C. After no further product formation is observed, the reaction is cooled to room temperature and ethyl acetate (1 L) is added. After the precipitation is complete, the product is filtered and washed with ethyl acetate. The product is recrystallized from acetonitrile - ethyl acetate mixtures.

Where the indole is needed, the respective indolenium salt is dissolved in a mixture of ethyl acetate and 1 M aqueous NaOH solution. The mixture is extracted with ethyl acetate. The combined organic layers are dried over anhydrous MgSO₄ and evaporated under reduced pressure to yield the indole derivative.

### Exchange of two spiropyrans

**43** (1 mmol) and **51** (1 mmol) are dissolved in ethanol (5 mL) with 1% acetic acid and stirred at 70 °C for 7 days. Water and ethyl acetate are added to the mixture and the organic phase is washed with water. The organic phase is dried over anhydrous MgSO₄ and evaporated under reduced pressure. The residue is purified by silica-gel column chromatography using petroleum ether - acetone mixtures to yield **12** and **13.**

**43** (1 mmol), **51** (1 mmol), **71** (0.1 mmol), and piperidine (0.1 mmol) are dissolved in ethanol (5 mL) with and stirred at 70 °C for 24h. Water and ethyl acetate are added to the mixture and the organic phase is washed with water. The organic phase is dried over anhydrous MgSO₄ and evaporated under reduced pressure. The residue is purified by silica-gel column chromatography using petroleum ether - acetone mixtures to yield **12** and **13.**

**89** (1 mmol), **90** (1 mmol), and 1,3,3-Trimethyl-2-methylen-indolin (0.1 mmol) are dissolved in ethanol (10 mL) and stirred at 70 °C for 7 days. Water and ethyl acetate are added to the mixture and the organic phase is washed with water. The organic phase is dried over anhydrous MgSO₄ and evaporated under reduced pressure. The residue is purified by silica-gel column chromatography using petroleum ether - acetone mixtures to yield **14** and unsubstituted spiropyran.

### Pre-activation of spiropyrans with base and reaction with salicylaldehydes

**18** (1 mmol) and Methylamine (0.1 mmol) are dissolved in n-butanol (20 mL) and water (2 mL) and heated to 120°C for 48 h. The mixture is cooled to room temperature and 1M aqueous hydrochloric acid (10 mL) is added. The mixture is stirred for 10 min, 1 M aqueous NaOH solution (20 mL) and ethyl acetate are added. The organic phase is separated and washed once with 1M aqueous NaOH solution and once with water. The organic phase is dried over anhydrous MgSO₄ and evaporated under reduced pressure. The residue can be directly used in the next reaction. For cases, where the separation in the next reaction is difficult, the residue is purified by silica-gel column chromatography using petroleum ether - acetone mixtures. **15** (1 mmol) from the pre-activation is dissolved in ethanol (10 mL) and methyl 3-formyl-4-hydroxybenzoate (1 mmol) and piperidine (0.1 mmol) are added. The reaction is stirred at 70 °C for 24 h. The reaction mixture is cooled to room temperature and water and ethyl acetate are added to the mixture and the organic phase is washed with water. The organic phase is dried over anhydrous MgSO₄ and evaporated under reduced pressure. The residue is purified by silica-gel column chromatography using petroleum ether - acetone mixtures to yield **32.**

**85**(1 mmol) and Methylamine (0.1 mmol) are dissolved in ethanol (20 mL) and heated to 70 °C for 24 h. The mixture is cooled to room temperature and 1M aqueous hydrochloric acid (10 mL) is added. The mixture is stirred for 10 min, 1 M aqueous NaOH solution (20 mL) and ethyl acetate are added. The organic phase is separated and washed once with 1M aqueous NaOH solution and once with water. The organic phase is dried over anhydrous MgSO₄ and evaporated under reduced pressure. The residue is dissolved in ethanol (10 mL) and methyl 3-formyl-4-hydroxybenzoate (1 mmol) and piperidine (0.1 mmol) are added. The reaction is stirred at 70 °C for 24 h. The reaction mixture is cooled to room temperature and water and ethyl acetate are added to the mixture and the organic phase is washed with water. The organic phase is dried over anhydrous MgSO₄ and evaporated under reduced pressure. The residue is purified by silica-gel column chromatography using petroleum ether - acetone mixtures to yield **86.**

### Knoevenagel condensation on Spiropyrans

**38** (1 mmol), malononitrile (1 mmol), and piperidine (0.1 mmol) are dissolved in ethanol (25 ml) and stirred at room temperature for 2h. Water and ethyl acetate are added to the mixture and the organic phase is washed with water. The organic phase is dried over anhydrous MgSO₄ and evaporated under reduced pressure. The residue is purified by silica-gel column chromatography using petroleum ether - acetone mixtures to yield **53**.

### Numbering scheme for spiropyrans and indolenium salts

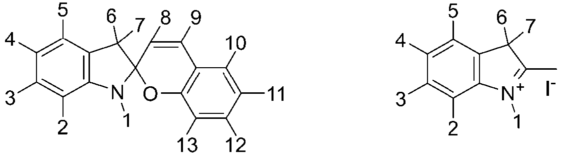

**As non-limiting examples, the following spiropyrans and indolenium salts, 2-methylenindoline, have been prepared by following the specified procedures:**

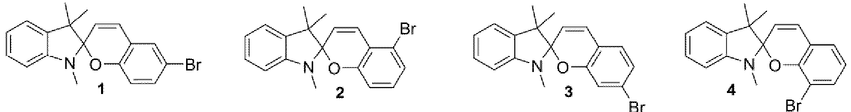

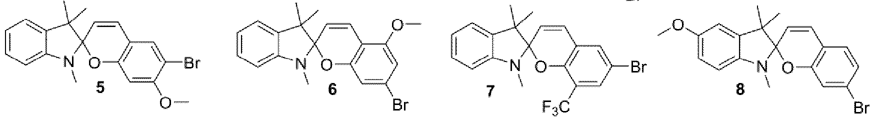

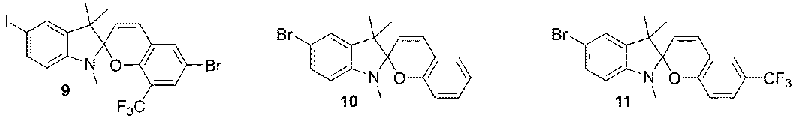

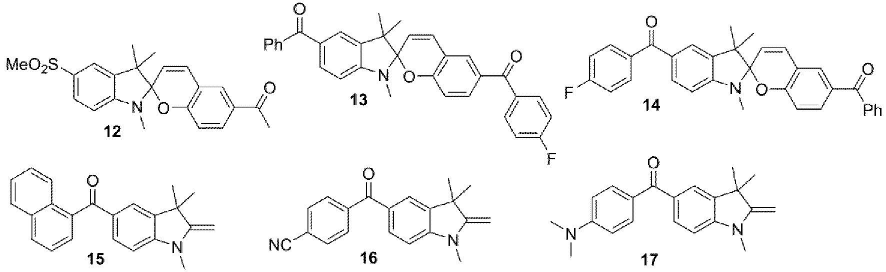

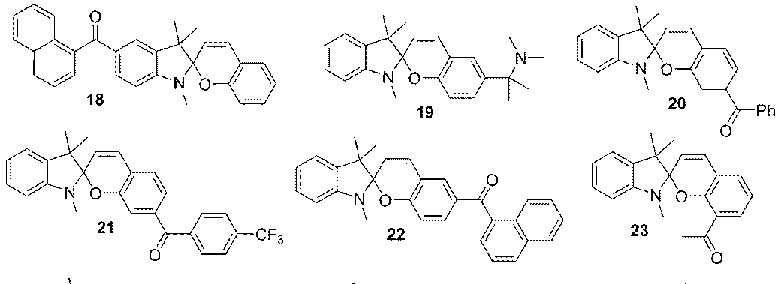

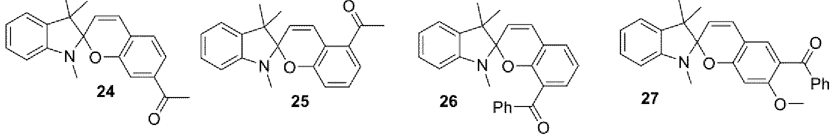

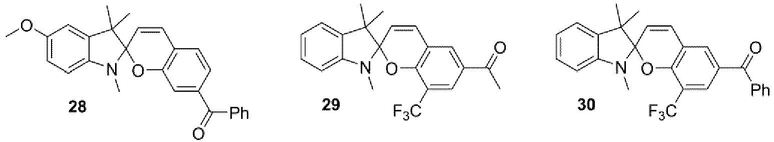

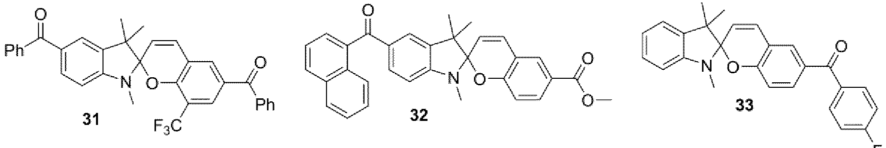

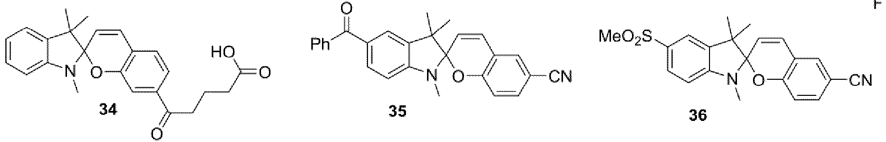

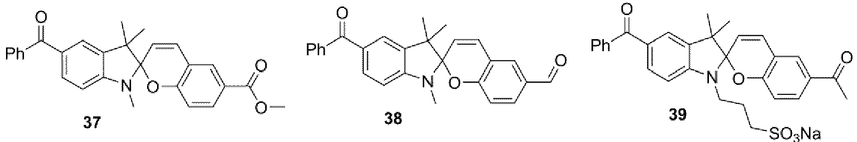

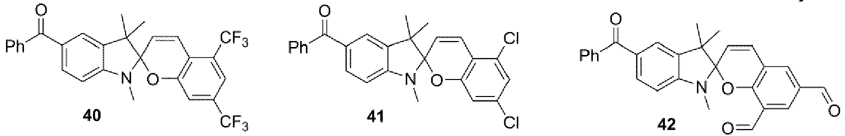

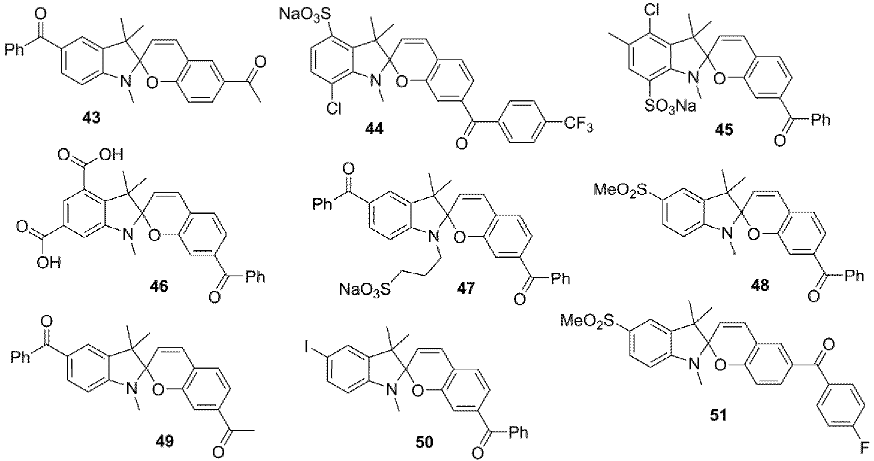

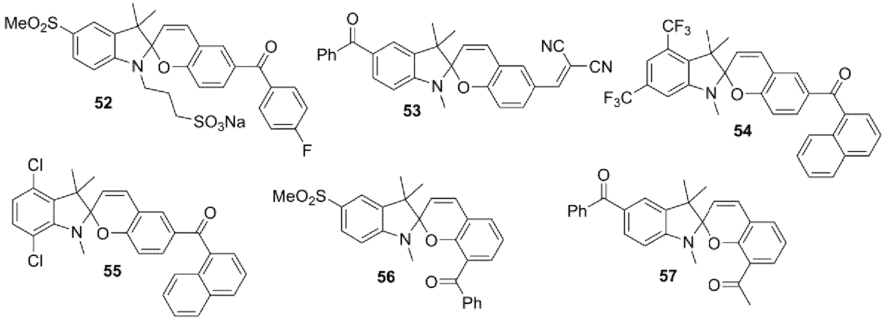

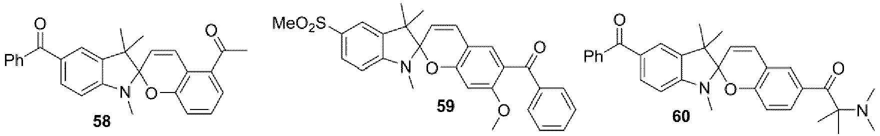

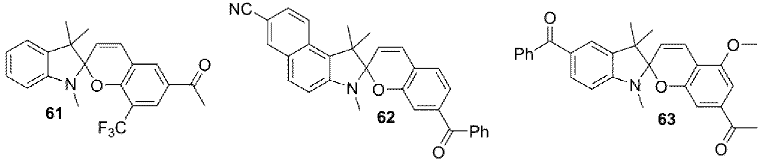

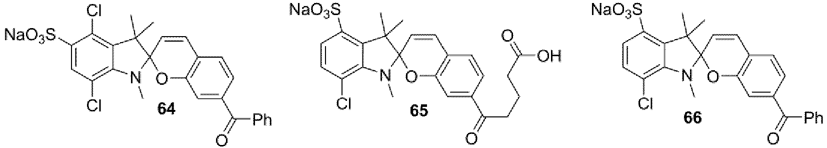

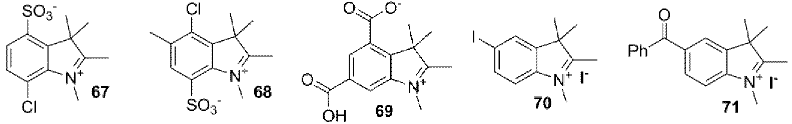

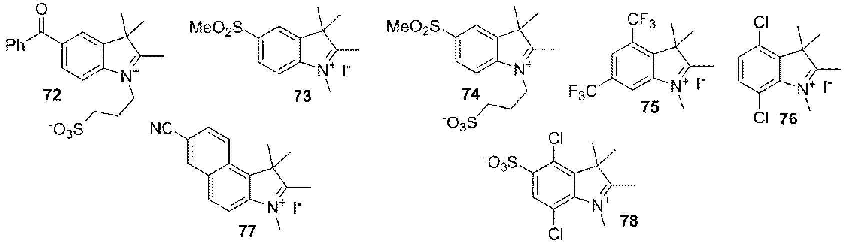

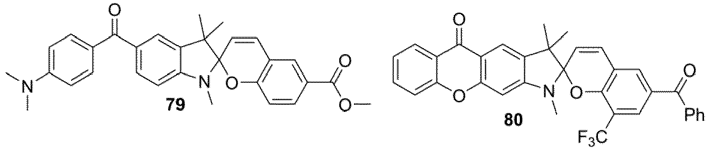

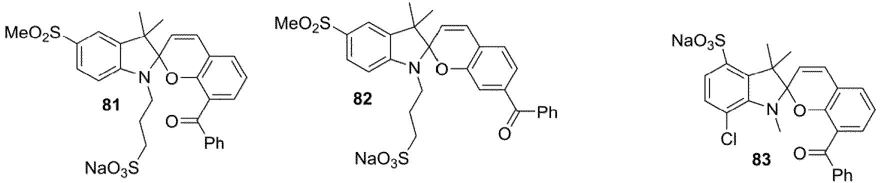

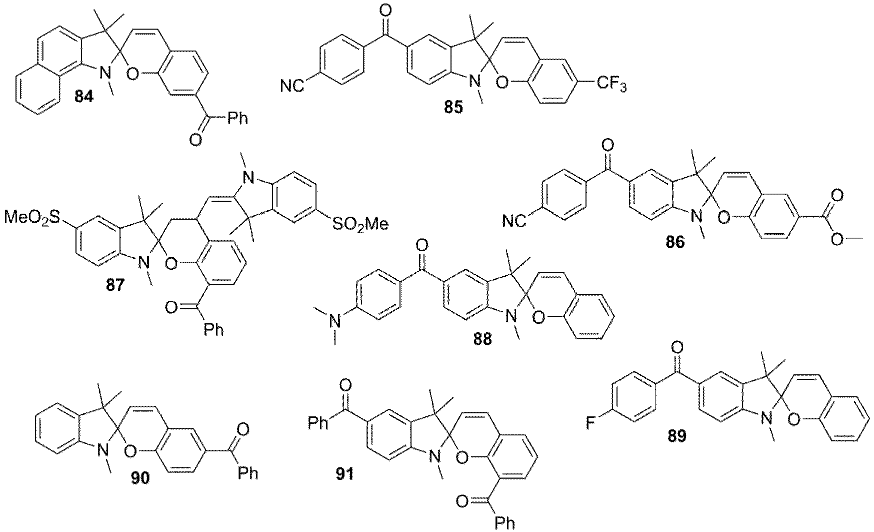

### ¹H-NMR Data

(The methylene group of the free Fischer Base and indolenium salts exchanges in the deuterated methanol and is not visible.)
**1** ¹H NMR (300 MHz, Methylene Chloride-*d*₂) δ 7.23 - 7.10 (m, 3H), 7.06 (m, 1H), 6.88 - 6.77 (m, 2H), 6.60 (d, *J =* 8.1 Hz, 1H), 6.52 (dd, *J =* 7.8, 0.8 Hz, 1H), 5.76 (d, *J =* 10.3 Hz, 1H), 2.71 (s, 3H), 1.28 (s, 3H), 1.15 (s, 3H).
**4** ¹H NMR (300 MHz, Methylene Chloride-*d*₂) δ 7.32 (dd, *J* = 8.0, 1.5 Hz, 1H), 7.17 (td, *J* = 7.6, 1.3 Hz, 1H), 7.12 - 7.01 (m, 2H), 6.90 - 6.80 (m, 2H), 6.73 (dd, *J =* 8.0, 7.5 Hz, 1H), 6.60 - 6.49 (m, 1H), 5.74 (d, *J =* 10.2 Hz, 1H), 2.72 (s, 3H), 1.30 (s, 3H), 1.16 (s, 3H).
**5** ¹H NMR (300 MHz, Methylene Chloride-*d*₂) δ 7.22 (s, 1H), 7.20 - 7.11 (m, 1H), 7.11 - 7.04 (m, 1H), 6.90 - 6.74 (m, 2H), 6.52 (dt, *J* = 7.7, 0.7 Hz, 1H), 6.38 (d, *J* = 0.6 Hz, 1H), 5.61 (d, *J* = 10.2 Hz, 1H), 3.76 (s, 3H), 2.72 (s, 3H), 1.29 (s, 3H), 1.15 (s, 3H).
**9** ¹H NMR (300 MHz, Methylene Chloride-*d*₂) δ 7.52 - 7.43 (m, 2H), 7.40 (d, *J =* 2.5 Hz, 1H), 7.33 (d, *J* = 1.8 Hz, 1H), 6.88 (d, *J* = 10.4 Hz, 1H), 6.33 (d, *J* = 8.1 Hz, 1H), 5.84 (d, *J* = 10.4 Hz, 1H), 2.65 (s, 3H), 1.27 (s, 3H), 1.17 (s, 3H).**11** ¹H NMR (500 MHz, Chloroform-d) δ 7.35 (ddd, *J =* 8.5, 2.3, 0.8 Hz, 1H), 7.31 (d, *J =* 2.2 Hz, 1H), 7.27 (dd, *J =* 8.2, 2.0 Hz, 1H), 7.15 (d, *J* = 2.0 Hz, 1H), 6.89 (dd, *J* = 10.4, 0.7 Hz, 1H), 6.77 (d, *J* = 8.5 Hz, 1H), 6.41 (d, *J* = 8.2 Hz, 1H), 5.75 (d, *J =* 10.3 Hz, 1H), 2.70 (s, 3H), 1.27 (s, 3H), 1.17 (s, 3H).
**12** ¹H NMR (500 MHz, Methylene Chloride-*d*₂) δ 7.78 - 7.73 (m, 3H), 7.56 (d, *J* = 1.9 Hz, 1H), 7.00 (dd, *J* = 10.4, 0.7 Hz, 1H), 6.74 (dt, *J* = 8.2, 0.7 Hz, 1H), 6.62 (d, *J* = 8.3 Hz, 1H), 5.77 (d, *J* = 10.3 Hz, 1H), 3.03 (s, 3H), 2.82 (s, 3H), 2.51 (s, 3H), 1.32 (s, 3H), 1.20 (s, 3H).
**13** ¹H NMR (500 MHz, Methylene Chloride-*d*₂) δ 7.72 (dd, *J* = 8.4, 3.1, 4H), 7.70 (d, *J* = 8.0 Hz, 2H), 7.68 - 7.52 (m, 3H), 7.50 - 7.42 (m, 4H), 6.96 (dd, *J* = 10.4, 0.7 Hz, 1H), 6.79 (dd, *J* = 8.2, 1.0 Hz, 1H), 6.60 (dd, *J* = 8.0, 0.8 Hz, 1H), 5.80 (d, *J* = 10.1 Hz, 1H), 2.86 (s, 3H), 1.36 (s, 3H), 1.23 (s, 3H).
**14** ¹H NMR (500 MHz, Methylene Chloride-*d*₂) δ 7.80 - 7.72 (m, 4H), 7.71 (d, *J* = 7.8 Hz, 2H), 7.68 - 7.60 (m, 2H), 7.57 - 7.51 (m, 2H), 7.49 (d, *J* = 7.5 Hz, 4H), 6.98 (d, *J* = 10.4 Hz, 1H), 6.79 (d, *J* = 8.2, 1H), 6.58 (dd, *J* = 8.1, 0.9 Hz, 1H), 5.76 (d, *J* = 10.2 Hz, 1H), 2.87 (s, 3H), 1.37 (s, 3H), 1.22 (s, 3H).
**18** ¹H NMR (500 MHz, Methylene Chloride-*d*₂) δ 8.01 - 7.96 (m, 2H), 7.96 - 7.92 (m, 1H), 7.79 (d, *J* = 1.8 Hz, 1H), 7.58 (dd, *J =* 8.2, 1.8 Hz, 1H), 7.56 - 7.46 (m, 4H), 7.13 (ddd, *J* = 8.1, 7.3, 1.7 Hz, 1H), 7.09 (dd, *J* = 7.5, 1.7 Hz, 1H), 6.92 (dd, *J* = 10.2, 0.7 Hz, 1H), 6.86 (td, *J* = 7.4, 1.1 Hz, 1H), 6.72 (dt, *J* = 8.1, 0.9 Hz, 1H), 6.44 (d, *J* = 8.2 Hz, 1H), 5.69 (d, *J* = 10.2 Hz, 1H), 2.81 (s, 3H), 1.32 (s, 3H), 1.19 (s, 3H).
**19** ¹H NMR (300 MHz, Methylene Chloride-*d*₂) δ 7.24 - 7.01 (m, 3H), 7.00 - 6.76 (m, 2H), 6.75 - 6.62 (m, 1H), 6.62 - 6.47 (m, 2H), 5.82 - 5.67 (m, 1H), 2.74 (s, 3H), 2.23 (s, 6H), 1.29 (s, 3H), 1.25 (s, 6H), 1.17 (s, 3H).
**20** ¹H NMR (300 MHz, Methylene Chloride-*d*₂) δ 7.80 - 7.68 (m, 2H), 7.61 - 7.52 (m, 1H), 7.51 - 7.42 (m, 2H), 7.30 (dd, *J* = 7.8, 1.6 Hz, 1H), 7.25 - 7.10 (m, 3H), 7.10 - 7.04 (m, 1H), 6.97 (dd, *J =* 10.3, 0.8 Hz, 1H), 6.82 (td, *J =* 7.4, 1.0 Hz, 1H), 6.57 - 6.50 (m, 1H), 5.87 (d, *J* = 10.3 Hz, 1H), 2.75 (s, 3H), 1.32 (s, 3H), 1.18 (s, 3H).
**21** ¹H NMR (300 MHz, Methylene Chloride-*d*₂) δ 7.87 - 7.79 (m, 2H), 7.76 - 7.69 (m, 2H), 7.32 (dd*, J =* 7.8, 1.6 Hz, 1H), 7.22 (d, *J =* 7.8 Hz, 1H), 7.15 (td, *J* = 7.6, 1.3 Hz, 1H), 7.11 - 7.04 (m, 2H), 6.98 (dd, *J* = 10.3, 0.8 Hz, 1H), 6.82 (td, *J* = 7.4, 1.0 Hz, 1H), 6.52 (dd, *J* = 7.8, 0.8 Hz, 1H), 5.89 (d, *J =* 10.3 Hz, 1H), 2.74 (s, 3H), 1.30 (s, 3H), 1.18 (s, 3H).
**22** ¹H NMR (300 MHz, Methylene Chloride-*d*₂) δ 8.07 - 7.92 (m, 3H), 7.71 - 7.44 (m, 6H), 7.16 (td, *J =* 7.7, 1.3 Hz, 1H), 7.12 - 7.05 (m, 1H), 6.95 - 6.79 (m, 2H), 6.78 - 6.71 (m, 1H), 6.62 - 6.49 (m, 1H), 5.78 (d, *J =* 10.3 Hz, 1H), 2.74 (s, 3H), 1.30 (s, 3H), 1.17 (s, 3H).
**23** ¹H NMR (300 MHz, Methylene Chloride-*d*₂) δ 7.61 (ddd, *J* = 7.9, 1.7, 0.3 Hz, 1H), 7.25 (dd, *J =* 7.3, 1.8 Hz, 1H), 7.16 (td, *J* = 7.6, 1.3 Hz, 1H), 7.09 (ddd, *J =* 7.3, 1.3, 0.6 Hz, 1H), 6.97 (d, *J =* 10.4 Hz, 1H), 6.92 - 6.80 (m, 2H), 6.54 (d, *J* = 7.7 Hz, 1H), 5.85 (d, *J =* 10.4 Hz, 1H), 2.72 (s, 3H), 2.00 (s, 3H), 1.31 (s, 3H), 1.21 (s, 3H).
**24** ¹H NMR (300 MHz, Methylene Chloride-*d*₂) δ 7.44 (dd, *J* = 7.8, 1.7 Hz, 1H), 7.33 - 7.27 (m, 1H), 7.23 - 7.14 (m, 2H), 7.09 (ddd, *J =* 7.3, 1.3, 0.6 Hz, 1H), 6.95 (dd, *J =* 10.3, 0.8 Hz, 1H), 6.85 (td, *J =* 7.4, 1.0 Hz, 1H), 6.58 - 6.51 (m, 1H), 5.86 (d, *J =* 10.2 Hz, 1H), 2.73 (s, 3H), 2.48 (s, 3H), 1.31 (s, 3H), 1.18 (s, 3H).
**25** ¹H NMR (300 MHz, Methylene Chloride-*d*₂) δ 7.66 (dd, *J* = 10.7, 0.8 Hz, 1H), 7.27 (dd, *J* = 7.7, 1.2 Hz, 1H), 7.23 - 7.11 (m, 2H), 7.11 - 7.03 (m, 1H), 6.92 - 6.79 (m, 2H), 6.55 - 6.47 (m, 1H), 5.81 (d, *J =* 10.7 Hz, 1H), 2.72 (s, 3H), 2.58 (s, 3H), 1.28 (s, 3H), 1.16 (s, 3H).
**26** ¹H NMR (300 MHz, Methylene Chloride-*d*₂) δ 7.48 - 7.36 (m, 3H), 7.28 - 7.15 (m, 4H), 7.01 (td, *J =* 7.6, 1.3 Hz, 1H), 6.96 - 6.88 (m, 2H), 6.88 - 6.80 (m, 1H), 6.70 (td, *J =* 7.4, 1.0 Hz, 1H), 6.24 (dd, *J =* 7.7, 0.8 Hz, 1H), 5.70 (d, *J* = 10.3 Hz, 1H), 2.55 (s, 3H), 1.07 (s, 3H), 1.05 (s, 3H).
**27** ¹H NMR (300 MHz, Methylene Chloride-*d*₂) δ 7.79 - 7.70 (m, 2H), 7.60 - 7.51 (m, 1H), 7.50 - 7.39 (m, 2H), 7.24 - 7.06 (m, 3H), 6.91 - 6.79 (m, 2H), 6.59 - 6.53 (m, 1H), 6.40 (d, *J* = 0.6 Hz, 1H), 5.64 (d, *J* = 10.2 Hz, 1H), 3.58 (s, 3H), 2.77 (s, 3H), 1.34 (s, 3H), 1.18 (s, 3H).
**28** ¹H NMR (300 MHz, Methylene Chloride-*d*₂) δ 7.76 - 7.65 (m, 2H), 7.60 - 7.51 (m, 1H), 7.49 - 7.40 (m, 2H), 7.29 (dd, *J* = 7.8, 1.6 Hz, 1H), 7.19 (d, *J* = 7.8 Hz, 1H), 7.13 - 7.07 (m, 1H), 6.95 (dd, *J* = 10.3, 0.8 Hz, 1H), 6.73 - 6.61 (m, 2H), 6.48 - 6.38 (m, 1H), 5.86 (d, *J* = 10.2 Hz, 1H), 3.75 (s, 3H), 2.69 (s, 3H), 1.29 (s, 3H), 1.18 (s, 3H).
**29** ¹H NMR (500 MHz, Methylene Chloride-*d*₂) δ 8.01 - 7.97 (m, 1H), 7.89 (d, *J* = 2.1 Hz, 1H), 7.18 (td, *J* = 7.7, 1.3 Hz, 1H), 7.09 (ddd, *J* = 7.3, 1.3, 0.5 Hz, 1H), 7.01 (d, *J* = 10.4 Hz, 1H), 6.88 (td, *J* = 7.4, 1.0 Hz, 1H), 6.55 (dt, *J* = 7.8, 0.7 Hz, 1H), 5.90 (d, *J* = 10.4 Hz, 1H), 2.69 (s, 3H), 2.54 (s, 3H), 1.31 (s, 3H), 1.20 (s, 3H).
**30** ¹H NMR (500 MHz, Methylene Chloride-*d*₂) δ 7.85 - 7.80 (m, 2H), 7.74 - 7.70 (m, 2H), 7.32 (dd, *J* = 7.8, 1.6 Hz, 1H), 7.22 (d, *J* = 7.8 Hz, 1H), 7.15 (td, *J* = 7.7, 1.3 Hz, 1H), 7.10 (dd, *J =* 1.7, 0.7 Hz, 1H), 7.06 (ddd, *J* = 7.2, 1.3, 0.6 Hz, 1H), 6.98 (dd, *J* = 10.2, 0.7 Hz, 1H), 6.82 (td, *J =* 7.4, 1.0 Hz, 1H), 6.52 (dt, *J =* 7.8, 0.7 Hz, 1H), 5.89 (d, *J =* 10.2 Hz, 1H), 2.74 (s, 3H), 1.31 (s, 3H), 1.18 (s, 3H).
**31** ¹H NMR (300 MHz, Methylene Chloride-*d*₂) δ 7.96 - 7.89 (m, 1H), 7.81 (d, *J* = 2.2 Hz, 1H), 7.79 - 7.68 (m, 6H), 7.67 - 7.59 (m, 1H), 7.59 - 7.37 (m, 5H), 7.04 (d, *J* = 10.5 Hz, 1H), 6.58 (d, *J* = 8.6 Hz, 1H), 5.92 (d, *J* = 10.4 Hz, 1H), 2.82 (s, 3H), 1.39 (s, 3H), 1.26 (s, 3H).
**32** ¹H NMR (500 MHz, Methylene Chloride-*d*₂) δ 8.06 - 7.91 (m, 2H), 7.88 (d, *J* = 1.7 Hz, 1H), 7.83 - 7.78 (m, 1H), 7.69 (dd, *J* = 8.2, 1.8 Hz, 1H), 7.59 (dd, *J* = 8.2, 1.8 Hz, 1H), 7.57 - 7.46 (m, 3H), 7.40 (t, *J* = 2.2 Hz, 1H), 7.16 (dd, *J* = 8.6, 2.5 Hz, 1H), 6.97 (dd, *J* = 10.3, 0.7 Hz, 1H), 6.85 (d, *J* = 8.2 Hz, 1H), 6.78 - 6.74 (m, 1H), 6.46 (d, *J* = 8.2 Hz, 1H), 5.77 (d, *J =* 10.3 Hz, 1H), 3.85 (s, 3H), 2.81 (s, 3H), 1.35 (s, 3H), 1.26 (s, 3H).
**33** ¹H NMR (500 MHz, Methylene Chloride-*d*₂) δ 7.81 - 7.75 (m, 2H), 7.61 - 7.54 (m, 2H), 7.23 - 7.13 (m, 3H), 7.08 (ddd, *J* = 7.3, 1.4, 0.5 Hz, 1H), 6.94 (dd, *J* = 10.3, 0.7 Hz, 1H), 6.85 (td, *J* = 7.4, 1.0 Hz, 1H), 6.77 (dt, *J* = 8.3, 0.7 Hz, 1H), 6.55 (dt, *J* = 7.8, 0.7 Hz, 1H), 5.81 (d, *J* = 10.3 Hz, 1H), 2.75 (s, 3H), 1.31 (s, 3H), 1.18 (s, 3H).
**34** ¹H NMR (500 MHz, Methanol-*d*₄) δ 7.46 (dd, *J =* 7.9, 1.7 Hz, 1H), 7.24 (d, *J =* 1.6 Hz, 1H), 7.22 (d, *J* = 7.9 Hz, 1H), 7.12 (td, *J* = 7.7, 1.3 Hz, 1H), 7.05 (dd, *J* = 7.3, 1.2 Hz, 1H), 7.01 (dd, *J* = 10.3, 0.7 Hz, 1H), 6.80 (td, *J* = 7.4, 1.0 Hz, 1H), 6.53 (d, *J* = 7.7 Hz, 1H), 5.92 (d, *J =* 10.2 Hz, 1H), 2.98 - 2.94 (m, 2H), 2.70 (s, 3H), 2.56 - 2.22 (m, 2H), 1.95 - 1.78 (m, 2H), 1.28 (s, 3H), 1.15 (s, 3H).
**35** ¹H NMR (300 MHz, Methylene Chloride-*d*₂) δ 7.82 - 7.66 (m, 4H), 7.62 - 7.39 (m, 5H), 6.93 (dd, *J* = 10.4, 0.7 Hz, 1H), 6.87 - 6.77 (m, 1H), 6.64 - 6.54 (m, 1H), 5.84 (d, *J* = 10.3 Hz, 1H), 2.83 (s, 3H), 1.32 (s, 3H), 1.21 (s, 3H).
**36** ¹H NMR (300 MHz, Methylene Chloride-*d*₂) δ 7.76 (dd, *J* = 8.3, 1.9 Hz, 1H), 7.56 (d, *J =* 1.9 Hz, 1H), 7.46 - 7.36 (m, 2H), 6.97 - 6.91 (m, 1H), 6.80 - 6.74 (m, 1H), 6.62 (d, *J* = 8.3 Hz, 1H), 5.82 (d, *J =* 10.3 Hz, 1H), 3.02 (s, 3H), 2.81 (s, 3H), 1.32 (s, 3H), 1.19 (s, 3H).
**37** ¹H NMR (300 MHz, Methylene Chloride-*d*₂) δ 7.81 (m, 2H), 7.77 - 7.65 (m, 4H), 7.60 - 7.53 (m, 1H), 7.52 - 7.44 (m, 2H), 6.98 (dd, *J* = 10.4, 0.7 Hz, 1H), 6.75 (dd, *J* = 9.1, 0.7 Hz, 1H), 6.60 - 6.50 (m, 1H), 5.78 (d, *J* = 10.3 Hz, 1H), 3.85 (s, 3H), 2.83 (s, 3H), 1.33 (s, 3H), 1.20 (s, 3H).
**38** ¹H NMR (300 MHz, Methylene Chloride-*d*₂) δ 9.84 (s, 1H), 7.81 - 7.63 (m, 6H), 7.62 - 7.53 (m, 1H), 7.53 - 7.45 (m, 2H), 7.02 (dd, *J =* 10.3, 0.7 Hz, 1H), 6.89 - 6.81 (m, 1H), 6.57 (dd, *J =* 7.8, 0.9 Hz, 1H), 5.83 (d, *J* = 10.3 Hz, 1H), 2.84 (s, 3H), 1.33 (s, 3H), 1.22 (s, 3H).
**39** ¹H NMR (300 MHz, Methanol-*d*₄) δ 7.88 - 7.80 (m, 2H), 7.75 - 7.57 (m, 5H), 7.57 - 7.47 (m, 2H), 7.10 (dd, *J =* 10.4, 0.7 Hz, 1H), 6.87 - 6.72 (m, 2H), 5.96 (d, *J* = 10.3 Hz, 1H), 3.58 - 3.37 (m, 2H), 2.83 (ddd, *J =* 8.0, 6.8, 3.1 Hz, 2H), 2.55 (s, 3H), 2.21 - 2.02 (m, 2H), 1.32 (s, 3H), 1.22 (s, 3H).
**40** ¹H NMR (500 MHz, Methylene Chloride-*d*₂) δ 7.75 - 7.73 (m, 1H), 7.73 (d, *J* = 1.5 Hz, 1H), 7.71 (dd, *J =* 8.1, 1.8 Hz, 1H), 7.68 (dd, *J* = 1.8, 0.5 Hz, 1H), 7.60 - 7.55 (m, 1H), 7.52 - 7.47 (m, 2H), 7.44 (dt, *J =* 1.5, 0.8 Hz, 1H), 7.27 (ddt, *J* = 10.7, 2.9, 1.9 Hz, 1H), 7.24 - 7.21 (m, 1H), 6.58 (d, *J =* 8.1 Hz, 1H), 6.04 (d, *J =* 10.7 Hz, 1H), 2.84 (s, 3H), 1.35 (s, 3H), 1.23 (s, 3H).
**41** ¹H NMR (500 MHz, Methylene Chloride-*d*₂) δ 7.73 (t, *J =* 1.4 Hz, 1H), 7.72 (d, *J =* 1.5 Hz, 1H), 7.69 (dd, *J =* 8.2, 1.8 Hz, 1H), 7.67 - 7.66 (m, 1H), 7.60 - 7.53 (m, 1H), 7.53 - 7.44 (m, 2H), 7.25 (dd, *J* = 10.5, 0.8 Hz, 1H), 6.95 (d, *J =* 2.0 Hz, 1H), 6.69 (dd, *J* = 2.0, 0.8 Hz, 1H), 6.55 (d, *J* = 8.1 Hz, 1H), 5.84 (d, *J* = 10.5 Hz, 1H), 2.82 (s, 3H), 1.32 (s, 3H), 1.20 (s, 3H).
**42** ¹H NMR (500 MHz, Methylene Chloride-*d*₂) δ 10.16 (s, 1H), 9.90 (s, 1H), 8.14 (d, *J =* 2.1 Hz, 1H), 7.88 (d, *J =* 2.1 Hz, 1H), 7.77 - 7.72 (m, 2H), 7.72 - 7.69 (m, 2H), 7.59 - 7.55 (m, 1H), 7.53 - 7.45 (m, 2H), 7.08 (d, *J* = 10.5 Hz, 1H), 6.60 (d, *J =* 8.7 Hz, 1H), 5.95 (d, *J* = 10.5 Hz, 1H), 2.88 (s, 3H), 1.35 (s, 3H), 1.29 (s, 3H).
**43** 1H NMR (500 MHz, Methylene Chloride-d2) δ 7.78 - 7.72 (m, 4H), 7.71 - 7.67 (m, 2H), 7.59 - 7.55 (m, 1H), 7.51 - 7.47 (m, 2H), 6.99 (dd, J *=* 10.3, 0.7 Hz, 1H), 6.77 (dt, J = 8.3, 0.7 Hz, 1H), 6.58 - 6.53 (m, 1H), 5.79 (d, J = 10.3 Hz, 1H), 2.84 (s, 3H), 2.51 (s, 3H), 1.33 (s, 3H), 1.21 (s, 3H).
**44** ¹H NMR (300 MHz, Methanol-*d*₄) δ 7.88 (d, *J* = 8.5 Hz, 2H), 7.82 (d, *J* = 8.0 Hz, 2H), 7.42 (d, *J =* 8.6 Hz, 1H), 7.32 - 7.22 (m, 2H), 7.17 - 7.05 (m, 3H), 5.96 (d, *J =* 10.4 Hz, 1H), 3.18 (s, 3H), 1.63 (s, 3H), 1.36 (s, 3H).
**45** ¹H NMR (300 MHz, Methanol-*d*₄) δ 8.28 (d, *J* = 1.9 Hz, 1H), 8.00 (d, *J* = 8.9 Hz, 1H), 7.85 (d, *J* = 8.5 Hz, 1H), 7.80 (dd, *J* = 9.0, 1.9 Hz, 1H), 7.75 - 7.69 (m, 1H), 7.62 - 7.55 (m, 1H), 7.48 (t*, J* = 7.4 Hz, 1H), 7.30 (d, *J =* 7.7 Hz, 1H), 7.25 (dd, *J =* 7.8, 1.6 Hz, 1H), 7.17 - 7.04 (m, 1H), 6.04 (d, *J =* 10.3 Hz, 1H), 3.11 (s, 3H), 2.89 (s, 3H), 1.66 (s, 3H), 1.36 (s, 3H).
**47** ¹H NMR (300 MHz, Methanol-*d*₄) δ 7.76 - 7.54 (m, 8H), 7.54 - 7.43 (m, 4H), 7.34 - 7.21 (m, 2H), 7.15 - 7.02 (m, 2H), 6.78 (d, *J =* 8.8 Hz, 1H), 6.03 (d, *J =* 10.2 Hz, 1H), 3.62 - 3.34 (m, 2H), 2.94 - 2.75 (m, 2H), 2.25 - 2.01 (m, 2H), 1.33 (s, 3H), 1.22 (s, 3H).
**49** ¹H NMR (300 MHz, Methylene Chloride-*d*₂) δ 7.80 - 7.66 (m, 4H), 7.63 - 7.54 (m, 1H), 7.53 - 7.43 (m, 3H), 7.35 - 7.25 (m, 1H), 7.20 (d, *J =* 7.9 Hz, 1H), 6.98 (dd, *J =* 10.3, 0.8 Hz, 1H), 6.55 (dd, *J =* 7.8, 0.8 Hz, 1H), 5.86 (d, *J =* 10.2 Hz, 1H), 2.83 (s, 3H), 2.49 (s, 3H), 1.35 (s, 3H), 1.21 (s, 3H).
**50** ¹H NMR (300 MHz, Methylene Chloride-*d*₂) δ 7.84 - 7.66 (m, 2H), 7.56 (m, 1H), 7.51 - 7.39 (m, 2H), 7.36 - 7.24 (m, 2H), 7.23 - 7.15 (m, 1H), 7.16 - 7.08 (m, 1H), 6.97 (dd, *J =* 10.3, 0.8 Hz, 1H), 6.33 (d, *J =* 8.3 Hz, 1H), 5.83 (d, *J =* 10.2 Hz, 1H), 2.72 (s, 3H), 1.28 (s, 3H), 1.17 (s, 3H).
**51** ¹H NMR (500 MHz, Methylene Chloride-*d*₂) δ 7.83 - 7.73 (m, 3H), 7.63 - 7.54 (m, 3H), 7.24 - 7.15 (m, 2H), 6.99 (dd, *J* = 10.4, 0.7 Hz, 1H), 6.77 (dt, *J =* 8.2, 0.8 Hz, 1H), 6.63 (d, *J* = 8.3 Hz, 1H), 5.79 (d, *J =* 10.3 Hz, 1H), 3.03 (s, 3H), 2.85 (s, 3H), 1.36 (s, 3H), 1.21 (s, 3H).
52 ¹H NMR (500 MHz, Methanol-*d*₄) δ 7.89 - 7.81 (m, 2H), 7.79 (dd, *J =* 8.4, 2.0 Hz, 1H), 7.70 - 7.60 (m, 3H), 7.35 - 7.24 (m, 2H), 7.11 (d, *J =* 10.3 Hz, 1H), 6.94 (d, *J =* 8.3 Hz, 1H), 6.82 (d, *J =* 8.4 Hz, 1H), 5.98 (d, *J* = 10.3 Hz, 1H), 3.61 - 3.42 (m, 2H), 3.12 (s, 3H), 2.93 - 2.78 (m, 2H), 2.23 - 2.03 (m, 2H), 1.38 (s, 3H), 1.25 (s, 3H).
**53** ¹H NMR (300 MHz, Methylene Chloride-*d*₂) δ 7.79 - 7.63 (m, 7H), 7.62 - 7.54 (m, 1H), 7.53 - 7.45 (m, 2H), 6.99 (d, *J =* 10.4 Hz, 1H), 6.85 (d, *J =* 8.5 Hz, 1H), 6.62 - 6.54 (m, 1H), 5.87 (d, *J =* 10.3 Hz, 1H), 2.85 (s, 3H), 1.33 (s, 3H), 1.22 (s, 3H).
**55** ¹H NMR (300 MHz, Methylene Chloride-*d*₂) δ 8.05 - 7.92 (m, 2H), 7.68 - 7.60 (m, 2H), 7.58 - 7.43 (m, 3H), 7.14 (d, *J* = 8.8 Hz, 1H), 7.03 (d, *J =* 8.6 Hz, 1H), 6.96 - 6.89 (m, 2H), 6.80 - 6.74 (m, 1H), 6.70 (d, *J =* 8.6 Hz, 1H), 5.69 (d, *J =* 10.3 Hz, 1H), 3.15 (s, 3H), 1.46 (s, 3H), 1.22 (s, 3H).
**56** ¹H NMR (300 MHz, Methylene Chloride-*d*₂) δ 7.59 (dd, *J =* 8.3, 1.9 Hz, 1H), 7.51 - 7.15 (m, 8H), 7.06 - 6.91 (m, 2H), 6.29 (d, *J* = 8.2 Hz, 1H), 5.68 (d, *J =* 10.3 Hz, 1H), 2.99 (s, 3H), 2.64 (s, 3H), 1.08 (s, 6H).
**57** ¹H NMR (300 MHz, Methylene Chloride-*d*₂) δ 7.82 - 7.41 (m, 8H), 7.28 (dd, *J* = 7.4, 1.8 Hz, 1H), 7.01 (d, *J =* 10.3 Hz, 1H), 6.92 (dd, *J =* 7.9, 7.3 Hz, 1H), 6.56 (d, *J =* 8.6 Hz, 1H), 5.85 (d, *J* = 10.3 Hz, 1H), 2.83 (s, 3H), 2.09 (s, 3H), 1.36 (s, 3H), 1.25 (s, 3H).
**58** ¹H NMR (300 MHz, Methylene Chloride-*d*₂) δ 7.78 - 7.64 (m, 5H), 7.61 - 7.53 (m, 1H), 7.52 - 7.44 (m, 2H), 7.31 (dd, *J =* 7.8, 1.2 Hz, 1H), 7.20 (t, *J* = 7.9 Hz, 1H), 6.89 (ddd, *J =* 8.1, 1.2, 0.8 Hz, 1H), 6.53 (dd, *J* = 7.9, 0.8 Hz, 1H), 5.82 (d, *J =* 10.7 Hz, 1H), 2.83 (s, 3H), 2.59 (s, 3H), 1.32 (s, 3H), 1.21 (s, 3H).
**59** ¹H NMR (300 MHz, Methylene Chloride-*d*₂) δ 7.83 - 7.66 (m, 3H), 7.63 - 7.49 (m, 2H), 7.50 - 7.36 (m, 2H), 7.21 (s, 1H), 6.91 (d, *J =* 10.3 Hz, 1H), 6.63 (d, *J =* 8.3 Hz, 1H), 6.38 (s, 1H), 5.62 (d, *J* = 10.2 Hz, 1H), 3.59 (s, 3H), 3.03 (s, 3H), 2.86 (s, 3H), 1.39 (s, 3H), 1.21 (s, 3H).
**60** ¹H NMR (300 MHz, Methylene Chloride-*d*₂) δ 7.79 - 7.65 (m, 6H), 7.62 - 7.53 (m, 1H), 7.49 (m, 2H), 7.22 - 7.14 (m, 1H), 6.79 - 6.66 (m, 1H), 6.63 - 6.50 (m, 1H), 5.74 (d, *J =* 10.2 Hz, 1H), 2.84 (s, 3H), 2.23 (s, 6H), 1.29 (s, 3H), 1.24 (s, 6H), 1.17 (s, 3H).
**61** ¹H NMR (300 MHz, Methylene Chloride-*d*₂) δ 8.02 (dd, *J* = 2.2, 0.7 Hz, 1H), 7.92 (d, *J* = 2.2 Hz, 1H), 7.79 - 7.68 (m, 4H), 7.62 - 7.53 (m, 1H), 7.53 - 7.45 (m, 2H), 7.05 (d, *J* = 10.5 Hz, 1H), 6.61 - 6.54 (m, 1H), 5.91 (d, *J =* 10.4 Hz, 1H), 2.79 (s, 3H), 2.55 (s, 3H), 1.36 (s, 3H), 1.25 (s, 3H).
**62** ¹H NMR (300 MHz, Methylene Chloride-*d*₂) δ 8.16 (d, *J* = 1.8 Hz, 1H), 8.00 - 7.92 (m, 1H), 7.85 - 7.78 (m, 1H), 7.75 - 7.67 (m, 2H), 7.59 - 7.38 (m, 4H), 7.34 - 7.18 (m, 2H), 7.13 - 6.98 (m, 3H), 5.93 (d, *J =* 10.3 Hz, 1H), 2.90 (s, 3H), 1.64 (s, 3H), 1.34 (s, 3H).
**63** ¹H NMR (500 MHz, Methylene Chloride-*d*₂) δ 7.77 - 7.65 (m, 4H), 7.59 - 7.54 (m, 1H), 7.53 - 7.46 (m, 2H), 7.33 - 7.25 (m, 1H), 7.03 (d, *J =* 1.4 Hz, 1H), 6.97 (s, 1H), 6.54 (d, *J =* 8.2 Hz, 1H), 5.79 (d, *J* = 10.5 Hz, 1H), 3.92 (s, 3H), 2.83 (s, 3H), 2.47 (s, 3H), 1.34 (s, 3H), 1.21 (s, 3H).
**64** ¹H NMR (300 MHz, Methanol-*d*₄) δ 7.83 (s, 1H), 7.77 - 7.68 (m, 2H), 7.67 - 7.58 (m, 1H), 7.50 (tt, *J* = 7.5, 0.8 Hz, 2H), 7.36 - 7.21 (m, 2H), 7.19 - 7.06 (m, 2H), 5.91 (d, *J =* 10.3 Hz, 1H), 3.20 (s, 3H), 1.51 (s, 3H), 1.28 (s, 3H).
**65** ¹H NMR (300 MHz, Methanol-*d*₄) δ 7.49 (dd, *J =* 7.8, 1.7 Hz, 1H), 7.43 (d, *J =* 8.6 Hz, 1H), 7.29 (d, *J =* 1.4 Hz, 1H), 7.22 (d, *J =* 7.9 Hz, 1H), 7.13 (d, *J =* 8.6 Hz, 1H), 7.10 - 7.04 (m, 1H), 5.91 (d, *J* = 10.3 Hz, 1H), 3.15 (s, 3H), 2.95 (q, *J* = 5.9, 4.7 Hz, 2H), 2.27 - 2.16 (m, 2H), 1.98 - 1.87 (m, 2H), 1.61 (s, 3H), 1.35 (s, 3H).
**66** ¹H NMR (300 MHz, Methanol-*d*₄) δ 7.77 - 7.68 (m, 2H), 7.65 - 7.57 (m, 1H), 7.54 - 7.46 (m, 2H), 7.42 (d, *J* = 8.6 Hz, 1H), 7.32 - 7.21 (m, 2H), 7.18 - 7.02 (m, 3H), 5.93 (d, *J =* 10.3 Hz, 1H), 3.18 (s, 3H), 1.64 (s, 3H), 1.36 (s, 3H).
**67** ¹H NMR (500 MHz, Methanol-*d*₄) δ 8.11 (d, *J =* 8.6 Hz, 1H), 7.71 (d, *J =* 8.6 Hz, 1H), 4.32 (s, 3H), 1.89 (s, 6H).
**68** ¹H NMR (300 MHz, Methanol-*d*₄) δ 8.16 (s, 1H), 4.57 (s, 3H), 2.51 (s, 3H), 1.76 (s, 6H).
**69** ¹H NMR (500 MHz, Methanol-*d*₄) δ 8.88 (d, *J =* 1.4 Hz, 1H), 8.60 (d, *J =* 1.4 Hz, 1H), 4.12 (s, 3H), 1.82 (s, 6H).
**70** ¹H NMR (300 MHz, Methanol-*d*₄) δ 8.19 (dd, *J* = 1.7, 0.5 Hz, 1H), 8.02 (dd, *J* = 8.4, 1.6 Hz, 1H), 7.65 - 7.60 (m, 1H), 4.02 (s, 3H), 1.60 (s, 6H).
**71** ¹H NMR (300 MHz, Methanol-*d*₄) δ 8.16 (t, *J =* 1.1 Hz, 1H), 8.00 (d, *J =* 1.1 Hz, 2H), 7.85 - 7.74 (m, 2H), 7.74 - 7.66 (m, 1H), 7.62 - 7.51 (m, 2H), 4.13 (s, 3H), 1.67 (s, 6H).
**72** ¹H NMR (300 MHz, Methanol-*d*₄) δ 8.20 - 8.05 (m, 1H), 8.03 - 7.84 (m, 1H), 7.84 - 7.60 (m, 2H), 7.60 - 7.39 (m, 2H), 7.36 - 7.00 (m, 2H), 4.85 - 4.21 (m, 2H), 3.12 - 2.75 (m, 2H), 2.45 - 1.92 (m, 2H), 1.71 - 1.45 (m, 6H).
**73** ¹H NMR (500 MHz, Methanol-*d*₄) δ 8.43 (s, 1H), 8.31 (d, *J =* 8.3 Hz, 1H), 8.17 (s, 1H), 4.19 (s, 3H), 3.33 (s, 3H), 1.75 (s, 6H).
**74** ¹H NMR (500 MHz, Deuterium Oxide) δ 8.38 (s, 1H), 8.26 (d, *J* = 8.5 Hz, 1H), 8.13 (d, *J* = 8.7 Hz, 1H), 3.34 (s, 3H), 3.14 (t, *J* = 6.9 Hz, 2H), 2.42 (p, *J* = 7.3 Hz, 2H), 2.20 - 1.99 (m, 2H), 1.66 (s, 6H).
**75** ¹H NMR (300 MHz, Methanol-*d*₄) δ 8.64 (s, 1H), 8.27 (s, 1H), 4.18 (s, 3H), 1.75 (s, 6H).
**76** ¹H NMR (300 MHz, Methanol-*d*₄) δ 8.00 (s, 1H), 7.66 (s, 1H), 4.31 (s, 3H), 1.76 (s, 6H).
**77** ¹H NMR (300 MHz, Methanol-*d*₄) δ 8.69 (s, 1H), 8.51 (dd, *J* = 8.8, 0.9 Hz, 1H), 8.45 - 8.32 (m, 1H), 8.16 (d, *J =* 9.0 Hz, 1H), 7.98 (dd, *J =* 8.8, 1.7 Hz, 1H), 4.19 (s, 3H), 1.85 (s, 6H).
**79** ¹H NMR (500 MHz, Methylene Chloride-*d*₂) δ 7.82 - 7.78 (m, 2H), 7.77 - 7.72 (m, 2H), 7.66 (dd, *J* = 8.1, 1.7 Hz, 1H), 7.57 (d, *J* = 1.7 Hz, 1H), 6.97 (dd, *J* = 10.4, 0.7 Hz, 1H), 6.78 - 6.74 (m, 1H), 6.74 - 6.69 (m, 2H), 6.55 (d, *J* = 8.1 Hz, 1H), 5.78 (d, *J =* 10.3 Hz, 1H), 3.85 (s, 3H), 3.06 (s, 6H), 2.82 (s, 3H), 1.32 (s, 3H), 1.20 (s, 3H).
**80** ¹H NMR (500 MHz, Methylene Chloride-*d*₂) δ 8.27 (dd, *J =* 8.0, 1.8 Hz, 1H), 8.22 (d, *J* = 8.4 Hz, 1H), 7.91 (d, *J* = 2.1 Hz, 1H), 7.83 (d, *J* = 2.1 Hz, 1H), 7.76 (dd, *J* = 8.2, 1.4 Hz, 2H), 7.70 (ddd, *J =* 8.7, 7.1, 1.8 Hz, 1H), 7.66 - 7.61 (m, 1H), 7.53 (t, *J =* 7.7 Hz, 2H), 7.46 (dd, *J =* 8.4, 1.0 Hz, 1H), 7.36 (ddd, *J =* 8.1, 7.1, 1.1 Hz, 1H), 7.07 (d, *J =* 10.4 Hz, 1H), 6.69 (d, *J =* 8.4 Hz, 1H), 5.94 (d, *J =* 10.4 Hz, 1H), 2.87 (s, 3H), 1.69 (s, 3H), 1.42 (s, 3H).
**81** ¹H NMR (500 MHz, Methanol-*d*₄) δ 7.62 - 7.59 (m, 1H), 7.45 - 7.42 (m, 3H), 7.40 (d, *J* = 7.6 Hz, 1H), 7.36 - 7.34 (m, 2H), 7.33 - 7.27 (m, 2H), 7.11 (d, *J* = 10.3 Hz, 1H), 7.05 (t, *J =* 7.6 Hz, 1H), 6.58 (d, *J* = 8.4 Hz, 1H), 5.90 (d, *J* = 10.3 Hz, 1H), 3.58 - 3.39 (m, 2H), 3.09 (s, 3H), 2.86 - 2.76 (m, 2H), 2.09 - 1.97 (m, 2H), 1.34 (s, 3H), 1.13 (s, 3H).
**82** ¹H NMR (500 MHz, Methanol-*d*₄) δ 7.77 (ddd, *J* = 11.0, 8.4, 1.7 Hz, 3H), 7.68 - 7.59 (m, 2H), 7.58 - 7.50 (m, 2H), 7.35 (d, *J =* 7.8 Hz, 1H), 7.30 (dd, *J* = 7.8, 1.6 Hz, 1H), 7.15 (d, *J =* 10.3 Hz, 1H), 7.07 (d, *J =* 1.4 Hz, 1H), 6.93 (d, *J =* 8.4 Hz, 1H), 6.06 (d, *J =* 10.3 Hz, 1H), 3.59 - 3.43 (m, 2H), 3.12 (s, 3H), 2.91 - 2.80 (m, 2H), 2.23 - 2.06 (m, 2H), 1.39 (s, 3H), 1.26 (s, 3H).
**83** ¹H NMR (500 MHz, Methanol-*d*₄) δ 7.43 - 7.33 (m, 5H), 7.30 (d, *J* = 8.6 Hz, 1H), 7.24 (dd, *J =* 8.7, 6.9 Hz, 2H), 7.05 (d, *J =* 10.4 Hz, 1H), 7.02 - 6.95 (m, 2H), 5.72 (d, *J =* 10.4 Hz, 1H), 2.97 (s, 3H), 1.44 (s, 3H), 1.24 (s, 3H).
**84** ¹H NMR (300 MHz, Methylene Chloride-*d*₂) δ 8.45 - 8.31 (m, 1H), 7.90 - 7.77 (m, 1H), 7.77 - 7.68 (m, 2H), 7.60 - 7.49 (m, 1H), 7.49 - 7.28 (m, 7H), 7.23 (t, *J =* 8.3 Hz, 1H), 7.14 - 6.98 (m, 2H), 5.96 (d, *J =* 10.3 Hz, 1H), 3.37 (s, 3H), 1.39 (s, 3H), 1.19 (s, 3H).
**87** ¹H NMR (500 MHz, Methylene Chloride-*d*₂) δ 7.72 (dd, *J* = 8.4, 1.9 Hz, 1H), 7.61 (dd, *J* = 8.3, 1.9 Hz, 1H), 7.59 (d, *J =* 1.9 Hz, 1H), 7.50 - 7.47 (m, 2H), 7.45 (ddd, *J* = 7.5, 1.6, 0.8 Hz, 1H), 7.42 - 7.37 (m, 1H), 7.35 (d, *J =* 1.9 Hz, 1H), 7.28 - 7.21 (m, 3H), 6.96 (t*, J* = 7.6 Hz, 1H), 6.64 (d, *J =* 8.4 Hz, 1H), 6.36 (d, *J =* 8.3 Hz, 1H), 4.42 (d, *J* = 10.2 Hz, 1H), 3.13 (s, 3H), 3.02 (s, 3H), 2.99 (s, 3H), 2.54 (s, 3H), 2.37 (dd, *J =* 14.2, 4.9 Hz, 1H), 2.20 - 2.10 (m, 1H), 1.67 (s, 3H), 1.65 (s, 3H), 1.31 (s, 3H), 1.25 (s, 3H), 1.22 - 1.18 (m, 1H).
**91** 1H NMR (500 MHz, Methylene Chloride-d2) δ 7.72 - 7.68 (m, 2H), 7.58 - 7.54 (m, 2H), 7.53 - 7.47 (m, 4H), 7.41 (d, J = 1.8 Hz, 1H), 7.40 - 7.37 (m, 1H), 7.31 (dd, J = 7.6, 1.7 Hz, 1H), 7.27 (dd, J = 7.5, 1.7 Hz, 1H), 7.26 - 7.22 (m, 2H), 6.97 (dd, J = 8.9, 1.4 Hz, 2H), 6.25 (d, J = 8.2 Hz, 1H), 5.70 (d, J = 10.3 Hz, 1H), 2.66 (s, 3H), 1.09 (s, 3H), 1.08 (s, 3H).

### B. Formulation

In several cases it may be advantageous, when the dual color photoinitiator is lyophilized before formulating.

### Example Formulation 1

Initiator **66** (1 mg) was dissolved in a mixture of methyldiethanolamine (1 g), PEG-diacrylate (MW575) (1 g) and gelatine methacrylate (8 g). The mixture was shaken at 50°C until the components were well mixed and allowed to cool to room temperature before printing.

### Example Formulation 2

Initiator **43** (2 mg) was dissolved in methyldiethanolamine (0.4 g) and diurethane dimethacrylate, mixture of isomers, CAS 72869-86-4 (10 g). The mixture was stirred until homogenization and could be directly used for printing.

### Example Formulation 3

Initiator **55** (2 mg) was dissolved in methyldiethanolamine (0.4 g) and diurethane dimethacrylate, mixture of isomers, CAS 72869-86-4 (10 g). The mixture was stirred until homogenization and could be directly used for printing.

### Example Formulation 4

Initiator **56** (10 mg) was dissolved in acryloyl morpholine (0.4 g), methyldiethanolamine (0.4 g) and diurethane dimethacrylate, mixture of isomers, CAS 72869-86-4 (10 g). The mixture was stirred until homogenization and could be directly used for printing.

### C. Printing

The above described formulations are used for volumetric printing.

The setup for volumetric printing is as follows: a cuvette with four transparent windows is irradiated in one direction with a light sheet of wavelength 1, while an image is projected onto the light sheet from a different angle with wavelength 2. The image is changed to produce a movie, while the light sheet is moved through the cuvette. The prints result in solidification only in volumes where the light of both wavelengths intersects. The residual uncured resin is removed to obtain the shaped body, which is further washed with solvent and post-processed.

It has been found that Formulation 1, Formulation 2, Formulation 3 and Formulation 4 were successful for volumetric printing under the use of light of two different wavelengths. Exemplarily the following wavelengths can be used:
Formulation 1,3,4: 375 nm and 500-700 nm
Formulation 2: 405 nm and 500-700 nm

The post-processing is exemplarily described for the shaped body produced from Formulation 4:
Post-processing example 1: After washing with ethanol, the three-dimensional object is subjected to irradiation with light of a wavelength of 420 nm for 30 min (Thorlabs M420L3, 750 mW) which results in that the absorption of the object in the visible wavelength range is significantly reduced.
Post-processing example 2: After washing with ethanol, the three-dimensional object is placed in a heatable chamber in which the three-dimensional object is tempered at 120°C for 30 min which results in that the absorption in the visible wavelength range is significantly reduced.

### Dual color photoinitiation properties depending on the spiropyran motif

In an example it has been found that, when R⁴ is H and R¹¹ is benzoyl the resulting spiropyran is not suitable as dual color photoinitiator due to very inefficient switching reflected in a low quantum yield and a very slow thermal back reaction, which does not allow for prints with z-resolution.

When R⁴ and R¹¹ are benzoyl, the resulting spiropyran is suitable as dual color photoinitiator, with a sufficiently fast thermal back reaction, a sufficiently high switching efficiency and a sufficiently high reactivity to allow volumetric printing in the xolography process.

When R⁴ and R¹³ (structure **91**) are benzoyl which is accessible with the method described here in, a similar switching efficiency but surprisingly also an enhanced thermal back reaction rate and a higher reactivity are observed.

Furthermore, it has been found that, when R⁴ is benzoyl the resulting motif surprisingly results in an extinction coefficient at 405 nm which is sufficiently high to use 405 nm as the first wavelength necessary for switching to the intermediate state.

In contrast, when R¹¹ is benzoyl and R⁴ is H, the extinction coefficient at 405 nm is not sufficiently high, so that the first wavelength needs to be shifted to 375 nm. An irradiation with the first wavelength being 405 nm may be advantageous compared to 375 nm, since laser diodes are available with higher power at lower costs. Furthermore, 405 nm is less harmful to cells, which may be of high relevance when prints shall be performed in the presence of cells. When R¹¹ is acyl the extinction coefficient at 405 nm is not sufficient for printing but the reactivity of the intermediate state with visible light and a co-initiator is surprisingly high, compared to respective structures where R¹¹ is benzoyl, ester, cyano or CF₃. The combination of R⁴ being benzoyl and R¹¹ being acyl (**43**) results in a surprisingly well performing dual color photoinitiator which has superior properties compared to dual color photoinitiators with just one carbonyl group or two identical carbonyl substituents.

In another example it has been found that, when R⁴ is SO₂Me and R¹¹ is 4-F-benzoyl (**51**), the switching is surprisingly efficient, with a quantum yield near unity and the thermal back reaction is accelerated to the few seconds thermal half-life regime, allowing for decent print performance. When R⁴ is SO₂Me and R¹³ is benzoyl (instead of R¹¹) (**56**), the switching is as efficient with a quantum yield near unity, but surprisingly the thermal back reaction rate as well as the initiation efficiency are increased even further, so that enhanced print performance over **51** regarding resolution, print speed and hardness of the green state is achieved.

From large sets of examples design rules for specific properties can be derived, exemplarily:
- The back reaction rate as well as the switching efficiency may be accelerated with stronger acceptors at R⁴.
- A benzoyl substituent may cause a bathochromic shift of the spiropyran absorption spectrum from strongest to weakest: R⁴>R¹²>R¹¹>R¹³>R¹⁰
- A benzoyl substituent on the pyran ring may cause a suitable thermal back reaction rate in the order from high to low: R¹⁰>R¹²>R¹³>R¹¹

The features disclosed in the description and claims may be relevant to the realization of the various designs either individually or in any combination.

### General findings for the printing process

The photoinitiators **43, 48, 51** and **56** have been investigated in terms of printing at different concentrations of dual color photoinitiators and with variations of the first wavelength. The initiators each bear one aroyl substituent in a different position, which causes shifts in the absorption spectra of the initial state. Volumetric printing is performed in a setup, where a cuvette with four transparent windows is irradiated in one direction with a light sheet of wavelength 1, while an image is projected onto the light sheet from a perpendicular direction with wavelength 2. The image is changed to produce a movie, while the cuvette is moved through the light sheet. The prints may result in solidification only in volumes where the light of both wavelengths intersects. The residual uncured resin is removed to obtain the shaped body, which is further washed with solvent and post-processed.

**Table 1: Extinction coefficients in L mol⁻¹ cm⁻¹ of dual color photoinitiators in the tail region of their absorption spectrum of the initial state and thermal half-life at room temperature. Measurements have been performed in a typical resin suited for volumetric printing: urethane dimethacrylate containing 3.5% N-methyldiethanolamine and 4.5% acryloyl morpholine.**

| Dual color photoinitiator | 405 nm | 395 nm | 385 nm | 375 nm | Thermal half-life |
|---|---|---|---|---|---|
| **43** | 260 | 640 | 1700 | 4100 | 15 s |
| **48** | 90 | 170 | 490 | 1500 | 2.8 s |
| **51** | <10 | 60 | 150 | 350 | 10 s |
| **56** | <10 | <10 | 40 | 160 | 3.4 s |

While photochemistry is ideally performed at a wavelength, where the absorbing species has its absorption maximum, surprisingly the tests did not result in successful prints when the light sheet is generated at a wavelength around the absorption maximum of the initial state. Instead, only curing at the walls of the cuvette is observed. This may be explained by Beer's law, since at the wavelength around the maximum, the photons are absorbed in close proximity to the cuvette wall, causing also the polymerization in close proximity to the cuvette wall, while only a minor fraction of the light reaches the middle of the cuvette.

To circumvent this problem, the concentration has been lowered, so that the absorption at the absorption maximum/first wavelength has been reduced to 1 or less.

Surprisingly, the penetration depth issue has been solved, but no curing was observed at all. Presumably, the concentration of the dual color photoinitiator has been too low in these experiments to allow the formation of a sufficient number of radicals.

Surprisingly, prints in the middle of the cuvette have been successful without curing at the walls, when the first wavelength was chosen to irradiate in the red tail of the absorption spectrum, where the initial state has a lower extinction coefficient. This is counter-intuitive, as one would expect less radicals to be formed, when the initiator absorbs less photons, but surprisingly more radicals are formed in the middle of the cuvette. However, when the first wavelength is shifted further, where the initial state has an even lower extinction coefficient, no curing is observed anymore or the intensity is largely increased, resulting in only curing at the cuvette walls. When the concentration is increased to an extent, that curing can be observed, the curing is caused mainly at the walls again. This may be explained by little impurities, with a relatively higher extinction coefficient than the initial state or the relatively higher intensity of the first wavelength necessary to cause the switching. By comparing prints of different concentrations and first wavelengths, the following conditions have been found to result in improved print results compared to the state of the art. When the first wavelength is adjusted accordingly to the extinction coefficient, the concentration of the initiator can be sufficiently increased to achieve a dual color effect. Surprisingly, these conditions are independent of the structure of the initiator itself, as they rather seem to be related to the surprising combination of restrictions given by Beer's law and the dual color effect:
1) The extinction coefficient of the dual color photoinitiator at the first wavelength should be in the range of 5000 - 0 L mol⁻¹ cm⁻¹, preferably 2500 - 10 L mol⁻¹ cm⁻¹ , more preferably 1000 - 20 L mol⁻¹ cm⁻¹ , even more preferably 500 - 50 L mol⁻¹ cm⁻¹, most preferably 300 - 100 L mol⁻¹ cm⁻¹.
2) The absorption of the resin at the first wavelength over a distance of 1 cm, using a non-absorbing solvent as reference, e.g. acetonitrile should be in the range of 1-0.05, preferably 0.9-0.1, more preferably 0.8-0.15.

For the Initiators given in Table 1, the ideal first wavelengths are: **43:** 405 nm, **48:** 395 nm, **51:** 385 nm, and **56:** 375 nm.

Surprisingly, the preferred properties of the initiator and the resin are independent of the object which shall be printed but rely on the size of the container and the path length the light of the first wavelength has to travel. Since Beer's law involves the path length of the light, the problem of penetration depth is of lower importance for smaller build rooms, e.g. 1x1x1 mm³, while for build rooms >5x5x5 mm³, preferably >10x10x10 mm³, the above criteria ensure a highly improved print performance. To give a non-limiting example, when formulations containing 51 are dual color polymerized, no curing is observed using 405 nm as first wavelength, while 395 nm and 375 nm give green states of acceptable quality, the highest degree of polymerization was achieved using 385 nm. Furthermore, the adaption of the extinction coefficient allowed to increase the build room and still maintain sufficiently polymerized green states.

### ITEMIZED EMBODIMENTS

ITEM 1 A process for the manufacture of a spiropyran represented by the following formula (1): 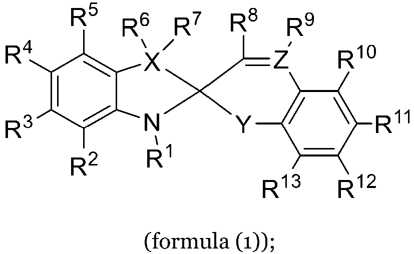 the process comprising the steps of
   - providing a precursor, wherein the precursor is a spiropyran represented by the following formula (2): 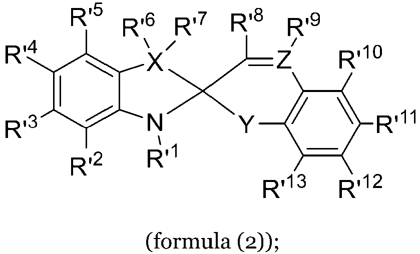
   - providing a reactant, wherein the reactant is
      an indolenium salt represented by the following formula (3): 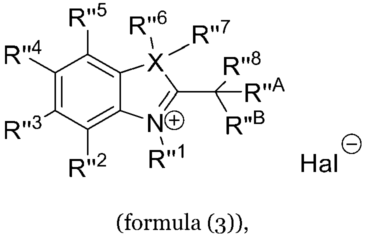
      or the corresponding 2-methyleneindoline compound; or
      a salicylaldehyde represented by the following formula (4): 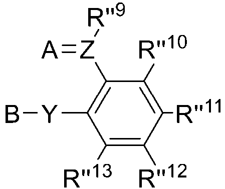 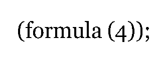 ; or
      a spiropyran represented by the following formula 5: 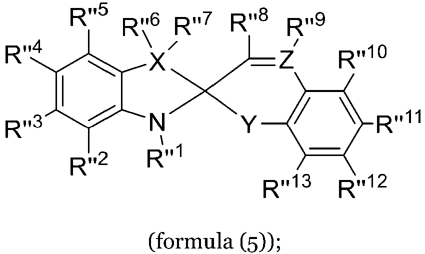
   - optionally pre-activating the precursor
   - providing a reaction mixture comprising the precursor or optionally a preactivated precursor, and the reactant to obtain said spiropyran of formula (1);
      wherein the spiropyran, the precursor, and the reactant are different from each other;
      wherein if the reactant is an indolenium salt of formula (3), the obtained spiropyran of formula (1) is represented by the following formula (1A): 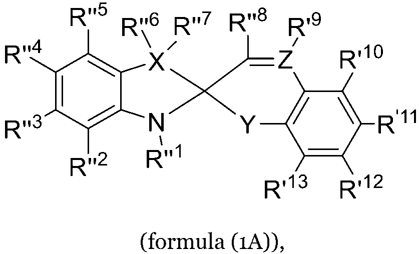
         wherein R"¹ to R"⁸ of formula (1A) are independently the same as R"¹ to R"⁸ of formula (3) and R'⁹ to R'¹³ of formula (1A) are independently the same as R'⁹ to R'¹³ of formula (2);
         wherein if the reactant is a salicylaldehyde of formula (4), the obtained spiropyran of formula (1) is represented by the following formula (1B): 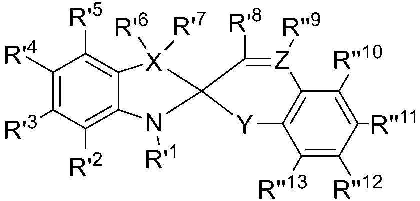 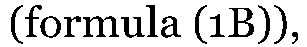
      wherein R'¹ to R'⁸ of formula (1B) are independently the same as R'¹ to R'⁸ of formula (2) and R"⁹ to R"¹³ of formula (1B) are independently the same as R"⁹ to R"¹³ of formula (4);
      wherein if the reactant is a spiropyran of formula (5),
      the obtained spiropyran of formula (1) is represented by formula (1A), wherein R"¹ to R"⁸ of formula (1A) are independently the same as R"¹ to R"⁸ of formula (5) and R'⁹ to R'¹³ of formula (1A) are independently the same as R'⁹ to R'¹³ of formula (2), or
      the obtained spiropyran of formula (1) is represented by formula (1B), wherein R'¹ to R'⁸ of formula (1B) are independently the same as R'¹ to R'⁸ of formula (2) and R"⁹ to R"¹³ of formula (1B) are independently the same as R"⁹ to R"¹³ of formula (5);
      wherein X is selected from S, C, or N; if X is S, then R⁶, R⁷, R'⁶, R'⁷, R"⁶, R"⁷ may not be present accordingly; if X is N, then R⁷, R'⁷, R"⁷ may not be present accordingly;
      wherein Y is selected from O, S, or N; where Y is N, the substituent contains the atoms necessary to complete a cyclic structure with R¹³ selected from the group consisting of benzimidazole, indoline, indole, dihydroquinoline, and tetrahydroquinoline;
      wherein Z is selected from N or C;
      wherein if present A is selected from O, S, or Se;
      wherein if present B is selected from H or D;
      wherein if present Hal⁻ is a halogen anion or an anionic compound;
      wherein if present R¹ to R¹³, R'¹ to R'¹³, and R"¹ to R"¹³ are independently selected from the group consisting of H; D; halogen; NO₂; CN; OH; SH; CF₃; substituted or unsubstituted C₁-C₂₀-alkyl; substituted or unsubstituted C₃-C₂₀-cycloalkyl; substituted or unsubstituted C₆-C₄₈-aryl; substituted or unsubstituted C₂-C₄₂-heteroaryl; substituted or unsubstituted C₂-C₄₉-alkyl acyl; substituted or unsubstituted C₂-C₄₉-aryl acyl; substituted or unsubstituted C₁-C₂₀-alkoxy; substituted or unsubstituted C₆-C₄₈-aryloxy; NH₂; substituted or unsubstituted C₁-C₂₀-alkyl ester; substituted or unsubstituted C₆-C₄₈-aryl ester; substituted or unsubstituted C₁-C₂₀ alkyl amide; substituted or unsubstituted C₆-C₄₈-aryl amide; NR'₂; SiR'₃; -O-SiR'₃, wherein R' is independently selected from the group consisting of substituted or unsubstituted C₁-C₂₀-alkyl and substituted or unsubstituted C₆-C₄₈-aryl, two R' may form a ring structure; substituted or unsubstituted carboxylic acids and salts thereof; substituted or unsubstituted sulfonic acids and salts thereof;
      substituted or unsubstituted sulfonic esters; substituted or unsubstituted sulfonic amides; formyl; ether, thioether; carbonate; carbonate ester; sulfates; boronic acids; boronic esters; phosphonic acids; phosphonic esters; phosphines; phosphates; peroxycarbonic acids; thiocarbonic acids; sulfinic acids; sulfinic esters; sulfonates; thiolesters, sulfoxides; sulfones; alkylsulfones; hydrazides; thioaldehydes; ketones; thioketones; oximes; hydrazines; nitroso; azo; diazo; diazonium; isocyanides; cyanate; isocyanate; thiocyanate; isothiocyanate; hydroperoxide; peroxide; acetals; ketal; orthoester; orthocarbonate esters; ammonium; imines; imides; azide; nitrate; isonitrile; nitrosoxy; substituted or unsubstituted carbamates; substituted or unsubstituted ethers; substituted or unsubstituted polyether carbamates; substituted or unsubstituted arylazo; substituted or unsubstituted C₂-C₂₀-alkynyl and substituted or unsubstituted C₂-C₂₀-alkenyl;
      wherein the one or more substituents if present in one or more of R¹ to R¹³, are independently selected from the group consisting of D; halogen; NO₂; CN, C₂-C₄₉-alkyl acyl; substituted or unsubstituted C₁-C₂₀-alkoxy; substituted or unsubstituted C₆-C₄₈-aryloxy; substituted or unsubstituted C₂-C₄₉-aryl acyl; (meth)acrylate; tosyl; sulfonic acid or salts thereof; carboxylic acid or salts thereof; boronic acid or salts thereof; phosphonic acid or salts thereof; NR'₃⁺, wherein R' is independently selected from the group consisting of H, D, substituted or unsubstituted C₁-C₂₀-alkyl and substituted or unsubstituted C₆-C₄₈-aryl, two R' may form a ring structure; NH₂; and OH;
      wherein if present two adjacent groups of R² to R⁵, R'² to R'5, R"² to R"5, R¹⁰ to R¹³; R'¹⁰ to R'¹³ and R"¹⁰ to R"¹³ may be independently linked to each other to form a fused ring structure; and
      wherein if present R"^{A} to R"^{B} are independently selected from H and D.
ITEM 2 The process according to item 1,
   wherein in formula (1) at least one of R² to R⁵ and R¹⁰ to R¹³ is a substituent selected from one of the following structures: 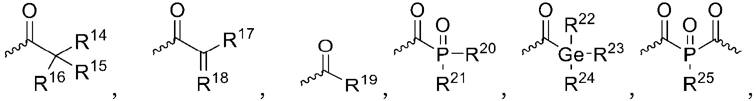 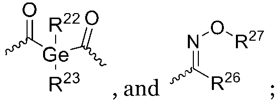
   wherein R¹⁴ to R²⁷ are independently selected from the group consisting of H; D; halogen; NO₂; CN; OH; SH; substituted or unsubstituted C₁-C₂₀-alkyl; substituted or unsubstituted C₃-C₂₀-cycloalkyl; substituted or unsubstituted C₆-C₄₈-aryl; substituted or unsubstituted C₂-C₄₂-heteroaryl; substituted or unsubstituted C₂-C₄₉-alkyl acyl; substituted or unsubstituted C₂-C₄₉-aryl acyl; substituted or unsubstituted C₁-C₂₀-alkoxy; substituted or unsubstituted C₆-C₄₈-aryloxy; NH₂; substituted or unsubstituted C₁-C₂₀-alkyl ester; substituted or unsubstituted C₆-C₄₈-aryl ester; substituted or unsubstituted C₁-C₂₀ alkyl amide; substituted or unsubstituted C₆-C₄₈-aryl amide; NR'₂; SiR'₃; -O-SiR'₃, wherein R' is independently selected from the group consisting of substituted or unsubstituted C₁-C₂₀-alkyl and substituted or unsubstituted C₆-C₄₈-aryl, two R' may form a ring structure; substituted or unsubstituted carboxylic acids and salts thereof; substituted or unsubstituted sulfonic acids and salts thereof; substituted or unsubstituted sulfonic esters; substituted or unsubstituted sulfonic amides; formyl; ether; thioether; carbonate; carbonate ester; sulfates; boronic acids; boronic esters; phosphonic acids; phosphonic esters; phosphines; phosphates; peroxycarbonic acids; thiocarbonic acids; sulfinic acids; sulfinic esters; sulfonates; thiolesters, sulfoxides; sulfones; alkylsulfones; hydrazides; thioaldehydes; ketones; thioketones; oximes; hydrazines; nitroso; azo; diazo; diazonium; isocyanides; cyanate; isocyanate; thiocyanate; isothiocyanate; hydroperoxide; peroxide; acetals; ketal; orthoester; orthocarbonate esters; ammonium; imines; imides; azide; nitrate; isonitrile; nitrosoxy; substituted or unsubstituted carbamates; substituted or unsubstituted ethers; substituted or unsubstituted polyether carbamates; substituted or unsubstituted arylazo; substituted or unsubstituted C₂-C₂₀-alkynyl and substituted or unsubstituted C₂-C₂₀-alkenyl; wherein the one or more substituents, if present in one or more of R¹⁴-R²⁷, are independently selected from the group consisting of D; halogen; NO₂; CN; C₂-C₄₉-alkyl acyl; substituted or unsubstituted C₁-C₂₀-alkoxy; substituted or unsubstituted C₆-C₄₈-aryloxy; substituted or unsubstituted C₂-C₄₉-aryl acyl; (meth)acrylate; tosyl; sulfonic acid or salts thereof; carboxylic acid or salts thereof; boronic acid or salts thereof; phosphonic acid or salts thereof; NR'₃⁺, wherein R' is independently selected from the group consisting of H, D, substituted or unsubstituted C₁-C₂₀-alkyl and substituted or unsubstituted C₆-C₄₈-aryl, two R' may form a ring structure; NH₂; and OH; and R¹⁵ and R¹⁶ may be linked to each other to form a unsubstituted or substituted ring structure;
      and/ or
   wherein in formula (1) at least one of R² to R⁵ and R¹⁰ to R¹³ is a substituent selected from the group consisting of chlorine; bromine; iodine; formyl; carbonate; carbonate ester; ester; amide; CF₃; substituted or unsubstituted C₂-C₄₉-alkyl acyl; substituted or unsubstituted C₂-C₄₉-aryl acyl; ketone; acyl; acetyl; oxime; aldehyde, NO₂; CN; (meth)acrylate; sulfone; alkylsulfones; sulfonamide; SO₂Me; SO₂NH₂; and tosyl;
      and/ or
   wherein in formula (1) at least one of R² to R⁵ and R¹⁰ to R¹³ is a substituent selected from the group consisting of sulfonic acid or salts thereof; carboxylic acid or salts thereof; boronic acid or salts thereof; phosphonic acid or salts thereof; NR'₃⁺, wherein R' is independently selected from the group consisting of H, D, substituted or unsubstituted C₁-C₂₀-alkyl and substituted or unsubstituted C₆-C₄₈-aryl, two R' may form a ring structure; substituted or unsubstituted sulfonic esters; substituted or unsubstituted sulfonic amides; phosphonic esters; phosphines; phosphates; sulfinic acids; sulfinic esters; sulfonates; sulfoxides; sulfones; alkylsulfones; oximes; isocyanides; cyanate; isocyanate; thiocyanate; isothiocyanate; ammonium; substituted or unsubstituted carbamates; (meth)acrylate; tosyl; NH₂ and OH;
      and/ or
   wherein in formula (1) R¹ is selected from the group consisting of H, D, substituted or unsubstituted C₁-C₆-alkyl, -CH₂-CH₂-OH, -CH₂-COOH, -CH₂-CH₂-COOH, -CH₂-CH₂-CH₂-nMe₃⁺, -CH₂-CH₂-CH₂-SO₃⁻, phenyl and benzyl, preferably R¹ is methyl, - CH₂-CH₂-OH, phenyl or benzyl, -CH₂-COOH, -CH₂-CH₂-COOH, -CH₂-CH₂- CH₂-nMe₃⁺ and -CH₂-CH₂- CH₂-SO₃⁻
      and/or
   the substituent on any of R¹⁴ to R²⁷, preferably R¹⁹, may contain the atoms necessary to complete a cyclic structure with one of R⁵-R⁸ or R¹⁰-R¹³.
ITEM 3 The process according to one of the preceding items, wherein in formula (1) at least one of R² to R⁵ and additionally at least one of R¹⁰ to R¹³ are substituents selected from one of the following formulae: 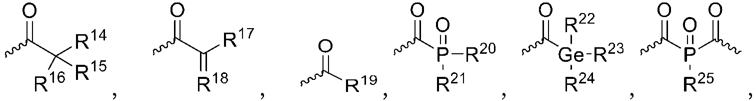 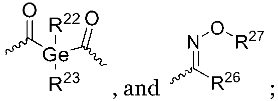
   wherein R¹⁴ to R²⁷ are defined as in item 2,
      and/ or
   wherein at least one of R¹⁰ and R¹² to R¹³ is a substituent selected from one of the following structures: 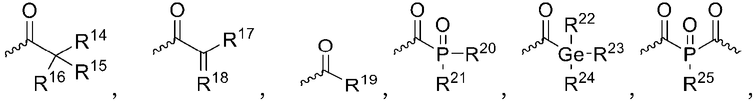 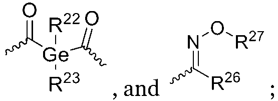
   wherein R¹⁴ to R²⁷ are defined as in item 2.
ITEM 4 The process according to one of the preceding items, wherein in formula (2) at least one of R'² to R'⁵ and R'¹⁰ to R'¹³, preferably at least one of R'¹⁰ to R'¹³, is a substituent selected from the group consisting of electron withdrawing substituents, acetyl, benzoyl, CN, CF₃ or one of the following structures: 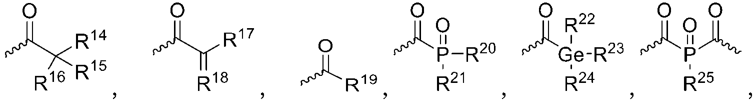 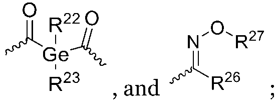
   wherein R¹⁴ to R²⁷ are defined as in item 2, or alternatively
   wherein in formula (2) at least one of R'² to R'⁵, preferably R'⁴, is a substituent selected from the group consisting of electron donating substituents, alkoxy, methoxy, or one of the following structures: 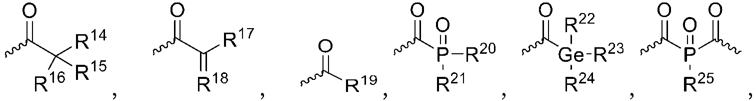 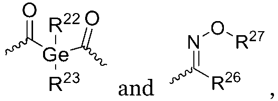
   wherein R¹⁴ to R²⁷ are defined as in item 2;
      and/or
   wherein at least one of R'¹⁰ to R'¹³ is CF₃;
      and/or
   wherein R'⁸ is alkyl, preferably methyl.
ITEM 5 The process according to one of the preceding items, wherein prior to providing the reaction mixture, the precursor is preactivated with a nucleophile preferably, an amine base, more preferably primary or secondary amine base, to obtain a preactivated precursor.
ITEM 6 The process according to one of the preceding items, wherein the reactant is the indolenium salt of formula (3): 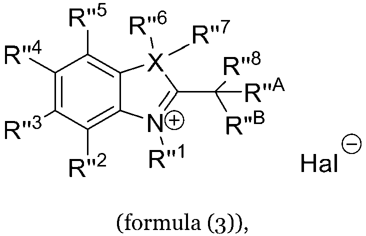
   or the corresponding 2-methyleneindoline compound;
   wherein X, Hal, R"^{A}, R"^{B} and R"¹ to R"⁸ are substituents as defined in item 1;
   wherein preferably the amount of electron withdrawing substituents in R"² to R"⁵ of formula (3) is higher than the amount of electron withdrawing substituents in R'¹⁰ to R'¹³ of formula (2);
      and/ or
   wherein the substituent in R"⁴ of formula (3) is stronger electron withdrawing group than the substituent in R'⁴ of formula (2),
      and/ or
   wherein the substituent in R"⁴ of formula (3) is chosen from the group consisting of carbonyl; chlorine; bromine; iodine; formyl; carbonate; carbonate ester; ester; amide; CF₃; substituted or unsubstituted C₂-C₄₉-alkyl acyl; substituted or unsubstituted C₂-C₄₉-aryl acyl; ketone; acyl; acetyl; benzoyl; oxime; aldehyde, NO₂; CN; (meth)acrylate; sulfones; alkylsulfones; sulfonamides; SO₂Me; SO₂NH₂.
ITEM 7 The process according to one of the preceding items, wherein the reaction mixture further comprises a catalytic amount of a base, preferably a secondary amine base.
ITEM 8 The process according to one of the precedings items, wherein the indolenium salt of formula (2) is formed from the corresponding 2 methyleneindoline compound and an acid, wherein preferably the acid is an organic or inorganic acid, more preferably the acid is selected from the group consisting of hydrochloric acid, acetic acid or formic acid.
ITEM 9 The process according to one of the preceding items, wherein prior to mixing the precursor with the reactant in a reaction mixture, the reactant is activated by converting the indolenium salt of formula (3) into a corresponding 2-methyleneindoline compound, (3A), which is then added to the reaction mixture, wherein preferably the reactant is activated with a base, more preferably the reactant is activated with an alkylamine, a hydroxide, or a carbonate.
ITEM 10 The process according to one of the preceding items, wherein an excess of more than 1.1 equivalents of the indolenium salt of formula (3) or the corresponding methyleneindoline of formula (3A) based on the precursor, preferably an excess of 1.1 to 1.2 equivalents of the indolenium salt or the corresponding methyleneindoline of formula (3A) based on the precursor, is added to the reaction mixture.
ITEM 11 The process according to one of the preceding items 1 to 9, wherein an excess of more than 2.0 equivalents of indolenium salt of formula (3) based on the precursor is added to the reaction mixture to form an adduct and then the adduct is treated with acid to form the spiropyran of formula (2).
ITEM 12 The process according to items 1 to 5, wherein the reactant is a salicylaldehyde of formula (4): 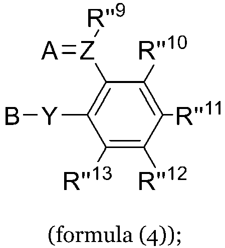
   wherein A, B, Z, Y, and R"⁹ to R"¹³ are substituents as defined in item 1, wherein preferably R"⁹ is H or D; and R"¹⁰ to R"¹³ are independently selected from the group consisting of H; D; halogen; tosyl; formyl; carbonate; carbonate ester; ester; amide; CF₃; substituted or unsubstituted C₂-C₄₉-alkyl acyl; substituted or unsubstituted C₂-C₄₉-aryl acyl; ketone; acyl; acetyl; benzoyl; oxime; aldehyde, NO₂; CN, (meth)acrylate; sulfone; alkylsulfone; sulfonamide; SO₂Me; SO₂NH₂; sulfonic acid or salts thereof; carboxylic acid or salts thereof; boronic acid or salts thereof; phosphonic acid or salts thereof; NR'₃⁺, wherein R' is independently selected from the group consisting of H, D, substituted or unsubstituted C₁-C₂₀-alkyl and substituted or unsubstituted C₆-C₄₈-aryl, two R' may form a ring structure; substituted or unsubstituted sulfonic esters; substituted or unsubstituted sulfonic amides; phosphonic esters; phosphines; phosphates; sulfinic acids; sulfinic esters; sulfonates; sulfoxides; sulfones; alkylsulfones; oximes; isocyanides; cyanate; isocyanate; thiocyanate; isothiocyanate; ammonium; substituted or unsubstituted carbamates; NH₂; OH; substituted or unsubstituted C₁-C₂₀-alkyl; substituted or unsubstituted C₃-C₂₀-cycloalkyl; substituted or unsubstituted C₆-C₄₈-aryl; substituted or unsubstituted C₁-C₂₀-alkoxy; substituted or unsubstituted C₆-C₄₈-aryloxy; substituted or unsubstituted C₁-C₂₀-alkyl ester; substituted or unsubstituted C₆-C₄₈-aryl ester; SiR'₃, -O-SiR'₃ wherein R' is independently selected from the group consisting of substituted or unsubstituted C₁-C₂₀-alkyl and substituted or unsubstituted C₆-C₄₈-aryl, two R' may form a ring structure, and
   or the reactant is a spiropyran of formula (5): 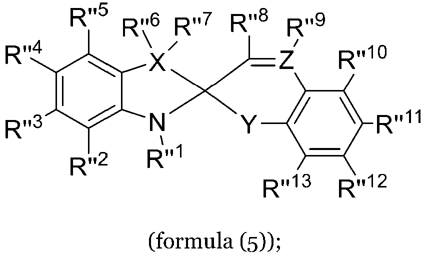
   wherein the reaction mixture may comprise a further catalyst, wherein preferably the catalyst is an amine base, more preferably the catalyst is a primary or secondary amine base.
ITEM 13 The process according to one of the preceding items, wherein the reaction mixture comprises a solvent, preferably alcohol, more preferably ethanol,
   wherein the obtained spiropyran has a lower solubility compared to the precursor in said solvent;
      and/ or
   wherein the reaction mixture further comprises a scavenger and said scavenger is able to bind to the obtained spiropyran, to bind to an obtained side product, or to deactivate an obtained side product in the reaction mixture, preferably the scavenger is an acid.
ITEM 14 A process for the manufacture of a precursor represented by the following formula (2): 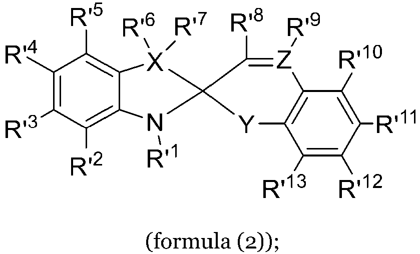
   wherein R'¹, R'⁶ to R'⁹ are independently selected from the group consisting of H, D, substituted or unsubstituted C₁-C₁₀-alkyl, preferably methyl; substituted or unsubstituted C₆-C₃₂-aryl, preferably phenyl; substituted or unsubstituted C₂-C₂₀-alkynyl and substituted or unsubstituted C₂-C₂₀-alkenyl and benzyl;
   X is C;
   Z is C;
   Y is O;
   R'² to R'⁵ and R'¹⁰ to R'¹³ are independently selected from the group consisting of H, D, F, Cl, Br, substituted or unsubstituted C₁-C₁₀-alkyl, substituted or unsubstituted C₆-C₃₂-aryl, substituted or unsubstituted C₁-C₂₀-alkoxy, substituted or unsubstituted C₆-C₄₈-aryloxy, CF₃, CN; wherein two adjacent groups of may be linked to each other to form a fused ring structure, preferably, a fused aromatic C₆-ring; and a substituent of the following formula: 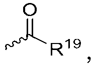
   wherein R¹⁹ is defined as in item 2;
   wherein at least one of R² to R⁵ and R¹⁰ to R¹³ is a substituent of the following formula: 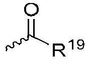
   the process comprising the steps of:
      - providing a reactant, wherein the reactant is a spiropyran represented by the following formula (2A): 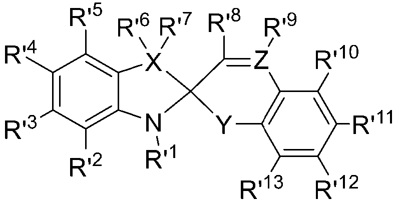 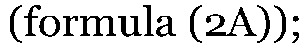 wherein at least one of R'² to R'⁵ and R'¹⁰ to R'¹³ is a halogen atom selected from the group consisting of Cl, Br and I; and the rest of the substituents are the same as in the precursor of formula (2);
      - reacting the halogen atom of the reactant in a metal-halogen exchange reaction, preferably in a metal-halogen exchange reaction with an organolithium reagent or Grignard reagent, to obtain a metal-spiropyran species,
      - subsequently reacting the metal-spiropyran species with a Weinreb-amide of the following formula: 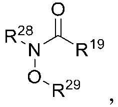 wherein R²⁸ and R²⁹ are selected from the group consisting of substituted or unsubstituted C₁-C₁₀-alkyl; substituted or unsubstituted C₆-C₃₂-aryl; substituted or unsubstituted C₂-C₂₀-alkynyl and substituted or unsubstituted C₂-C₂₀-alkenyl, preferably R²⁸ and R²⁹ are each methyl,
   to obtain the precursor of formula (2), wherein preferably the precursor of formula (2) is obtained after an acidic aqueous work-up.
ITEM 15 A process according to item 14, wherein at least one of R² to R⁵ of the reactant of formula (2a) is a halogen atom selected from the group consisting of Cl, Br and I and at least one of R¹⁰ to R¹³ of the reactant of formula (2a) is also a halogen atom selected from the group consisting of Cl, Br and I, and wherein at least two or more halogen atoms react in a metal-halogen exchange reaction an organolithium reagent or Grignard reagent and subsequently with a Weinreb-amide to obtain a precursor according to formula (2).
ITEM 16 A spiropyran represented by the following formula 1: 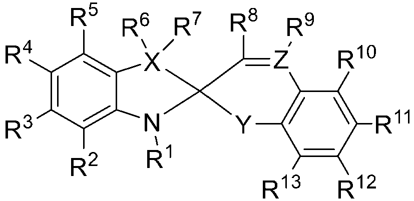 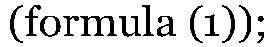
   wherein X is selected from S, C, or N; if X is S, then R⁶, R⁷ may not be present accordingly; if X is N, then R⁷ may not be present accordingly,
   wherein Y is selected from O, S, or N; where Y is N, the substituent contains the atoms necessary to complete a cyclic structure with R¹³ selected from the group consisting of benzimidazole, indoline, indole, dihydroquinoline, and tetrahydroquinoline;
   wherein Z is selected from N or CR⁹;
   wherein R¹ to R¹³ are independently selected from the group consisting of H; D; halogen; NO₂; CN; OH; SH; substituted or unsubstituted C₁-C₂₀-alkyl; substituted or unsubstituted C₃-C₂₀-cycloalkyl; substituted or unsubstituted C₆-C₄₈-aryl; substituted or unsubstituted C₂-C₄₂-heteroaryl; substituted or unsubstituted C₂-C₄₉-alkyl acyl; substituted or unsubstituted C₂-C₄₉-aryl acyl; substituted or unsubstituted C₁-C₂₀-alkoxy; substituted or unsubstituted C₆-C₄₈-aryloxy; NH₂; substituted or unsubstituted C₁-C₂₀-alkyl ester; substituted or unsubstituted C₆-C₄₈-aryl ester; substituted or unsubstituted C₁-C₂₀ alkyl amide; substituted or unsubstituted C₆-C₄₈-aryl amide; NR'₂; SiR'₃; -O-SiR'₃, wherein R' is independently selected from the group consisting of substituted or unsubstituted C₁-C₂₀-alkyl and substituted or unsubstituted C₆-C₄₈-aryl, two R' may form a ring structure; substituted or unsubstituted carboxylic acids and salts thereof; substituted or unsubstituted sulfonic acids and salts thereof; substituted or unsubstituted sulfonic esters; substituted or unsubstituted sulfonic amides; formyl; ether; thioether; carbonate; carbonate ester; sulfates; boronic acids; boronic esters; phosphonic acids; phosphonic esters; phosphines; phosphates; peroxycarbonic acids; thiocarbonic acids; sulfinic acids; sulfinic esters; sulfonates; thiolesters, sulfoxides; sulfones; alkylsulfones; hydrazides; thioaldehydes; ketones; thioketones; oximes; hydrazines; nitroso; azo; diazo; diazonium; isocyanides; cyanate; isocyanate; thiocyanate; isothiocyanate; hydroperoxide; peroxide; acetals; ketal; orthoester; orthocarbonate esters; ammonium; imines; imides; sulfonium salts; iodonium salts; diazo; azide; nitrate; isonitrile; nitrosoxy; substituted or unsubstituted thiocarbamates; substituted or unsubstituted carbamates; substituted or unsubstituted ethers; substituted or unsubstituted polyether carbamates; substituted or unsubstituted arylazo; substituted or unsubstituted C₂-C₂₀-alkynyl and substituted or unsubstituted C₂-C₂₀-alkenyl; wherein two adjacent groups of may be linked to each other to form a fused ring structure, preferably, a fused aromatic C₆-ring;
   wherein the one or more substituents, if present in one or more of R¹ to R¹³, are independently selected from the group consisting of D; halogen; NO₂; CN, C₂-C₄₉-alkyl acyl; substituted or unsubstituted C₁-C₂₀-alkoxy; substituted or unsubstituted C₆-C₄₈-aryloxy; substituted or unsubstituted C₂-C₄₉-aryl acyl; (meth)acrylate; tosyl; sulfonic acid or salts thereof; carboxylic acid or salts thereof; boronic acid or salts thereof; phosphonic acid or salts thereof; NR'₃⁺, wherein R' is independently selected from the group consisting of H, D, substituted or unsubstituted C₁-C₂₀-alkyl and substituted or unsubstituted C₆-C₄₈-aryl, two R' may form a ring structure; NH₂; and OH; wherein if present two adjacent groups of R¹⁰ to R¹³, and R² to R⁵ may be independently linked to each other to form a fused ring structure;
      and
   wherein at least one substituent for R² to R⁵ and R¹⁰ to R¹³ is selected from the group consisting of carbonyl; chlorine; bromine; iodine; formyl; carbonate; carbonate ester; ester; amide; CF₃; substituted or unsubstituted C₂-C₄₉-alkyl acyl; substituted or unsubstituted C₂-C₄₉-aryl acyl; ketone; acyl; acetyl; benzoyl; oxime; aldehyde, NO₂; CN; (meth)acrylate; sulfones; alkylsulfones; sulfonamides; SO₂Me; SO₂NH₂; methoxy; and tosyl;
   or is selected from one of the following formulae: 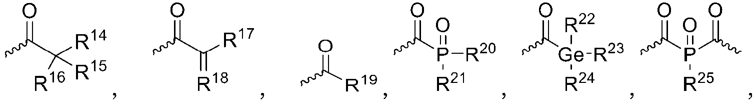 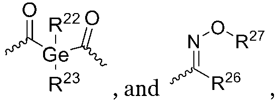
   wherein R¹⁴ to R²⁷ are independently selected from the group consisting of H; D; halogen; NO₂; CN; OH; SH; CF₃; benzoyl; substituted or unsubstituted C₁-C₂₀-alkyl; substituted or unsubstituted C₃-C₂₀-cycloalkyl; substituted or unsubstituted C₆-C₄₈-aryl; substituted or unsubstituted C₂-C₄₂-heteroaryl; substituted or unsubstituted C₂-C₄₉-alkyl acyl; substituted or unsubstituted C₂-C₄₉-aryl acyl; substituted or unsubstituted C₁-C₂₀-alkoxy; substituted or unsubstituted C₆-C₄₈-aryloxy, and NH₂; substituted or unsubstituted C₁-C₂₀-alkyl ester; substituted or unsubstituted C₆-C₄₈-aryl ester; substituted or unsubstituted C₁-C₂₀ alkyl amide; substituted or unsubstituted C₆-C₄₈-aryl amide; NR'₂, SiR'₃, -O-SiR'₃ wherein R' is independently selected from the group consisting of substituted or unsubstituted C₁-C₂₀-alkyl and substituted or unsubstituted C₆-C₄₈-aryl, two R' may form a ring structure; substituted or unsubstituted carboxylic acids and salts thereof; substituted or unsubstituted sulfonic acids and salts thereof; substituted or unsubstituted sulfonic esters; substituted or unsubstituted sulfonic amides; formyl; ether, thioether; carbonate; carbonate ester; sulfates; boronic acids; boronic esters; phosphonic acids; phosphonic esters; phosphines; phosphates; peroxycarbonic acids; thiocarbonic acids; sulfinic acids; sulfinic esters; sulfonates; thiolesters, sulfoxides; sulfones; alkylsulfones; hydrazides; thioaldehydes; ketones; thioketones; oximes; hydrazines; nitroso; azo; diazo; diazonium; isocyanides; cyanate; isocyanate; thiocyanate; isothiocyanate; hydroperoxide; peroxide; acetals; ketal; orthoester; orthocarbonate esters; ammonium; imines; imides; azide; nitrate; isonitrile; nitrosoxy; substituted or unsubstituted carbamates; substituted or unsubstituted ethers; substituted or unsubstituted polyether carbamates; substituted or unsubstituted arylazo; substituted or unsubstituted C₂-C₂₀-alkynyl and substituted or unsubstituted C₂-C₂₀-alkenyl;
      wherein the one or more substituents, if present in one or more of R¹⁴-R²⁷, are independently selected from the group consisting of D; halogen; NO₂; CN, C₂-C₄₉-alkyl acyl; substituted or unsubstituted C₁-C₂₀-alkoxy; substituted or unsubstituted C₆-C₄₈-aryloxy; substituted or unsubstituted C₂-C₄₉-aryl acyl; (meth)acrylate; tosyl; sulfonic acid or salts thereof, carboxylic acid or salts thereof, boronic acid or salts thereof, phosphonic acid or salts thereof, NR'₃⁺, wherein R' is independently selected from the group consisting of H, D, substituted or unsubstituted C₁-C₂₀-alkyl and substituted or unsubstituted C₆-C₄₈-aryl, two R' may form a ring structure; NH₂; and OH; and R¹⁵ and R¹⁶ may be linked to each other to form a unsubstituted or substituted ring structure,
      and
   wherein at least one other substituent for R² to R⁵ and R¹⁰ to R¹³ is selected from the group consisting of chlorine; bromine; iodine; formyl; carbonate; carbonate ester; ester; amide; CF₃; substituted or unsubstituted C₂-C₄₉-alkyl acyl; substituted or unsubstituted C₂-C₄₉-aryl acyl; ketone; acyl; oxime; aldehyde, NO₂; CN; (meth)acrylate; sulfones; alkylsulfones; sulfonamides; SO₂Me; SO₂NH₂; methoxy; and tosyl;
      and/or
   wherein at least one other substituent for R² to R⁵ and R¹⁰ to R¹³ is selected from sulfonic acid or salts thereof; carboxylic acid or salts thereof; boronic acid or salts thereof; phosphonic acid or salts thereof; NR'₃⁺, wherein R' is independently selected from the group consisting of H, D, substituted or unsubstituted C₁-C₂₀-alkyl and substituted or unsubstituted C₆-C₄₈-aryl, two R' may form a ring structure; substituted or unsubstituted sulfonic esters; substituted or unsubstituted sulfonic amides; phosphonic esters; phosphines; phosphates; sulfinic acids; sulfinic esters; sulfonates; sulfoxides; sulfones; alkylsulfones; oximes; isocyanides; cyanate; isocyanate; thiocyanate; isothiocyanate; ammonium; substituted or unsubstituted carbamates; (meth)acrylate; tosyl; NH₂; and OH
      and/or
   the substituent on any of R¹⁴ to R²⁷, preferably R¹⁹, may contain the atoms necessary to complete a cyclic structure with one of R⁵-R⁸ or R¹⁰-R¹³,
ITEM 17 A spiropyran according to item 16, wherein at least one of R² to R⁵ is selected from one of the following formulae: 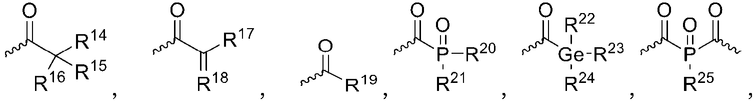 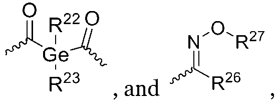
   wherein R¹⁴ to R²⁷ are defined as in item 16,
      and
   wherein at least one of R¹⁰ to R¹³ is selected from the group consisting of chlorine; bromine; iodine; formyl; carbonate; carbonate ester; ester; amide; CF₃; substituted or unsubstituted C₂-C₄₉-alkyl acyl; substituted or unsubstituted C₂-C₄₉-aryl acyl; ketone; acyl; acetyl, benzoyl; oxime; aldehyde; NO₂; CN; (meth)acrylate; sulfones; alkylsulfones; sulfonamides; SO₂Me; SO₂NH₂; methoxy; and tosyl;
      and/or
   wherein at least one other substituent for R¹⁰ to R¹³ is selected from sulfonic acid or salts thereof; carboxylic acid or salts thereof; boronic acid or salts thereof; phosphonic acid or salts thereof; NR'₃⁺, wherein R' is independently selected from the group consisting of H, D, substituted or unsubstituted C₁-C₂₀-alkyl and substituted or unsubstituted C₆-C₄₈-aryl, two R' may form a ring structure; substituted or unsubstituted sulfonic esters; substituted or unsubstituted sulfonic amides; phosphonic esters; phosphines; phosphates; sulfinic acids; sulfinic esters; sulfonates; sulfoxides; sulfones; alkylsulfones; oximes; isocyanides; cyanate; isocyanate; thiocyanate; isothiocyanate; ammonium; substituted or unsubstituted carbamates; (meth)acrylate; tosyl; NH₂; and OH.
ITEM 18 A spiropyran according to items 16 to 17, wherein at least one of R¹⁰ to R¹³ is selected from one of the following formulae: 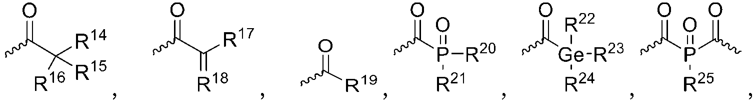 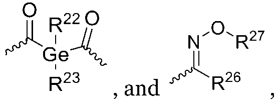
   wherein R¹⁴ to R²⁷ are defined as in item 16,
      and
   wherein at least one of R² to R⁵ is selected from chlorine; bromine; iodine; formyl; carbonate; carbonate ester; ester; amide; CF₃; substituted or unsubstituted C₂-C₄₉-alkyl acyl; substituted or unsubstituted C₂-C₄₉-aryl acyl; ketone; acyl; acetyl; benzoyl; oxime; aldehyde; NO₂; CN; (meth)acrylate; sulfones; alkylsulfones; sulfonamides; SO₂Me; SO₂NH₂; methoxy; and tosyl
      and/or
   wherein at least one other substituent for R² to R⁵ is selected from sulfonic acid or salts thereof; carboxylic acid or salts thereof; boronic acid or salts thereof; phosphonic acid or salts thereof; NR'₃⁺, wherein R' is independently selected from the group consisting of H, D, substituted or unsubstituted C₁-C₂₀-alkyl and substituted or unsubstituted C₆-C₄₈-aryl, two R' may form a ring structure; substituted or unsubstituted sulfonic esters; substituted or unsubstituted sulfonic amides; phosphonic esters; phosphines; phosphates; sulfinic acids; sulfinic esters; sulfonates; sulfoxides; sulfones; alkylsulfones; oximes; isocyanides; cyanate; isocyanate; thiocyanate; isothiocyanate; ammonium; substituted or unsubstituted carbamates; (meth)acrylate; tosyl; NH₂; and OH.
ITEM 19 A spiropyran according to items 16 to 18, wherein at least one of R² to R⁵ and additionally at least one of R¹⁰ to R¹³ are substituents selected from one of the following formulae: 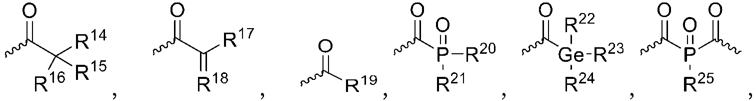 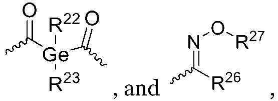
   wherein R¹⁴ to R²⁷ are defined as in claim 16.
      and / or
   wherein the selected substituent at one R² to R⁵ is different from the selected substituent at one of R¹⁰ to R¹³,
      and/ or
   wherein at least one of R¹⁰ and R¹² to R¹³ is a substituent selected from one of the following formulae: 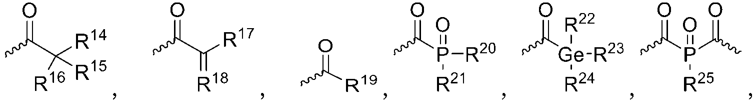 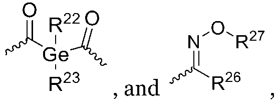
   wherein R¹⁴ to R²⁷ are defined as in item 16.
ITEM 20 A spiropyran according to one of the preceding items 16 to 19, wherein at least two substituents of R², R³, R⁵, and R¹⁰-R¹³ are independently selected from the group consisting of 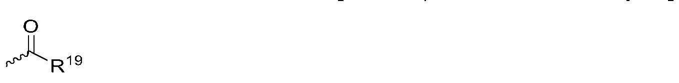 or an electron withdrawing group.
ITEM 21 A spiropyran according to one of the preceding items 16 to 20, wherein at least two substituents of R²-R⁵, R¹⁰, R¹², R¹³ are independently selected from the group consisting of 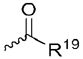 or an electron withdrawing group.
ITEM 22 A spiropyran according to one of the preceding items 16 to 21, wherein one of R²-R⁵ is 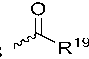 and one of R¹⁰-R¹³ is 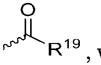 wherein R¹⁹ is the same.
ITEM 23 A spiropyran according to one of the preceding items 16 to 22, wherein one of R²-R⁵ is 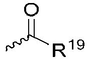 and one of R¹⁰-R¹³ is 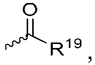 wherein R¹⁹ is different.
ITEM 24 A spiropyran according to one of the preceding items 16 to 23, wherein R²-R⁵ is an electron withdrawing group, and / or
   wherein at least one of R⁴, R¹⁰, R¹², R¹³ is 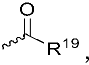 at least one of R¹², R¹³ is 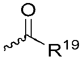 and more preferably R¹³ is 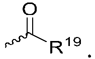
ITEM 25 A spiropyran according to one of the preceding items 16 to 24, wherein at least one of R²-R⁵ is a substituted or unsubstituted arylacyl and at least one of R¹⁰-R¹³ is a substituted or unsubstituted alkylacyl.
ITEM 26 A spiropyran according to one of the preceding items 16 to 25, wherein R⁴ is an electron withdrawing group and R¹³ is 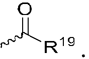
ITEM 27 A spiropyran according to one of the preceding items 16 to 26, wherein R¹¹ is H or an electron withdrawing group.
ITEM 28 A spiropyran according to one of the preceding items 16 to 27, wherein at least one of R²-R⁵ and R¹⁰-R¹³ is a substituted arylacyl and contains an electron withdrawing group as a substituent, preferably the substituent is selected from the group consisting of CN, CF₃, F, Cl, Br, I, OCF₃, substituted or unsubstituted alkylester, substituted or unsubstituted aryl ester, SO₂Me and SO₂NH₂.
ITEM 29 A spiropyran according to one of the preceding items 16 to 28, wherein R¹ is selected from the group consisting of H, D, substituted or unsubstituted C₁-C₆-alkyl, -CH₂-CH₂-OH, -CH₂-COOH, -CH₂-CH₂-COOH, -CH₂-CH₂-CH₂-nMe₃⁺, -CH₂-CH₂-CH₂-SO₃⁻, phenyl and benzyl, preferably R¹ is methyl, -CH₂-CH₂-OH, phenyl or benzyl, -CH₂-COOH, -CH₂-CH₂-COOH, -CH₂-CH₂- CH₂-nMe₃⁺ and -CH₂-CH₂- CH₂-SO₃⁻.
ITEM 30 A process for locally polymerizing a starting material by using a spiropyran according to one of the preceding items 16 to 29 as a photoinitiator and irradiating the spiropyran with light of least one wavelength, preferably with light of at least two different wavelengths.
ITEM 31 A process for locally polymerizing a starting material by dual color photopolymerization, comprising:
   - providing a polymerizable starting material containing photoinitiator molecules wherein the photoinitiator molecule is a spiropyran according to one of the preceding items 16 to 29, which can be converted by sequential optical excitation into a reactive state in which the photoinitiator molecules locally trigger polymerization of the starting material; and
   - photopolymerizing the starting material in a local volume by irradiating light of a first wavelength and light of a second wavelength, different from the first wavelength, into the local volume, whereby in the local volume
   - the photoinitiator molecules are converted, due to the absorption of the light of the first wavelength, from an initial state in which the photoinitiator molecules substantially do not absorb the light of the second wavelength, into an intermediate state with changed optical properties compared to the initial state, such that the photoinitiator molecules in the intermediate state absorb the light of the second wavelength; and
   - the photoinitiator molecules are transferred from the intermediate state to the reactive state due to the absorption of the light of the second wavelength, which triggers the polymerization locally.
ITEM 32 A process for the formation of shaped body by dual color photopolymerization, comprising:
   - providing a polymerizable starting material containing photoinitiator molecules wherein the photoinitiator molecule is a spiropyran according to one of the preceding items 16 to 29, which can be converted by sequential optical excitation into a reactive state in which the photoinitiator molecules locally trigger polymerization of the starting material; and
   - photopolymerizing the starting material in a local volume by irradiating light of a first wavelength and light of a second wavelength, different from the first wavelength, into the local volume, whereby in the local volume
   - the photoinitiator molecules are converted, due to the absorption of the light of the first wavelength, from an initial state in which the photoinitiator molecules substantially do not absorb the light of the second wavelength, into an intermediate state with changed optical properties compared to the initial state, such that the photoinitiator molecules in the intermediate state absorb the light of the second wavelength; and
   - the photoinitiator molecules are transferred from the intermediate state to the reactive state due to the absorption of the light of the second wavelength, which triggers the polymerization locally; and
   - the photoinitiator molecules may be transferred from the intermediate state to the initial state spontaneously in a thermal reaction.
ITEM 33 The process according to any of the items 30 to 32, further comprising a step of post-processing the shaped body is provided, wherein the post-processing comprises a thermal treatment of the shaped body and/or an optical treatment of the shaped body.
ITEM 34 The process according to item 30 or 33, further comprising a step in which a three-dimensional object is formed from the shaped body by removal of the shaped body from the polymerizable starting material.
ITEM 35 The process according to item 34, wherein the post-processing of the three-dimensional object comprises modifying the optical properties of the three-dimensional object resulting in a decrease of the absorption properties of the three-dimensional object for at least one wavelength in a wavelength range between 300 nm and 2000 nm and/or increasing the transmissive properties of the three-dimensional object for at least one wavelength in the wavelength range between 300 nm and 2000 nm.
ITEM 36 The process according to any of the items 30 to 35, wherein the optical treatment is carried out by irradiating light with an intensity and a wavelength by which a three-dimensional object is not removed from the polymerizable material.

## Claims

1. A formulation for volumetric printing comprising
- 1 to 99.9999 wt.-%, with respect to the weight of the total formulation, of a photopolymerizable compound; and
- 0.0001 to 20 wt.-%, with respect to the weight of the total formulation, of a spiropyran of formula (1) 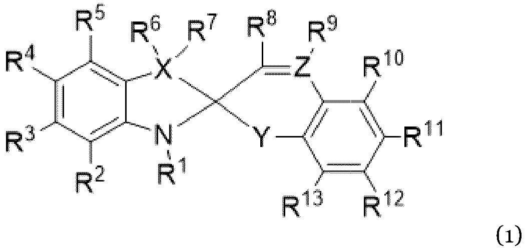
wherein
- X is selected from S, C, or N; wherein, if X is S, then R⁶ and R⁷ are not present; and if X is N, then R⁷, is not present;
- Y is selected from O, S, or N; wherein, if Y is N, Y contains the atoms necessary to complete together with R¹³ a cyclic structure selected from the group consisting of benzimidazole, indoline, indole, dihydroquinoline, and tetrahydroquinoline;
- Z is selected from N or C, wherein, if Z is N, R⁹ is not present;
- if present R¹ to R¹³, are independently selected from the group consisting of H; D; halogen; NO₂; CN; OH; SH; CF₃; substituted or unsubstituted C₁-C₂₀-alkyl; substituted or unsubstituted C₃-C₂₀-cycloalkyl; substituted or unsubstituted C₆-C₄₈-aryl; substituted or unsubstituted C₂-C₄₂-heteroaryl; substituted or unsubstituted C₂-C₄₉-alkyl acyl; substituted or unsubstituted C₂-C₄₉-aryl acyl; substituted or unsubstituted C₁-C₂₀-alkoxy; substituted or unsubstituted C₆-C₄₈-aryloxy; NH₂; substituted or unsubstituted C₁-C₂₀-alkyl ester; substituted or unsubstituted C₆-C₄₈-aryl ester; substituted or unsubstituted C₁-C₂₀ alkyl amide; substituted or unsubstituted C₆-C₄₈-aryl amide; NR'₂; SiR'₃; -O-SiR'₃, wherein R' is independently selected from the group consisting of substituted or unsubstituted C₁-C₂₀-alkyl and substituted or unsubstituted C₆-C₄₈-aryl, two R' may form a ring structure; substituted or unsubstituted carboxylic acids and salts thereof; substituted or unsubstituted sulfonic acids and salts thereof; substituted or unsubstituted sulfonic esters; substituted or unsubstituted sulfonic amides; formyl; ether, thioether; carbonate; carbonate ester; sulfates; boronic acids; boronic esters; phosphonic acids; phosphonic esters; phosphines; phosphates; peroxycarbonic acids; thiocarbonic acids; sulfinic acids; sulfinic esters; sulfonates; thiolesters, sulfoxides; sulfones; alkylsulfones; hydrazides; thioaldehydes; ketones; thioketones; oximes; hydrazines; nitroso; azo; diazo; diazonium; isocyanides; cyanate; isocyanate; thiocyanate; isothiocyanate; hydroperoxide; peroxide; acetals; ketal; orthoester; orthocarbonate esters; ammonium; imines; imides; azide; nitrate; isonitrile; nitrosoxy; substituted or unsubstituted carbamates; substituted or unsubstituted ethers; substituted or unsubstituted polyether carbamates; substituted or unsubstituted arylazo; substituted or unsubstituted C₂-C₂₀-alkynyl and substituted or unsubstituted C₂-C₂₀-alkenyl;
- the one or more substituents if present in one or more of R¹ to R¹³, are independently selected from the group consisting of D; halogen; NO₂; CN, C₂-C₄₉-alkyl acyl; substituted or unsubstituted C₁-C₂₀-alkoxy; substituted or unsubstituted C₆-C₄₈-aryloxy; substituted or unsubstituted C₂-C₄₉-aryl acyl; (meth)acrylate; tosyl; sulfonic acid or salts thereof; carboxylic acid or salts thereof; boronic acid or salts thereof; phosphonic acid or salts thereof; NR'₃⁺, wherein R' is independently selected from the group consisting of H, D, substituted or unsubstituted C₁-C₂₀-alkyl and substituted or unsubstituted C₆-C₄₈-aryl, two R' may form a ring structure; NH₂; and OH;
- if present two adjacent groups of R² to R⁵, and R¹⁰ to R¹³ may be independently linked to each other to form a fused ring structure;
wherein it is provided that
(a) R¹³ is 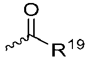 wherein R¹⁹ substituted or unsubstituted C₆-C₄₈-aryl; and/or
(b) at least one of R² to R⁵ is independently selected from a carboxylic acid and salts thereof, and a sulfonic acid and salts thereof; and/or
(c) R¹ is selected from the group consisting of -CH₂-COOH, -CH₂-CH₂-COOH, and -CH₂-CH₂-CH₂-SO₃⁻.

2. The formulation according to claim 1, wherein
(a) R¹³ is 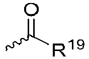 wherein R¹⁹ substituted or unsubstituted C₆-C₄₈-aryl; and
(b) at least one of R² to R⁵ is an electron withdrawing group, preferably selected from the group consisting of CN, F, Cl, OCF₃, NO₂, an ester group, formyl, acetyl, benzoyl, substituted benzoyl, acyl groups, SO₂CF₃, SO₂Me, SO₂Ph, SO₂NH₂, SF₅, NR₃⁺, pyridinium, halogen, fluorinated alkyl, and fluorinated aryls.

3. The formulation according to claim 1 or 2, wherein R¹³ is 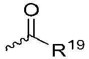 wherein R¹⁹ is unsubstituted or substituted C₆-C₄₈-aryl, wherein the substituted C₆-C₄₈-aryl preferably contains a substituent selected from the group consisting of CN, CF₃, F, Cl, Br, I, OCF₃, substituted or unsubstituted alkylester, substituted or unsubstituted aryl ester, SO₂Me and SO₂NH₂.

4. The formulation according to claim 1 or 2, wherein R¹³ is selected from 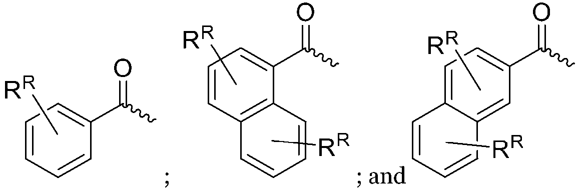 wherein R^{R} is independently selected from the group consisting of hydrogen, deuterium, methyl, tert-butyl, substituted and unsubstituted phenyl, methoxy, CN, CF₃, F, Cl, Br, I, OCF₃, C₁-C₂₀-alkyl ester, C₆-C₄₈-aryl ester, alkyl acyl, aryl acyl, acetyl, benzoyl, NMe₂, SO₂Me, and SO₂NH₂.

5. A process for locally polymerizing a staring material using a spiropyran of formula (1) as a photoinitiator and irradiating the spiropyran of formula (1) with light of at least one wavelength 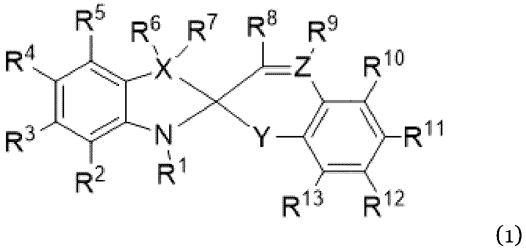 wherein
- X is selected from S, C, or N; wherein, if X is S, then R⁶ and R⁷ are not present; and if X is N, then R⁷, is not present;
- Y is selected from O, S, or N; wherein, if Y is N, Y contains the atoms necessary to complete together with R¹³ a cyclic structure selected from the group consisting of benzimidazole, indoline, indole, dihydroquinoline, and tetrahydroquinoline;
- Z is selected from N or C, wherein, if Z is N, R⁹ is not present;
- if present R¹ to R¹³, are independently selected from the group consisting of H; D; halogen; NO₂; CN; OH; SH; CF₃; substituted or unsubstituted C₁-C₂₀-alkyl; substituted or unsubstituted C₃-C₂₀-cycloalkyl; substituted or unsubstituted C₆-C₄₈-aryl; substituted or unsubstituted C₂-C₄₂-heteroaryl; substituted or unsubstituted C₂-C₄₉-alkyl acyl; substituted or unsubstituted C₂-C₄₉-aryl acyl; substituted or unsubstituted C₁-C₂₀-alkoxy; substituted or unsubstituted C₆-C₄₈-aryloxy; NH₂; substituted or unsubstituted C₁-C₂₀-alkyl ester; substituted or unsubstituted C₆-C₄₈-aryl ester; substituted or unsubstituted C₁-C₂₀ alkyl amide; substituted or unsubstituted C₆-C₄₈-aryl amide; NR'₂; SiR'₃; -O-SiR'₃, wherein R' is independently selected from the group consisting of substituted or unsubstituted C₁-C₂₀-alkyl and substituted or unsubstituted C₆-C₄₈-aryl, two R' may form a ring structure; substituted or unsubstituted carboxylic acids and salts thereof; substituted or unsubstituted sulfonic acids and salts thereof; substituted or unsubstituted sulfonic esters; substituted or unsubstituted sulfonic amides; formyl; ether, thioether; carbonate; carbonate ester; sulfates; boronic acids; boronic esters; phosphonic acids; phosphonic esters; phosphines; phosphates; peroxycarbonic acids; thiocarbonic acids; sulfinic acids; sulfinic esters; sulfonates; thiolesters, sulfoxides; sulfones; alkylsulfones; hydrazides; thioaldehydes; ketones; thioketones; oximes; hydrazines; nitroso; azo; diazo; diazonium; isocyanides; cyanate; isocyanate; thiocyanate; isothiocyanate; hydroperoxide; peroxide; acetals; ketal; orthoester; orthocarbonate esters; ammonium; imines; imides; azide; nitrate; isonitrile; nitrosoxy; substituted or unsubstituted carbamates; substituted or unsubstituted ethers; substituted or unsubstituted polyether carbamates; substituted or unsubstituted arylazo; substituted or unsubstituted C₂-C₂₀-alkynyl and substituted or unsubstituted C₂-C₂₀-alkenyl;
- the one or more substituents if present in one or more of R¹ to R¹³, are independently selected from the group consisting of D; halogen; NO₂; CN, C₂-C₄₉-alkyl acyl; substituted or unsubstituted C₁-C₂₀-alkoxy; substituted or unsubstituted C₆-C₄₈-aryloxy; substituted or unsubstituted C₂-C₄₉-aryl acyl; (meth)acrylate; tosyl; sulfonic acid or salts thereof; carboxylic acid or salts thereof; boronic acid or salts thereof; phosphonic acid or salts thereof; NR'₃⁺, wherein R' is independently selected from the group consisting of H, D, substituted or unsubstituted C₁-C₂₀-alkyl and substituted or unsubstituted C₆-C₄₈-aryl, two R' may form a ring structure; NH₂; and OH;
- if present two adjacent groups of R² to R⁵, and R¹⁰ to R¹³ may be independently linked to each other to form a fused ring structure;
wherein it is provided that
(a) R¹³ is 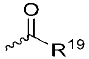 wherein R¹⁹ substituted or unsubstituted C₆-C₄₈-aryl; and/or
(b) at least one of R² to R⁵ is independently selected from a carboxylic acid and salts thereof, and a sulfonic acid and salts thereof; and/or
(c) R¹ is selected from the group consisting of -CH₂-COOH, -CH₂-CH₂-COOH, and -CH₂-CH₂-CH₂-SO₃⁻.

6. The process according to claim 5, wherein
(a) R¹³ is 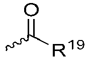 wherein R¹⁹ substituted or unsubstituted C₆-C₄₈-aryl; and
(b) at least one of R² to R⁵ is an electron withdrawing group, preferably selected from the group consisting of CN, F, Cl, OCF₃, NO₂, an ester group, formyl, acetyl, benzoyl, substituted benzoyl, acyl groups, SO₂CF₃, SO₂Me, SO₂Ph, SO₂NH₂, SF₅, NR₃⁺, pyridinium, halogen, fluorinated alkyl, and fluorinated aryls.

7. The formulation according to claim 5 or 6, wherein R¹³ is 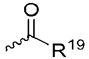 wherein R¹⁹ is unsubstituted or substituted C₆-C₄₈-aryl, wherein the substituted C₆-C₄₈-aryl preferably contains a substituent selected from the group consisting of CN, CF₃, F, Cl, Br, I, OCF₃, substituted or unsubstituted alkylester, substituted or unsubstituted aryl ester, SO₂Me and SO₂NH₂.

8. The formulation according to claim 5 or 6, wherein R¹³ is selected from 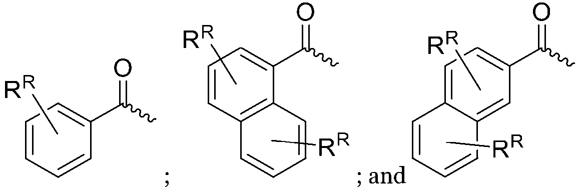 wherein R^{R} is independently selected from the group consisting of hydrogen, deuterium, methyl, tert-butyl, substituted and unsubstituted phenyl, methoxy, CN, CF₃, F, Cl, Br, I, OCF₃, C₁-C₂₀-alkyl ester, C₆-C₄₈-aryl ester, alkyl acyl, aryl acyl, acetyl, benzoyl, NMe₂, SO₂Me, and SO₂NH₂.

9. A process for locally polymerizing a starting material by dual color photopolymerization and forming a shaped body, comprising the following steps:
- providing a container which is at least partially filled with a polymerizable starting material containing photoinitiator molecules, which can be converted by sequential optical excitation into a reactive state in which the photoinitiator molecules locally trigger polymerization of the starting material; and
- photopolymerizing the starting material in the container by irradiating light of a first wavelength and light of a second wavelength, different from the first wavelength, into the container, whereby in the container
- the photoinitiator molecules are converted, due to the absorption of the light of the first wavelength, from an initial state in which the photoinitiator molecules substantially do not absorb the light of the second wavelength, into an intermediate state with changed optical properties compared to the initial state, such that the photoinitiator molecules in the intermediate state absorb the light of the second wavelength; and
- the photoinitiator molecules are transferred from the intermediate state to the reactive state due to the absorption of the light of the second wavelength, which triggers the polymerization locally to form the shaped body; and
- wherein the initial state of the photoinitiator molecules in the photopolymerizable material have an extinction coefficient at the first wavelength which is lower than 5000 L mol⁻¹cm⁻¹.

10. The process according to claim 9, wherein the process further comprises a step of post-processing the shaped body comprising a thermal treatment of the shaped body and/or an optical treatment of the shaped body.

11. The process according to claim 10, wherein the post-processing comprises modifying the optical properties of the three-dimensional object resulting in a decrease of the absorption properties of the three-dimensional object for at least one wavelength in a wavelength range between 300 nm and 2000 nm, preferably between 350 nm and 1000 nm, more preferably between 400 nm and 900 nm, most preferably between 450 nm and 800 nm and/or in an increase of the transmissive properties of the three-dimensional object for at least one wavelength in the wavelength range between 300 nm and 2000 nm, preferably between 350 nm and 1000 nm, more preferably between 400 nm and 900 nm, most preferably between 450 nm and 800 nm.

12. The process according to any one of claims 9 to 11, wherein the photoinitiator molecule is the spiropyran of formula (1) as it is defined in claim 1.

13. A process for locally polymerizing a starting material by using a spiropyran as a photoinitiator and irradiating the spiropyran with light of least one wavelength, preferably with light of at least two different wavelengths, wherein the spiropyran represented by the following formula 1: 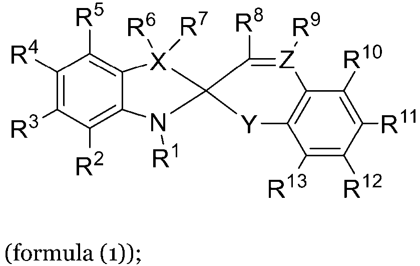
wherein X is selected from S, C, or N; if X is S, then R⁶, R⁷ may not be present accordingly; if X is N, then R⁷ may not be present accordingly,
wherein Y is selected from O, S, or N; where Y is N, the substituent contains the atoms necessary to complete a cyclic structure with R¹³ selected from the group consisting of benzimidazole, indoline, indole, dihydroquinoline, and tetrahydroquinoline;
wherein Z is selected from N or CR⁹;
wherein R¹ to R¹³ are independently selected from the group consisting of H; D; halogen; NO₂; CN; OH; SH; substituted or unsubstituted C₁-C₂₀-alkyl; substituted or unsubstituted C₃-C₂₀-cycloalkyl; substituted or unsubstituted C₆-C₄₈-aryl; substituted or unsubstituted C₂-C₄₂-heteroaryl; substituted or unsubstituted C₂-C₄₉-alkyl acyl; substituted or unsubstituted C₂-C₄₉-aryl acyl; substituted or unsubstituted C₁-C₂₀-alkoxy; substituted or unsubstituted C₆-C₄₈-aryloxy; NH₂; substituted or unsubstituted C₁-C₂₀-alkyl ester; substituted or unsubstituted C₆-C₄₈-aryl ester; substituted or unsubstituted C₁-C₂₀ alkyl amide; substituted or unsubstituted C₆-C₄₈-aryl amide; NR'₂; SiR'₃; -O-SiR'₃, wherein R' is independently selected from the group consisting of substituted or unsubstituted C₁-C₂₀-alkyl and substituted or unsubstituted C₆-C₄₈-aryl, two R' may form a ring structure; substituted or unsubstituted carboxylic acids and salts thereof; substituted or unsubstituted sulfonic acids and salts thereof; substituted or unsubstituted sulfonic esters; substituted or unsubstituted sulfonic amides; formyl; ether; thioether; carbonate; carbonate ester; sulfates; boronic acids; boronic esters; phosphonic acids; phosphonic esters; phosphines; phosphates; peroxycarbonic acids; thiocarbonic acids; sulfinic acids; sulfinic esters; sulfonates; thiolesters, sulfoxides; sulfones; alkylsulfones; hydrazides; thioaldehydes; ketones; thioketones; oximes; hydrazines; nitroso; azo; diazo; diazonium; isocyanides; cyanate; isocyanate; thiocyanate; isothiocyanate; hydroperoxide; peroxide; acetals; ketal; orthoester; orthocarbonate esters; ammonium; imines; imides; sulfonium salts; iodonium salts; diazo; azide; nitrate; isonitrile; nitrosoxy; substituted or unsubstituted thiocarbamates; substituted or unsubstituted carbamates; substituted or unsubstituted ethers; substituted or unsubstituted polyether carbamates; substituted or unsubstituted arylazo; substituted or unsubstituted C₂-C₂₀-alkynyl and substituted or unsubstituted C₂-C₂₀-alkenyl; wherein two adjacent groups of may be linked to each other to form a fused ring structure, preferably, a fused aromatic C₆-ring;
wherein the one or more substituents, if present in one or more of R¹ to R¹³, are independently selected from the group consisting of D; halogen; NO₂; CN, C₂-C₄₉-alkyl acyl; substituted or unsubstituted C₁-C₂₀-alkoxy; substituted or unsubstituted C₆-C₄₈-aryloxy; substituted or unsubstituted C₂-C₄₉-aryl acyl; (meth)acrylate; tosyl; sulfonic acid or salts thereof; carboxylic acid or salts thereof; boronic acid or salts thereof; phosphonic acid or salts thereof; NR'₃⁺, wherein R' is independently selected from the group consisting of H, D, substituted or unsubstituted C₁-C₂₀-alkyl and substituted or unsubstituted C₆-C₄₈-aryl, two R' may form a ring structure; NH₂; and OH; wherein if present two adjacent groups of R¹⁰ to R¹³, and R² to R⁵ may be independently linked to each other to form a fused ring structure;
and
wherein at least one substituent for R² to R⁵ and R¹⁰ to R¹³ is selected from the group consisting of carbonyl; chlorine; bromine; iodine; formyl; carbonate; carbonate ester; ester; amide; CF₃; substituted or unsubstituted C₂-C₄₉-alkyl acyl; substituted or unsubstituted C₂-C₄₉-aryl acyl; ketone; acyl; acetyl; benzoyl; oxime; aldehyde, NO₂; CN; (meth)acrylate; sulfones; alkylsulfones; sulfonamides; SO₂Me; SO₂NH₂; methoxy; and tosyl;
or is selected from one of the following formulae: 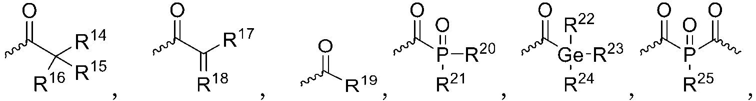 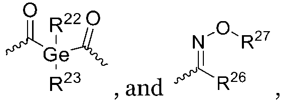
wherein R¹⁴ to R²⁷ are independently selected from the group consisting of H; D; halogen; NO₂; CN; OH; SH; CF₃; benzoyl; substituted or unsubstituted C₁-C₂₀-alkyl; substituted or unsubstituted C₃-C₂₀-cycloalkyl; substituted or unsubstituted C₆-C₄₈-aryl; substituted or unsubstituted C₂-C₄₂-heteroaryl; substituted or unsubstituted C₂-C₄₉-alkyl acyl; substituted or unsubstituted C₂-C₄₉-aryl acyl; substituted or unsubstituted C₁-C₂₀-alkoxy; substituted or unsubstituted C₆-C₄₈-aryloxy, and NH₂; substituted or unsubstituted C₁-C₂₀-alkyl ester; substituted or unsubstituted C₆-C₄₈-aryl ester; substituted or unsubstituted C₁-C₂₀ alkyl amide; substituted or unsubstituted C₆-C₄₈-aryl amide; NR'₂, SiR'₃, - O-SiR'₃ wherein R' is independently selected from the group consisting of substituted or unsubstituted C₁-C₂₀-alkyl and substituted or unsubstituted C₆-C₄₈-aryl, two R' may form a ring structure; substituted or unsubstituted carboxylic acids and salts thereof; substituted or unsubstituted sulfonic acids and salts thereof; substituted or unsubstituted sulfonic esters; substituted or unsubstituted sulfonic amides; formyl; ether, thioether; carbonate; carbonate ester; sulfates; boronic acids; boronic esters; phosphonic acids; phosphonic esters; phosphines; phosphates; peroxycarbonic acids; thiocarbonic acids; sulfinic acids; sulfinic esters; sulfonates; thiolesters, sulfoxides; sulfones; alkylsulfones; hydrazides; thioaldehydes; ketones; thioketones; oximes; hydrazines; nitroso; azo; diazo; diazonium; isocyanides; cyanate; isocyanate; thiocyanate; isothiocyanate; hydroperoxide; peroxide; acetals; ketal; orthoester; orthocarbonate esters; ammonium; imines; imides; azide; nitrate; isonitrile; nitrosoxy; substituted or unsubstituted carbamates; substituted or unsubstituted ethers; substituted or unsubstituted polyether carbamates; substituted or unsubstituted arylazo; substituted or unsubstituted C₂-C₂₀-alkynyl and substituted or unsubstituted C₂-C₂₀-alkenyl;
wherein the one or more substituents, if present in one or more of R¹⁴-R²⁷, are independently selected from the group consisting of D; halogen; NO₂; CN, C₂-C₄₉-alkyl acyl; substituted or unsubstituted C₁-C₂₀-alkoxy; substituted or unsubstituted C₆-C₄₈-aryloxy; substituted or unsubstituted C₂-C₄₉-aryl acyl; (meth)acrylate; tosyl; sulfonic acid or salts thereof, carboxylic acid or salts thereof, boronic acid or salts thereof, phosphonic acid or salts thereof, NR'₃⁺, wherein R' is independently selected from the group consisting of H, D, substituted or unsubstituted C₁-C₂₀-alkyl and substituted or unsubstituted C₆-C₄₈-aryl, two R' may form a ring structure; NH₂; and OH; and R¹⁵ and R¹⁶ may be linked to each other to form a unsubstituted or substituted ring structure,
and
wherein R¹³ is substituted or unsubstituted C₂-C₄₉-aryl acyl.
